# EUROPEAN PATENT SPECIFICATION

(11) **EP 2 389 587 B1**
(45) Date of publication and mention of the grant of the patent: **25.12.2013**
(21) Application number: 10704840.7
(22) Date of filing: 26.01.2010
(51) Int. Cl.: G01N 33/68

(54) **DIAGNOSTIC AND PROGNOSTIC METHODS RELATING TO ALZHEIMER'S DISEASE**
DIAGNOSTISCHES UND PROGNOSTISCHES VERFAHREN IN VERBINDUNG MIT MORBUS ALZHEIMER
PROCÉDÉ DIAGNOSTIC ET PROGNOSTIC CONCERNANT LA MALADIE D'ALZHEIMER

(30) Priority: 26.01.2009 GB 0901232; 11.03.2009 GB 0904209
(43) Date of publication of application: 30.11.2011
(73) Proprietor: Electrophoretics Limited, Cobham Surrey KT11 3EP (GB); King's College London, Strand, London WC2R 2LS (GB)
(72) Inventor: LOVESTONE, Simon, Harold, London SE5 8AF (GB); CAMPBELL, James, Cobham KT11 3EP (GB); O'BRIEN, Darragh, Patrick, William, Cobham KT11 3EP (GB); GÜNTERT, Andreas, Christian, London SE5 8AF (GB); BYERS, Helen, Louise, Cobham KT11 3EP (GB)
(74) Representative: Richards, William John
(86) International application number: PCT/GB2010/000107
(87) International publication number: WO 2010/084327

(56) References cited:
- WO-A2-02/46767
- WO-A2-2006/035237
- WO-A2-2006/134390
- WO-A2-2007/068985
- US-A1- 2008 206 737
- DAYON L ET AL: "Relative quantification of proteins in human cerebrospinal fluids by MS/MS using 6-plex isobaric tags" ANALYTICAL CHEMISTRY, AMERICAN CHEMICAL SOCIETY, US LNKD- DOI:10.1021/AC702422X, vol. 80, no. 8, 15 April 2008 (2008-04-15), pages 2921-2931, XP002495978 ISSN: 0003-2700 [retrieved on 2008-03-01]

## Description

### Field of the Invention

The present invention relates to methods and compositions relating to Alzheimer's disease. In particular, the present invention provides methods of diagnostic and prognostic measurement of Alzheimer's disease using differentially expressed proteins.

### Background to the Invention

Alzheimer's disease (AD), the most common cause of dementia in older individuals, is a debilitating neurodegenerative disease for which there is currently no cure. It destroys neurons in parts of the brain, chiefly the hippocampus, which is a region involved in coding memories. Alzheimer's disease gives rise to an irreversible progressive loss of cognitive functions and of functional autonomy. The earliest signs of AD may be mistaken for simple forgetfulness, but in those who are eventually diagnosed with the disease, these initial signs inexorably progress to more severe symptoms of mental deterioration. While the time it takes for AD to develop will vary from person to person, advanced signs include severe memory impairment, confusion, language disturbances, personality and behaviour changes, and impaired judgement. Persons with AD may become non-communicative and hostile. As the disease ends its course in profound dementia, patients are unable to care for themselves and often require institutionalisation or professional care in the home setting. While some patients may live for years after being diagnosed with AD, the average life expectancy after diagnosis is eight years.

In the past, AD could only be definitively diagnosed by brain biopsy or upon autopsy after a patient died. These methods, which demonstrate the presence of the characteristic plaque and tangle lesions in the brain, are still considered the gold standard for the pathological diagnoses of AD. However, in the clinical setting brain biopsy is rarely performed and diagnosis depends on a battery of neurological, psychometric and biochemical tests, including the measurement of biochemical markers such as the ApoE and tau proteins or the beta-amyloid peptide in cerebrospinal fluid and blood.

Better biomarkers are needed for diagnosing AD and other dementias. A biological marker that fulfils the requirements for the diagnostic test for AD would have several advantages. An ideal biological marker would be one that identifies AD cases at a very early stage of the disease, before there is degeneration observed in the brain imaging and neuropathological tests. Detection of a biomarker or panel of biomarkers could be the first indicator for starting treatment as early as possible, and also very valuable in screening the effectiveness of new therapies, particularly those that are focussed on preventing the development of neuropathological changes. A biological marker would also be useful in the follow-up of the development of the disease.

Markers related to pathological characteristics of AD, such as plaques and tangles (Aβ and tau), have been the most extensively studied. The most promising has been from studies of CSF concentration of Aβ(1-40), Aβ(1-42) and tau or the combination of both proteins in AD. Many studies have reported a decrease in Aβ(1-42) in CSF, while the total Aβ protein or Aβ(1-40) concentration remain unchanged (Ida, Hartmann et al. 1996; Kanai, Matsubara et al. 1998; Andreasen, Hesse et al. 1999).

Whilst cerebrospinal fluid (CSF) levels of Aβ and tau are promising biomarkers for diagnosis of AD they are not showing such diagnostic utility in more accessible body fluids. Cerebrospinal fluid is difficult to obtain from human patients. Its collection typically involves a serious invasive technique such as lumbar puncture, which is performed under sedation. This is a highly skilled and complex procedure, requiring qualified and specially trained medical staff. Furthermore, it is time consuming and may require anaesthetic, as well as extended co-operation from the patient. Moreover, collection of cerebrospinal fluid is an uncomfortable and often painful procedure, with prolonged headache being a common symptom, as well as carrying inherent risks of infection and possible paralysis to the patient.

In the light of the limitations of cerebrospinal fluid as a routine clinical sample, considerable interest resides in plasma as a source of biomarkers for neurodegenerative conditions such as Alzheimer's disease. WO 06/035237 describes proteomics studies that identified a number of differentially expressed proteins and described certain methods for the diagnosis of Alzheimer's disease.

Further studies are described in WO2006/134390, US2008/206737 and WO02/46767.

However, it remains the case that biomarkers known in the art to be associated with Alzheimer's disease have had limited or insignificant prognostic value. Whilst current clinical diagnosis of Alzheimer's disease based on general neurological symptoms and imprecise cognitive function tests is reasonably robust, it remains a problem to describe, and in particular to predict, the likely progress of disease in living patients. Thus, prognosis, as well as diagnosis, remains a problem in the art in connection with living patients.

The present invention seeks to overcome problem(s) associated with the prior art.

### Summary of the Invention

Broadly the present disclosure is directed to methods, reagents and kits for the diagnostic and prognostic monitoring of patients at risk of or suffering from Alzheimer's disease. More specifically the present disclosure describes three protein markers whose levels in plasma vary wherein the level of each protein provides information on a certain aspect of a patient's risk of developing the disease, and/or on the rate of progression of any such disease. The present invention is defined in the claims and relates to assaying the protein marker gelsolin.

A method of determining the nature or degree of progression of cognitive decline in Alzheimer's disease in a human or animal subject is described, the method comprising detecting the level of a differentially expressed protein identified by the methods described herein in a tissue sample or body fluid sample from said subject.

A method of determining the approximate age of onset Alzheimer's disease in a human or animal subject at risk of developing the disease is described, the method comprising detecting the level of a differentially expressed protein marker identified by the methods described herein in a tissue sample or body fluid sample from said subject.

A method of determining an individual's risk of developing Alzheimer's disease is described, the method comprising detecting the level of a differentially expressed protein marker identified by the methods described herein in a tissue sample or body fluid sample from said subject.

A method of predicting and/or monitoring the response of a subject with AD to treatment is described, the method comprising detecting the level of a differentially expressed protein marker identified by the methods described herein in a tissue sample or body fluid sample from said subject. In this context it is understood that the subject may be a human subject or may be a non-human subject. Non-human subjects include non-vertebrate and vertebrate models of AD including gene amplification, gene knockdown and transgenic models.

In certain embodiments it is preferable to measure levels of the biomarker of the present invention in serial diagnostic samples taken from the same patient. Changes in the levels of biomarker with time may provide additional, clinically useful information as to the occurrence, continued rate of progression and/or the response of the patient to treatment for AD.

Reagents and kits useful in performing the methods are described.

In particular, it is a specific advantage of the invention that the diagnostic and prognostic methods involve assay of particular protein markers (biomarkers) from blood. Blood is easily and quickly collected with minimal invasiveness. Furthermore, collection of blood requires substantially less medical training and qualification than collection of cerebrospinal fluid, making it cheaper and less demanding to obtain. Moreover, risks to the patient can be advantageously minimised or eliminated by basing the methods of the invention on detection in blood.

Furthermore, the inventors identify a defined group of biomarkers which share certain properties, in particular the ability to be detected in blood and to give reliable diagnostic and/or prognostic indications in connection with Alzheimer's disease. Thus, the invention advantageously provides methods for aiding the diagnosis of Alzheimer's disease, and methods for aiding prediction of the prognosis for patients which have Alzheimer's disease. The methods may also be applied in monitoring the effectiveness of treatment of patients suffering from Alzheimer's disease whereby successful treatment is evidenced by a move in the biomarker plasma levels back towards, or back to, that of a non-Alzheimer's state.

Specifically, the present invention identifies and describes proteins that are differentially expressed in the plasma of individuals with Alzheimer's disease relative to their expression in the normal state and, in particular, identifies and describes proteins associated with defining the age of onset and likely rate of cognitive decline in Alzheimer's disease. Further, the present invention provides methods aiding diagnostic and prognostic measurement of Alzheimer's disease using the differentially expressed proteins. Still further, the present disclosure provides reagents and kits for the diagnosis and prognostic monitoring of Alzheimer's disease.

Thus the invention provides a method for aiding the diagnosis of Alzheimer's disease in a subject, said method comprising; providing a sample of a blood derivative, wherein said blood derivative is serum or plasma, obtained from said subject; assaying the amount of gelsolin present in said sample; comparing the amount of gelsolin present in said sample to a reference amount of gelsolin present in a sample from a healthy subject, wherein detection of a gelsolin level in the sample from said subject which is lower than the gelsolin level in the reference sample indicates an increased likelihood of Alzheimer's disease in said subject. It should be understood that the reference sample may be taken from an unrelated healthy subject or may be an earlier sample taken from the same subject prior to the onset of Alzheimer's disease symptoms.

The sample may comprise blood plasma.

Suitably said blood plasma may be depleted for one or more of albumin; transferrin; IgG; IgA; antitrypsin or haptoglobin. Suitably such depletion is prior to the analysis step(s) of the methods of the invention.

Suitably said blood plasma has been depleted for each of albumin; transferrin; IgG; IgA; antitrypsin or haptoglobin.

Suitably the protein is detected by western blotting.

Suitably the protein is detected by bead suspension array.

Suitably the protein is detected by planar array.

Suitably the protein is detected by isobaric protein tagging. This embodiment involves all having the same mass. This embodiment may be assayed using a TMTcalibrator type approach.

Suitably the protein is detected by isotopic protein tagging. This embodiment involves having different masses within the same identical chemical structure. This embodiment may be assayed using a TMT-SRM type approach. Suitably an isotopic dilution assay such as AQUA may be used.

Suitably the protein is detected by mass spectrometer-based assay.

Suitably the gelsolin protein is detected by reference to one or more of the following peptides of Table B: SEQ ID NO: 30, SEQ ID NO: 31, SEQ ID NO: 32.

Also described is the use for diagnostic, prognostic and therapeutic applications, relating to Alzheimer's disease, of a material which recognises, binds to or has affinity for a polypeptide, or a variant or mutant thereof, wherein the polypeptide is selected from gelsolin (SEQ ID NO:1), C1 protease inhibitor (SEQ ID NO:2), or Ceruloplasmin (SEQ ID NO:3).

Also described is the use as described above of a combination of materials, each of which respectively recognises, binds to or has affinity for one or more of said polypeptide(s), or a variant or mutant thereof.

Suitably the or each material is an antibody or antibody chip.

Suitably the material is an antibody with specificity for one or more of said polypeptide(s), or a fragment, variant or mutant thereof.

Also described is an assay device for use in the diagnosis of Alzheimer's disease, which comprises a solid substrate having a location containing a material, which recognizes, binds to or has affinity for a polypeptide, or a fragment, variant or mutant thereof, wherein the polypeptide is selected from gelsolin (SEQ ID NO:1), C1 protease inhibitor (SEQ ID NO:2), or Ceruloplasmin (SEQ ID NO:3).

Suitably the solid substrate has a plurality of locations each respectively containing a material which recognizes, binds to or has affinity for a polypeptide, or a fragment, variant or mutant thereof, wherein the polypeptide is selected from gelsolin (SEQ ID NO:1), C1 protease inhibitor (SEQ ID NO:2), or Ceruloplasmin (SEQ ID NO:3).

Suitably the material is an antibody or antibody chip.

Suitably the assay device as described above has a unique addressable location for each antibody, thereby to permit an assay readout for each individual polypeptide or for any combination of polypeptides.

Suitably the assay device as described above, includes an antibody to a polypeptide wherein the polypeptide is selected from gelsolin (SEQ ID NO:1), C1 protease inhibitor (SEQ ID NO:2), or Ceruloplasmin (SEQ ID NO:3).

Suitably the assay device as described above further has a location containing a material which recognizes, binds to or has affinity for glutathione S transferase P.

Suitably the material is an antibody or antibody chip.

Also described is a kit for use in the diagnosis of Alzheimer's disease, comprising an assay device as described above, and means for detecting the amount of one or more of the polypeptides in a sample of body fluid taken from a subject.

Also described is a kit for use in the detection of gelsolin polypeptide, said kit comprising one or more of the following peptides of Table B: SEQ ID NO: 30, SEQ ID NO: 31, SEQ ID NO: 32.

Also described is a kit for use in the diagnosis of Alzheimer's disease, comprising one or more of the following peptides of Table B: SEQ ID NO: 30, SEQ ID NO: 31, SEQ ID NO: 32. Suitably said kit comprises at least one further peptide of Table B.

Suitably one or more of said peptides comprises a heavy isotope. Suitably one or more of said peptides comprises several heavy isotopes. Such isotopes may comprise carbon-13 or nitrogen-15. The advantage of this embodiment is that the heavy isotopes provide a different mass to an otherwise unaltered peptide, thereby facilitating its detection/identification.

Suitably one or more of said peptides comprises a TMT tag. Suitably said kit comprises a further isotopic TMT tag for labelling of a sample polypeptide. Suitably such a tag may comprise TMT-6.

Also described is a method of determining the APOE ε4 genotype of a subject, said method comprising assaying the C1 protease inhibitor level in a sample of blood from said subject.

Also described is a method of predicting the age of onset of Alzheimer's disease for a subject, said method comprising assaying the ceruloplasmin levels in a sample of blood from said subject.

### Biomarkers

The biomarker is gelsolin (e.g. SEQ ID NO:1). Also described are C1 protease inhibitor (also referred to herein as 'C1 inhibitor' or 'C1 inh') (e.g. SEQ ID NO:2) and ceruloplasmin (e.g. SEQ ID NO:3). These markers each have the advantage of being detectable in blood.

Thus, the biomarker proteins demonstrated in this study that are important in discriminating AD and NDC are Gelsolin, C1 inhibitor and Ceruloplasmin.

Gelsolin was found in lower levels in AD and correlated with cognitive decline per year. Thus gelsolin is an essential biomarker according to the present invention.

The two other proteins found in the multivariate analysis to be important for discriminating AD and NDC were C1 inhibitor and Ceruloplasmin. C1 inhibitor protein and Ceruloplasmin were associated with other clinical parameters, i.e. APOE ε4 genotype and age of onset. Whilst these latter proteins did not show a statistically significant difference in plasma protein levels between AD and NDC, they were associated with APOE ε4 genotype and age of onset respectively, and thereby provide means of identifying an individual's risk of developing AD and/or of assessing duration of a diagnosed disease. Thus, all three biomarkers are important in the common area of Alzheimer's disease diagnosis, prognosis, and therapeutic monitoring.

### Gelsolin

Gelsolin, also called actin-depolymerizing factor (ADF) or Brevin, occurs intracellularly in cytosol and mitochondria, as well as extracellularly in blood plasma. The main function of this 82kDa sized protein is known to be as a key regulator of actin filament assembly and is regulated by Ca²⁺ (Sun et al., 1999). Interestingly, a single nucleotide mutation in the Gelsolin gene, which leads to the exchange of an amino acid, is the cause of familial amyloidosis Finnish type (Levy et al., 1990, Maury et al., 1990). Gelsolin has also been related to a familial type of cerebral amyloid angiopathy (Kiuru et al., 1999) and was shown to bind Aβ in a concentration-dependant manner (Chauhan et al., 1999, Ji et al., 2008). Gelsolin inhibits the fibrillization of Aβ peptides and can also defibrillize preformed Aβ fibrils (Ray et al., 2000). It was also shown that Gelsolin plays an important role in inhibiting Aβ-induced cytotoxicity by inhibiting apoptotic mitochondrial changes (Qiao et al., 2005). Amyloid plaques are one of the two main pathological findings in AD and different strategies have been undertaken to decrease the brain plaque load. The increased clearance of Aβ from the central nervous system (CNS) was shown to improve memory function in human (Gilman et al., 2005) and decrease behavioural deficits in transgenic mice (Janus et al., 2000). One strategy to achieve this, also called the peripheral sink hypothesis, is by shifting the Aβ equilibrium between blood plasma and CNS to the periphery (Matsuoka et al., 2003), be it with active or passive immunization or through other Aβ binding proteins including Gelsolin (Matsuoka et al., 2003). In line with this, the induction of peripheral expression of plasma Gelsolin was also shown to reduce brain Aβ and was suggested as a suitable gene-therapeutic approach for the prevention or treatment of AD (Hirko et al., 2007). Given that Gelsolin binds Aβ, reduces the toxicity of Aβ fibrils and lowers the Aβ burden in the CNS, it is plausible that decreased plasma Gelsolin levels in AD, as we demonstrate herein, contribute to a faster disease progression.

When the marker is gelsolin, suitably the blood level of gelsolin is compared with a normal or reference blood level of gelsolin. If the level of gelsolin detected in the patient is seen to be lower than the level of gelsolin in the normal or reference sample, this indicates an increased likelihood of the patient having Alzheimer's disease.

Gelsolin levels may also be advantageously used as a predictor of rate of cognitive decline. Specifically, lower gelsolin levels correlate with a greater level of cognitive decline per year. In other words, the degree to which gelsolin levels detected in the blood of a patient are lower than those detected in a normal or reference sample correlates with the degree of cognitive decline expected or predicted year by year for that patient.

Moreover, gelsolin levels are also surprisingly shown to correlate with the disease progression rate. In other words, the lower the level of gelsolin levels found in blood from a patient compared with a normal or reference sample, the faster the disease progression rate predicted for that patient.

It is an advantage of the invention that blood based markers of disease progression are taught herein. Furthermore, it is an advantage of the invention that the levels of blood based biomarkers may be used to predict disease progression rate of that patient.

### C1 protease inhibitor

Plasma protease C1 inhibitor (C1 inh) is an inhibitor of the complement pathway and a member of the so called serpins, serine protease inhibitors. C1 inh is an acute phase protein and its main function is the inhibition of the complement system to prevent spontaneous activation. A deficiency in C1 inh plays a causative role in the development of acquired and hereditary angiodema (Carugati et al., 2001). In AD, an activation of the complement pathway is known to occur already in very early stages (McGeer and McGeer, 2002) and several of its components, including C1 inh, have been shown to be associated with amyloid plaques (Veerhuis et al., 1998, Strohmeyer et al., 2002). C1 inh has recently also been suggested as a biomarker in AD plasma in patients treated with rosiglitazone (Akuffo et al., 2008). However, the role of C1 inh in the disease process remains unclear, since it was shown that C1 inh and CD59 do not effectively inhibit complement activation in AD (Yasojima et al., 1999).

C1 protease inhibitor levels may be used as an indicator of APOE ε4 (APOE epsilon 4) genotype. Suitably levels of C1 protease inhibitor are not used alone in the diagnosis of Alzheimer's disease, but are rather advantageously combined with other markers, or used alone in order to aid the diagnosis of an APOE ε4 genotype.

### Ceruloplasmin

Ceruloplasmin, also known as ferroxidase, is the major copper-carrying protein in the blood and plays also a role in iron metabolism. Copper deficiency has been attributed as one of the causes for AD and has been extensively studied and reviewed (Gaggelli et al., 2006). Ceruloplasmin levels have been studied in blood (Giometto et al., 1988, Hye et al., 2006, Kessler et al., 2006), CSF (Loeffler et al., 1994) and brain tissue (Connor et al., 1993, Loeffler et al., 1996, Loeffler et al., 2001) with different results. In our study, a significant (positive) correlation of Ceruloplasmin levels with age of onset was established. Due to its main function in copper transport and the observed correlation with age of onset, copper imbalance seems to have a main impact on the onset and course of AD.

The use of ceruloplasmin levels in aiding the diagnosis or prediction of age of onset of Alzheimer's disease is described. In particular, a positive correlation of ceruloplasmin levels with age of onset of Alzheimer's disease is disclosed herein. Suitably, ceruloplasmin levels are not used alone for the diagnosis of Alzheimer's disease. Suitably, ceruloplasmin levels may be used in combination with other markers in aiding the diagnosis of Alzheimer's disease, or preferably ceruloplasmin levels are used alone in order to aid the prediction of age of onset of Alzheimer's disease for a particular patient.

### Combinations

The invention may be applied as part of a panel of biomarkers in order to provide a more robust diagnosis or prognosis. Moreover, the invention may be applied as part of a panel of biomarkers in order to provide a more complete picture of the disease state or possible outcomes for a given patient.

At least gelsolin and optionally one of C1 protease inhibitor, and ceruloplasmin are assayed suitably in a broader panel of markers according to the present invention.

More suitably, at least two of gelsolin, C1 protease inhibitor and ceruloplasmin are assayed suitably in a broader panel of markers according to the present invention which at least includes gelsolin.

Suitably when two markers are assayed, those markers are gelsolin and C1 protease inhibitor. This permits aiding a diagnosis of disease, together with an indication of the APOE ε4 ("APOE epsilon 4") genotype, such as in a single assay format, advantageously avoiding performing a separate genotyping test for Apoε4.

Suitably, when two markers are assayed those markers are gelsolin and ceruloplasmin. This offers the advantage of aiding the prediction of age of onset for a particular patient, aiding the diagnosis of whether or not that patient has already developed the disease. Thus, if gelsolin levels are found to be normal, but ceruloplasmin levels indicate a particular age of onset, then re-testing or monitoring of that patient may be advantageously indicated based on the outcome of the gelsolin/ ceruloplasmin combined assay.

When two markers are assayed, those markers may be C1 protease inhibitor and ceruloplasmin. This combination is not expected to provide a direct indication of diagnosis of a diseased state. This combination offers the advantage of providing descriptive/predictive information about a patient, which may be useful in assessing risk for that particular patient. Moreover, when this combination of markers is used, then issues of counselling regarding a positive diagnosis of Alzheimer's disease are advantageously avoided. Moreover, this combination of markers might be usefully employed as a pre-screen, for example to provide an indication of susceptibility or probability of developing a disease, and patients may be scheduled for a full diagnostic test at an appropriate future point depending on the indications from the ceruloplasmin/C1 protease inhibitor combined results.

Suitably when more than two biomarkers are assayed, those biomarkers comprise gelsolin, C1 protease inhibitor and ceruloplasmin. This combination advantageously maximises the amount of information provided to a patient for a given analysis.

Of course, the skilled reader will appreciate that the specific biomarker of the present invention may be advantageously combined with other markers known in the art. Such extended panels which comprise the specific biomarkers discussed herein are of course intended to be embraced by the invention. Selection of further known markers for testing in such a panel embodiment may be accomplished by the skilled reader according to the appropriate sources. In this context additional biomarkers may relate to AD, to other neurological conditions from which a differential diagnosis of AD is required, or to other diseases commonly associated with patients with AD or whose symptoms mimic those of AD. One such set of additional markers related to AD are provided in WO 06/035237.

Thus a preferred group of markers comprises at least
Gelsolin (Swiss prot accession number P06396; SEQ ID NO: 1); and one or more proteins selected from
C1 protease inhibitor (SEQ ID NO: 2)
ceruloplasmin (SEQ ID NO: 3)
clusterin (SwissProt accession number P10909; SEQ ID NO:4)
complement c3 (P01024; SEQ ID NO:5)
serum amyloid P component (P02743; SAP; SEQ ID NO:6)
alpha-2-macroglobulin (P01023; A2M; SEQ ID NO:7)
gamma-fibrinogen (P02679; SEQ ID NO:8)
complement factor H (P08603; CFH; SEQ ID NO:9)
apolipoprotein E (P02649; ApoE; SEQ ID NO:10).

A method of aiding the diagnosis of Alzheimer's disease in a subject is described, said method comprising assaying at least two of gelsolin, C1 protease inhibitor and ceruloplasmin in a sample of blood from said subject. Suitably the levels of each of gelsolin, C1 protease inhibitor and ceruloplasmin are assayed in a sample of blood from said subject.

Suitably said subject is a human.

Suitably said subject is a non-human mammal.

Suitably said subject is a rodent.

### Sample

The sample may be any tissue that can be obtained from a subject suspected of having AD or of being at risk of developing AD. In the context of humans it is preferred that the sample is a body fluid. More preferably the sample is blood. Even more preferred the sample is blood plasma.

In particular, when the biomarker being assayed comprises one or more of gelsolin, C1 protease inhibitor or ceruloplasmin, then suitably cerebrospinal fluid is specifically excluded as the sample. Of course, in further embodiments of the invention involving assay of other biomarkers, cerebrospinal fluid may be analysed as part of a wider analysis.

The sample may comprise a substance derived from blood, such as plasma. Preparation of plasma from whole blood is easily accomplished by the person skilled in the art, such as by centrifugal removal of the cells present in whole blood.

Plasma can be obtained relatively easily and may reflect the sub-proteomes of other organs, including the brain. Both candidate protein panels and gel based proteomics have previously been used in plasma and serum to identify possible biomarkers with some success (Hye et al., 2006, Ray et al., 2007, Baranowska-Bik et al., 2008) but to the best of our knowledge non-gel based proteomics have not previously been used in the search for plasma markers in AD.

One of the problems with the proteomic analysis of blood plasma with mass spectrometry, is the huge dynamic range of plasma proteins. Protein levels span an extraordinary 10 orders of magnitude, which makes the investigation of low(er) abundant proteins nearly impossible (Anderson and Anderson, 2002, Jacobs et al., 2005). The instrumental settings in the LC/MS/MS, where the most prominent peaks in a short period of time are chosen for fragmentation, do not allow for the identification and quantitation of low abundant proteins in unfractionated plasma due to the high abundance of serum albumin and other proteins. This is reflected in a low number of proteins identified. One approach to reduce the dynamic range is to deplete samples of the highest abundant proteins and in this case we exemplify this approach using an immunoaffinity column to remove albumin, transferrin, IgG, IgA, antitrypsin, and haptoglobin. The number of identifiable and quantifiable proteins could be increased considerably and relative protein levels were compared between different samples.

Thus, more suitably, the sample according to the invention may be a processed plasma. For example, plasma may be processed to remove highly abundant proteins, and thereby to increase the number of detectable proteins, or to increase the detectability of proteins present in low absolute concentrations. Techniques for depletion of highly abundant proteins from plasma are well-known in the art. In particular, a multiple affinity removal system may conveniently be used to process plasma for analysis. Exemplary systems are described in the example section of this application.

Furthermore, the sample may suitably comprise plasma proteins. In this embodiment, plasma may be processed as described herein, and may then be subjected to size exclusion chromatography, buffer exchange, or other such treatments in order to arrive at a sample comprising the proteins from said plasma, which may offer advantages such as superior performance in analytical instruments.

The key principle for the properties of the sample, whichever particular form it takes, are that it is, or is derived from, blood.

### Reference Sample

The reference sample is suitably a sample from a subject that is not suffering from or suspected of suffering from AD. More suitably the reference sample is from a healthy subject. Ideally this is processed and analysed in the same manner as the sample being analysed. However, this may not be practical or desirable in which case the reference sample may be regarded as a reference value previously determined for a healthy subject, such as an abundance or concentration of (e.g.) gelsolin for a normal healthy individual. Ideally the reference sample or value is gender-matched and suitably age-matched, more suitably matched for genetic or ethnic background or other such criteria as are routinely applied in matching of clinical samples to controls, and insofar as the levels of the relevant biomarker in plasma are dependent on such factors. Suitably the reference sample may be an earlier sample taken from the same subject before the onset of Alzheimer's disease.

### Detection

A marker protein may have its expression modulated, i.e. quantitatively increased or decreased, in patients with Alzheimer's Disease. The degree to which expression differs in normal versus diseased states (or advanced versus early states) need only be large enough to be visualised via standard characterisation techniques, such as silver staining of 2D-electrophoretic gels, measurement of representative peptide ions using isobaric mass tagging and mass spectrometry or immunological detection methods including Western blotting, enzyme-linked immunosorbent assay (ELISA) or radioimmunoassay. Other such standard characterisation techniques by which expression differences may be visualised are well known to those skilled in the art. These include successive chromatographic separations of fractions and comparisons of the peaks, capillary electrophoresis, separations using micro-channel networks, including on a micro-chip, and mass spectrometry methods including multiple reaction monitoring (MRM) and TMTcalibrator (Dayton et al 2009).

Chromatographic separations can be carried out by high performance liquid chromatography as described in Pharmacia literature, the chromatogram being obtained in the form of a plot of absorbance of light at 280 nm against time of separation. The material giving incompletely resolved peaks is then rechromatographed and so on.

Capillary electrophoresis is a technique described in many publications, for example in the literature "Total CE Solutions" supplied by Beckman with their P/ACE 5000 system. The technique depends on applying an electric potential across the sample contained in a small capillary tube. The tube has a charged surface, such as negatively charged silicate glass. Oppositely charged ions (in this instance, positive ions) are attracted to the surface and then migrate to the appropriate electrode of the same polarity as the surface (in this instance, the cathode). In this electroosmotic flow (EOF) of the sample, the positive ions move fastest, followed by uncharged material and negatively charged ions. Thus, proteins are separated essentially according to charge on them.

Micro-channel networks function somewhat like capillaries and can be formed by photoablation of a polymeric material. In this technique, a UV laser is used to generate high energy light pulses that are fired in bursts onto polymers having suitable UV absorption characteristics, for example polyethylene terephthalate or polycarbonate. The incident photons break chemical bonds with a confined space, leading to a rise in internal pressure, mini-explosions and ejection of the ablated material, leaving behind voids which form micro-channels. The micro-channel material achieves a separation based on EOF, as for capillary electrophoresis. It is adaptable to micro-chip form, each chip having its own sample injector, separation column and electrochemical detector: see J.S.Rossier et al., 1999, Electrophoresis 20: pages 727-731.

Other methods include performing a binding assay for the marker protein. Any reasonably specific binding agent can be used. Preferably the binding agent is labelled. Preferably the assay is an immunoassay, especially between the biomarker and an antibody that recognises the protein, especially a labelled antibody. It can be an antibody raised against part or all of the marker protein, for example a monoclonal antibody or a polyclonal anti-human antiserum of high specificity for the marker protein.

Where the binding assay is an immunoassay, it may be carried out by measuring the extent of the protein/antibody interaction. Any known method of immunoassay may be used. A sandwich assay is preferred. In an exemplary sandwich assay, a first antibody to the marker protein is bound to the solid phase such as a well of a plastics microtitre plate, and incubated with the sample and with a labelled second antibody specific to the protein to be assayed. Alternatively, an antibody capture assay can be used. Here, the test sample is allowed to bind to a solid phase, and the anti-marker protein antibody is then added and allowed to bind. After washing away unbound material, the amount of antibody bound to the solid phase is determined using a labelled second antibody, anti- to the first.

In another embodiment, a competition assay is performed between the sample and a labelled marker protein or a peptide derived therefrom, these two antigens being in competition for a limited amount of anti-marker protein antibody bound to a solid support. The labelled marker protein or peptide thereof can be pre-incubated with the antibody on the solid phase, whereby the marker protein in the sample displaces part of the marker protein or peptide thereof bound to the antibody.

In yet another embodiment, the two antigens are allowed to compete in a single co-incubation with the antibody. After removal of unbound antigen from the support by washing, the amount of label attached to the support is determined and the amount of protein in the sample is measured by reference to standard titration curves established previously.

The binding agent in the binding assay may be a labelled specific binding agent, which may be an antibody or other specific binding agent. The binding agent will usually be labelled itself, but alternatively it may be detected by a secondary reaction in which a signal is generated, e.g. from another labelled substance.

The label may be an enzyme. The substrate for the enzyme may be, for example, colour-forming, fluorescent or chemiluminescent.

An amplified form of assay may be used, whereby an enhanced "signal" is produced from a relatively low level of protein to be detected. One particular form of amplified immunoassay is enhanced chemiluminescent assay. Conveniently, the antibody is labelled with horseradish peroxidase, which participates in a chemiluminescent reaction with luminol, a peroxide substrate and a compound which enhances the intensity and duration of the emitted light, typically 4-iodophenol or 4-hydroxycinnamic acid.

Another form of amplified immunoassay is immuno-PCR. In this technique, the antibody is covalently linked to a molecule of arbitrary DNA comprising PCR primers, whereby the DNA with the antibody attached to it is amplified by the polymerase chain reaction. See E. R. Hendrickson et al., Nucleic Acids Research 23: 522-529 (1995). The signal is read out as before.

The time required for the assay may be reduced by use of a rapid microparticle-enhanced turbidimetric immunoassay such as the type embodied by M. Robers et al., "Development of a rapid microparticle-enhanced turbidimetric immunoassay for plasma fatty acid-binding protein, an early marker of acute myocardial infarction", Clin. Chem. 1998;44:1564-1567.

The full automation of any immunoassay contemplated in a widely used clinical chemistry analyser such as the COBAS™ MIRA Plus system from Hoffmann-La Roche, described by M.Robers et al. supra, or the AxSYM™ system from Abbott Laboratories, should be possible and applied for routine clinical diagnosis of Alzheimer's disease.

It is also contemplated within the invention to use (i) an antibody array or 'chip', or a bead suspension array capable of detecting one or more proteins that interact with that antibody.

An antibody chip, antibody array or antibody microarray is an array of unique addressable elements on a continuous solid surface whereby at each unique addressable element an antibody with defined specificity for an antigen is immobilised in a manner allowing its subsequent capture of the target antigen and subsequent detection of the extent of such binding. Each unique addressable element is spaced from all other unique addressable elements on the solid surface so that the binding and detection of specific antigens does not interfere with any adjacent such unique addressable element.

A "bead suspension array" is an aqueous suspension of one or more identifiably distinct particles whereby each particle contains coding features relating to its size and colour or fluorescent signature and to which all of the beads of a particular combination of such coding features is coated with an antibody with a defined specificity for an antigen in a manner allowing its subsequent capture of the target antigen and subsequent detection of the extent of such binding. Examples of such arrays can be found at www.luminexcorp.com where application of the xMAP® bead suspension array on the Luminex® 100™ System is described.

Alternatively, the diagnostic sample can be subjected to isobaric mass tagging and LC-MS/MS as described herein. An example of preferred ways of carrying out isobaric protein tagging are set out in the examples section of this application.

Isobaric protein tagging using tandem mass tags has been shown before to be able to determine relative proteins levels in a highly accurate manner (Thompson et al., 2003, Dayon et al., 2008). In addition, numerous reports have been published in the last few years using iTRAQ for protein tagging in various tissues and fluids (Aggarwal et al., 2006). Especially for the discovery of biomarkers in various conditions, iTRAQ has been proved to be a highly suitable tool and has been used in cancer (Maurya et al., 2007, Garbis et al., 2008, Matta et al., 2008, Ralhan et al., 2008) and diabetes research (Lu et al., 2008) as well as in the quest for biomarkers in neurodegenerative disorders (Abdi et al., 2006) albeit in CSF.

### Multiple Selected Reaction Monitoring (mSRM or MRM)

MRM is the scan type with the highest duty cycle and is used for monitoring one or more specific ion transition(s) at high sensitivity. Here, Q1 is set on the specific parent m/z (Q1 is not scanning), the collision energy is set to produce the optimal diagnostic charged fragment of that parent ion, and Q3 is set to the specific m/z of that fragment. Only ions with this exact transition will be detected. Historically used to quantify small molecules such as drug metabolites, the same principle can be applied to peptides, either endogenous moieties or those produced from enzymatic digestion of proteins. Again historically experiments were performed using triple quadrupole mass spectrometers but the recent introduction of hybrid instrument designs, which combine quadrupoles with ion traps, enables similar and improved experiments to be undertaken. The 4000QTRAP instrument therefore allows peptide and biomolecule quantitation to be performed at very high specificity and sensitivity using Multiple Reaction Monitoring (MRM). This is largely due to the use of the LINAC® Collision Cell, which subsequently enables many MRM scans to be looped together into one experiment to detect the presence of many specific ions (up to 100 different ions) in a complex mixture. Consequently it is now feasible to measure and quantify multiple peptides from many proteins in a single chromatographic separation. The area under the MRM LC peak is used to quantitate the amount of the analyte present. In a typical quantitation experiment, a standard concentration curve is generated for the analyte of interest. When the unknown sample is then run under identical conditions, the concentration for the analyte in the unknown sample can be determined using the peak area and the standard concentration curve.

The diagnostic sample can be subjected to analysis by MRM on an ion-trap mass spectrometer. Based on the mass spectrometry profiles of the marker proteins described below single tryptic peptides with specific known mass and amino acid sequences are identified that possess good ionising characteristics. The mass spectrometer is then programmed to specifically survey for peptides of the specific mass and sequence and report their relative signal intensity. Using MRM it is possible to survey for up to 5, 10, 15, 20, 25, 30, 40, 50 or 100 different marker proteins in a single LC-MS run. The intensities of the MRM peptides of the specific biomarkers of the present invention in the diagnostic sample are compared with those found in samples from subjects without AD allowing the diagnosis or prognosis of AD to be made.

The MRM assay can be made more truly quantitative by the use of internal reference standards consisting of synthetic absolute quantification (AQUA) peptides corresponding to the MRM peptide of the marker protein wherein one or more atoms have been substituted with a stable isotope such as carbon-13 or nitrogen-15 and wherein such substitutions cause the AQUA peptide to have a defined mass difference to the native, lighter form of the MRM peptide derived from the diagnostic sample. By comparing the relative ion intensity of the native MRM and AQUA peptides the true concentration of the parent protein in the diagnostic sample can thus be determined. General methods of absolute quantitation by such isotope dilution methods are provided in Gerber, Scott A, et al. "Absolute quantification of proteins and phosphoproteins from cell lysates by tandem MS" PNAS, June 10, 2003. Vol 100. No 12. p 6940-6945.

In some cases, whilst it is desirable to use isotope-doped standards to provide absolute quantitation in an SRM experiment it is not possible to use the AQUA approach described above. In such cases it is possible to use a pair of isotopic mass tags i.e. two tags with identical chemical structure but different levels of isotopic substitutions giving each a unique mass. Using two forms of the Tandem Mass Tags® (TMT®) that differ in mass by 5 Da it is possible to label standard synthetic reference SRM peptides with a light tag prior to mixing to form a universal reference for all targeted peptides in an assay. Each patient sample is then subjected to trypsin digestion and the resulting peptides labelled with the heavy TMT tag. An aliquot of the TMT-labelled reference peptides is then added to the sample to give a final concentration of reference peptides that is relevant to the target range to be measured in the patient sample. The spiked sample is then subjected to a standard isotope dilution SRM assay and the concentrations of the SRM peptides from the patient sample are calculated by comparing ion intensities of the heavy form against those of the known concentrations of the lighter form.

An alternative form of MS-based assay for the relative or absolute quantitation of regulated peptides identified as biomarker candidates is the TMTcalibrator method developed by Proteome Sciences plc, Known amounts of synthetic peptides representing tryptic fragments of the candidate biomarker(s) with good MS/MS behaviour are labelled with four of the six reagents of the TMT6 set of isobaric mass tags (TMT-128 to TMT-131) and mixed in certain ratios. This allows a multi-point calibration curve reflecting physiological and/or disease-modified concentrations to be designed and implemented quickly. Subsequently, a diagnostic sample taken from a patient suffering from or suspected of suffering from AD is labelled with TMT6-126 and the calibration mix is added to the study sample. During MS/MS of individual peptides, the TMT6-reporter ions of the calibrant peptides are produced and used to establish a calibration curve. The absolute amount of the peptide in the study sample is then readily derived by reading the TMT6126 ion intensity against the calibration curve. Further information on TMTcalibrator assays can be obtained from the Proteome Sciences website (www.proteomics.com).

A preferred method of diagnosis comprises performing a binding assay for the marker protein. Any reasonably specific binding partner can be used. Preferably the binding partner is labelled. Preferably the assay is an immunoassay, especially between the marker and an antibody that recognises the protein, especially a labelled antibody. It can be an antibody raised against part or all of it, most preferably a monoclonal antibody or a polyclonal anti-human antiserum of high specificity for the marker protein.

Thus, the marker proteins described above are useful for the purpose of raising antibodies thereto which can be used to detect the increased or decreased concentration of the marker proteins present in a diagnostic sample. Such antibodies can be raised by any of the methods well known in the immunodiagnostics field.

The antibodies may be anti- to any biologically relevant state of the protein. Thus, for example, they can be raised against the unglycosylated form of a protein which exists in the body in a glycosylated form, against a more mature form of a precursor protein, e.g. minus its signal sequence, or against a peptide carrying a relevant epitope of the marker protein.

The sample can be taken from any valid body tissue, especially body fluid, of a mammalian or non-mammalian subject, but preferably blood, plasma, serum or urine. Other usable body fluids include cerebrospinal fluid (CSF), semen and tears. Preferably the subject is a mammalian species such as a mouse, rat, guinea pig, dog or primate. Most preferably the subject is human.

The preferred immunoassay is carried out by measuring the extent of the protein/antibody interaction. Any known method of immunoassay may be used. A sandwich assay is preferred. In this method, a first antibody to the marker protein is bound to the solid phase such as a well of a plastic microtitre plate, and incubated with the sample and with a labelled second antibody specific to the protein to be assayed. Alternatively, an antibody capture assay can be used. Here, the test sample is allowed to bind to a solid phase, and the anti-marker protein antibody is then added and allowed to bind. After washing away unbound material, the amount of antibody bound to the solid phase is determined using a labelled second antibody, anti- to the first.

In another embodiment, a competition assay is performed between the sample and a labelled marker protein or a peptide derived therefrom, these two antigens being in competition for a limited amount of anti-marker protein antibody bound to a solid support. The labelled marker protein or peptide thereof can be pre-incubated with the antibody on the solid phase, whereby the marker protein in the sample displaces part of the marker protein or peptide thereof bound to the antibody.

In yet another embodiment, the two antigens are allowed to compete in a single co-incubation with the antibody. After removal of unbound antigen from the support by washing, the amount of label attached to the support is determined and the amount of protein in the sample is measured by reference to standard titration curves established previously.

The label is preferably an enzyme. The substrate for the enzyme may be, for example, colour-forming, fluorescent or chemiluminescent.

The binding partner in the binding assay is preferably a labelled specific binding partner, but not necessarily an antibody. The binding partner will usually be labelled itself, but alternatively it may be detected by a secondary reaction in which a signal is generated, e.g. from another labelled substance.

It is highly preferable to use an amplified form of assay, whereby an enhanced "signal" is produced from a relatively low level of protein to be detected. One particular form of amplified immunoassay is enhanced chemiluminescent assay. Conveniently, the antibody is labelled with horseradish peroxidase, which participates in a chemiluminescent reaction with luminol, a peroxide substrate and a compound which enhances the intensity and duration of the emitted light, typically 4-iodophenol or 4-hydroxycinnamic acid.

Another preferred form of amplified immunoassay is immuno-PCR. In this technique, the antibody is covalently linked to a molecule of arbitrary DNA comprising PCR primers, whereby the DNA with the antibody attached to it is amplified by the polymerase chain reaction. See E. R. Hendrickson et al., Nucleic Acids Research 23: 522-529 (1995). The signal is read out as before.

The use of a rapid microparticle-enhanced turbidimetric immunoassay such as the type embodied by M. Robers et al., "Development of a rapid microparticle-enhanced turbidimetric immunoassay for plasma fatty acid-binding protein, an early marker of acute myocardial infarction", Clin. Chem. 1998;44:1564-1567, significantly decreases the time of the assay. Thus, the full automation of any immunoassay contemplated in a widely used clinical chemistry analyser such as the COBAS™ MIRA Plus system from Hoffmann-La Roche, described by M.Robers et al. supra, or the AxSYM™ system from Abbott Laboratories, should be possible and applied for routine clinical diagnosis of Alzheimer's disease.

Alternatively, the diagnostic sample can be subjected to two dimensional gel electrophoresis to yield a stained gel in which the position of the marker proteins is known and the relative intensity of staining at the appropriate spots on the gel can be determined by densitometry and compared with a corresponding control or comparative gel.

In a yet further embodiment the diagnostic sample can be subjected to analysis by a mass-spectrometer-based assay such as multiple reaction monitoring (MRM) on a triple quadrupole mass spectrometer or on certain types of ion-trap mass spectrometer. For each differentially expressed protein it is possible to identify a set of tryptic peptides with specific known mass (parent mass) and amino acid sequence and which upon fragmentation release fragments of specific mass (fragment mass) that are unique to each protein. The detection of a fragment mass from a defined parent mass ion is known as a transition.

Identification of such proteotypic peptides can be made based on the mass spectrometry profiles of the differentially expressed proteins seen during biomarker discovery, or may be designed in silico using predictive algorithms known to the skilled practitioner. The mass spectrometer is then programmed to specifically survey only for the specific parent mass and fragment mass transitions selected for each protein and reports their relative signal intensity. Using MRM it is possible to survey for up to 5, 10, 15, 20, 25, 30, 40, 50 or 100 different marker proteins in a single LC-MS run. The relative abundances of the proteotypic peptides for each marker protein in the diagnostic sample are compared with those found in samples from subjects without dementia allowing the diagnosis of Alzheimer's disease to be made. Alternatively comparison may be made with levels of the proteins from earlier samples from the same patient thus allowing prognostic assessment of the stage and/or rate of progression of Alzheimer's disease in said patient.

In a further embodiment of the invention the MRM assay can be made more truly quantitative by the use of internal reference standards consisting of synthetic absolute quantification (AQUA) peptides corresponding to the proteotypic peptide of the marker protein wherein one or more atoms have been substituted with a stable isotope such as carbon-13 or nitrogen-15 and wherein such substitutions cause the AQUA peptide to have a defined mass difference to the native proteotypic peptide derived from the diagnostic sample. Once AQUA peptides equivalent to each proteotypic peptide from the differentially expressed biomarkers of Alzheimer's disease have been produced, they can be mixed to form a reference standard that is then spiked into the tryptic digest of the patient sample. The combined sample is then subjected to a programmed mass spectrometer-based assay where the intensity of the required transitions from the native and AQUA peptides is detected. By comparing the relative ion intensity of the native peptides from the sample and the spiked AQUA reference peptides the true concentration of the parent protein in the diagnostic sample can thus be determined. General methods of absolute quantitation are provided in Gerber, Scott A, et al. "Absolute quantification of proteins and phosphoproteins from cell lysates by tandem MS" PNAS, June 10, 2003. Vol 100. No 12. p 6940-6945.

In a yet further embodiment of the invention an absolute quantitation can be made by using a TMT-SRM assay. Standard synthetic reference SRM peptides corresponding to the prototypic peptide of the marker protein are labelled with a light TMT tag having no isotope substitutions (light tag) prior to mixing to form a universal reference for all marker proteins in an assay. Each patient sample is then subjected to trypsin digestion and the resulting peptides labelled with the TMT tag having five isotopic substitution (heavy tag). An aliquot of the light TMT-labelled reference peptides is then added to the heavy TMT-labelled sample to give a final concentration of reference peptides that is relevant to the target range to be measured in the patient sample. The spiked sample is then subjected to a standard isotope dilution SRM assay and the concentrations of the SRM peptides from the patient sample are calculated by comparing ion intensities of the heavy form against those of the known concentrations of the lighter form.

Irrespective of the method chosen for measurement of the marker protein, the diagnosis and prognosis of Alzheimer's disease does not necessarily require a step of comparison of the concentration of the marker protein(s) with a control or reference sample but can be carried out with reference to a pre-determined reference value known to represent the presence and/or stage of disease.

The invention can be used to determine the stage and/or rate of progression of dementia in Alzheimer's disease, if desired, with reference to results obtained earlier from the same patient or by reference to standard values that are considered typical of the stage or rate of progression of the disease. In this way, the invention can be used to determine whether, for example after treatment of the patient with a drug or candidate drug, the disease has progressed or not, or that the rate of disease progression has been modified. The result can lead to a prognosis of the outcome of the disease.

The disclosure further includes the use for a diagnostic (and thus possibly prognostic) or therapeutic purpose of a partner material which recognises, binds to or has affinity for a marker protein specified above. Thus, for example, antibodies to the marker proteins, appropriately humanised where necessary, may be used in treatment. The partner material will usually be an antibody and used in any assay-compatible format, conveniently an immobilised format, e.g. as beads or a chip. Either the partner material will be labelled or it will be capable of interacting with a label.

The disclosure further includes a kit for use in a method of diagnosis and prognostic monitoring of Alzheimer's disease, which comprises a partner material, as described above, in an assay-compatible format, as described above, for interaction with a marker protein present in the diagnostic sample.

It is further contemplated within the invention to use (i) an antibody chip or array of chips, or a bead suspension array capable of detecting one or more proteins differentially expressed in Alzheimer's disease.

The method may further comprise determining an effective therapy for treating Alzheimer's disease.

In a further aspect, the present disclosure provides a method of treatment by the use of an agent that will restore the expression of one or more differentially expressed proteins in the Alzheimer's disease state towards that found in the normal state in order to prevent the development or progression of Alzheimer's disease. Preferably, the expression of the protein is restored to that of the normal state.

In a further aspect, the present disclosure provides a method whereby the pattern of differentially expressed proteins in a tissue sample or body fluid sample of an individual with Alzheimer's disease is used to predict the most appropriate and effective therapy to alleviate the Alzheimer's disease.

Also provided is a method of screening an agent to determine its usefulness in treating Alzheimer's disease, the method comprising:
(a) obtaining a sample of relevant tissue taken from, or representative of, a subject having Alzheimer's disease symptoms, who or which has been treated with the agent being screened;
(b) determining the presence, absence or degree of expression of the differentially expressed protein or proteins in the tissue from, or representative of, the treated subject; and,
(c) selecting or rejecting the agent according to the extent to which it changes the expression, activity or amount of the differentially expressed protein or proteins in the treated subject having Alzheimer's disease symptoms.

Preferably, the agent is selected if it converts the expression of the differentially expressed protein towards that of a normal subject. More preferably, the agent is selected if it converts the expression of the protein or proteins to that of the normal subject.

Also provided is a method of screening an agent to determine its usefulness in treating Alzheimer's disease, the method comprising:
(a) obtaining over time samples of relevant tissue or body fluid taken from, or representative of, a subject having Alzheimer's disease symptoms, who or which has been treated with the agent being screened;
(b) determining the presence, absence or degree of expression of a differentially expressed protein or proteins in said samples; and,
(c) determining whether the agent affects the change over time in the expression of the differentially expression protein in the treated subject having Alzheimer's disease symptoms.

Samples taken over time may be taken at intervals of weeks, months or years. For example, samples may be taken at monthly, two-monthly, three-monthly, four-monthly, six-monthly, eight-monthly or twelve-monthly intervals.

A change in expression over time may be an increase or decrease in expression, compared to the initial level of expression in samples from the subject and/or compared to the level of expression in samples from normal subjects. The agent is selected if it slows or stops the change of expression over time.

In the screening methods described above, subjects having differential levels of protein expression comprise:
(a) normal subjects and subjects having Alzheimer's disease; and,
(b) subjects having Alzheimer's disease symptoms which have not been treated with the agent and subjects having Alzheimer's disease which have been treated with the agent.

### Diagnosis and prognosis

The term "diagnosis", as used herein, includes the provision of any information concerning the existence, non-existence or probability of AD in a patient. It further includes the provision of information concerning the type or classification of the disorder or of symptoms which are or may be experienced in connection with it. It encompasses prognosis of the medical course of the condition. It further encompasses information concerning the age of onset.

The methods described herein are useful for both the diagnosis and/or prognosis of AD. AD may be indicated if one or more markers is present at increased or decreased concentration.

### Treatment

It will be understood that where treatment is concerned, treatment includes any measure taken by the physician to alleviate the effect of AD on a patient. Thus, although reversal of the damage or elimination of the damage or effects of AD is a desirable goal, effective treatment will also include any measures capable of achieving reduction in the degree of damage or severity of the effects or progression.

The present disclosure may also provide a method of treatment by the use of an agent that will restore the expression of one or more differentially expressed proteins in the AD state towards that found in the normal state in order to prevent the development or progression of AD. Preferably, the expression of the protein is restored to that of the normal state.

The present disclosure may also provide a method whereby the pattern of differentially expressed proteins in a sample from an individual with AD is used to predict the most appropriate and effective therapy to alleviate the neurological damage and/or dementia.

### Assay methods

Also provided is a method of screening an agent to determine its usefulness in treating AD, the method comprising:
(a) obtaining a sample from, or representative of, a subject having AD, who or which has been treated with the agent being screened;
(b) determining the presence, absence or degree of expression of a marker protein or proteins as disclosed herein in the sample from, or representative of, the treated subject; and,
(c) selecting or rejecting the agent according to the extent to which it changes the expression, activity or amount of the marker protein or proteins in the treated subject having symptoms of AD.

Preferably, the agent is selected if it converts the expression of the differentially expressed protein towards that of a normal subject. More preferably, the agent is selected if it converts the expression of the protein or proteins to that of the normal subject.

Also provided is a method of screening an agent to determine its usefulness in treating AD, the method comprising:
(a) obtaining over time samples from, or representative of, a subject having AD symptoms, who or which has been treated with the agent being screened;
(b) determining the presence, absence or degree of expression of a marker protein or proteins as disclosed herein in said samples; and,
(c) determining whether the agent affects the change over time in the expression of the marker protein in the treated subject having AD symptoms.

Samples taken over time may be taken at intervals of weeks, months or years. For example, samples may be taken at monthly, two-monthly, three-monthly, four-monthly, six-monthly, eight-monthly or twelve-monthly intervals.

A change in expression over time may be an increase or decrease in expression, compared to the initial level of expression in samples from the subject and/or compared to the level of expression in samples from normal subjects. The agent is selected if it slows or stops the change of expression over time.

In the screening methods described above, subjects having differential levels of protein expression comprise:
(a) normal subjects and subjects having AD symptoms; and,
(b) subjects having AD symptoms which have not been treated with the agent and subjects having AD symptoms which have been treated with the agent.

### Antibodies

Antibodies against the marker proteins disclosed herein can be produced using known methods. These methods of producing antibodies include immunising a mammal (e.g. mouse, rat, rabbit, horse, goat, sheep or monkey) with the protein. Antibodies may be obtained from immunised animals using any of a variety of techniques known in the art, and screened, preferably using binding of antibody to antigen of interest. Isolation of antibodies and/or antibody-producing cells from an animal may be accompanied by a step of sacrificing the animal.

As an alternative or supplement to immunising a mammal with a protein, an antibody specific for the protein may be obtained from a recombinantly produced library of expressed immunoglobulin variable domains, e.g. using lambda bacteriophage or filamentous bacteriophage which display functional immunoglobulin binding domains on their surfaces; for instance see WO92/01047. The library may be naive, that is constructed from sequences obtained from an organism which has not been immunised with the protein, or may be one constructed using sequences obtained from an organism which has been exposed to the antigen of interest.

The antibodies may bind or be raised against any biologically relevant state of the protein. Thus, for example, they can be raised against the unglycosylated form of a protein which exists in the body in a glycosylated form, against a more mature form of a precursor protein, e.g. minus its signal sequence, or against a peptide carrying a relevant epitope of the marker protein.

Antibodies may be polyclonal or monoclonal, and may be multispecific (including bispecific), chimeric or humanised antibodies. Antibodies used in the present invention may be modified in a number of ways. Indeed the term "antibody" should be construed as covering any binding substance having a binding domain with the required specificity. Thus, the disclosure covers antibody fragments, derivatives, functional equivalents and homologues of antibodies, including synthetic molecules and molecules whose shape mimics that of an antibody enabling it to bind an antigen or epitope.

Examples of antibody fragments, capable of binding an antigen or other binding partner, are the Fab fragment consisting of the VL, VH, CI and CH1 domains; the Fd fragment consisting of the VH and CH1 domains; the Fv fragment consisting of the VL and VH domains of a single arm of an antibody; the dAb fragment which consists of a VH domain; isolated CDR regions and F(ab')2 fragments, a bivalent fragment including two Fab fragments linked by a disulphide bridge at the hinge region. Single chain Fv fragments are also included.

Antibody fragments, which recognise specific epitopes, may be generated by known techniques. For example, such fragments include, but are not limited to, the F(ab')2 fragments which can be produced by pepsin digestion of the antibody molecule and the Fab fragments which can be generated by reducing the disulfide bridges of the F(ab')2 fragments. Alternative, Fab expression libraries may be constructed (Huse, et al., 1989, Science 246: 1275-1281) to allow rapid and easy identification of monoclonal Fab fragments with the desired specificity.

The term "monoclonal antibody" as used herein refers to an antibody obtained from a substantially homogenous population of antibodies, i.e. the individual antibodies comprising the population are identical apart from possible naturally occurring mutations that may be present in minor amounts. Monoclonal antibodies can be produced by the method first described by Kohler and Milstein, Nature, 256:495, 1975 or may be made by recombinant methods, see Cabilly et al, US Patent No. 4,816,567, or Mage and Lamoyi in Monoclonal Antibody Production Techniques and Applications, pages 79-97, Marcel Dekker Inc, New York, 1987.

In the hybridoma method, a mouse or other appropriate host animal is immunised with the antigen by subcutaneous, intraperitoneal, or intramuscular routes to elicit lymphocytes that produce or are capable of producing antibodies that will specifically bind to the nanoparticles used for immunisation. Alternatively, lymphocytes may be immunised in vitro. Lymphocytes then are fused with myeloma cells using a suitable fusing agent, such as polyethylene glycol, to form a hybridoma cell, see Goding, Monoclonal Antibodies: Principles and Practice, pp. 59-103 (Academic Press, 1986).

The hybridoma cells thus prepared can be seeded and grown in a suitable culture medium that preferably contains one or more substances that inhibit the growth or survival of the unfused, parental myeloma cells. For example, if the parental myeloma cells lack the enzyme hypoxanthine guanine phosphoribosyl transferase (HGPRT or HPRT), the culture medium for the hybridomas typically will include hypoxanthine, aminopterin, and thymidine (HAT medium), which substances prevent the growth of HGPRT-deficient cells.

Preferred myeloma cells are those that fuse efficiently, support stable high level expression of antibody by the selected antibody producing cells, and are sensitive to a medium such as HAT medium.

Culture medium in which hybridoma cells are growing is assayed for production of monoclonal antibodies directed against the protein. Preferably, the binding specificity is determined by enzyme-linked immunoabsorbance assay (ELISA). The monoclonal antibodies used in the invention are those that specifically bind to the protein.

In a preferred embodiment of the disclosure, the monoclonal antibody will have an affinity which is greater than micromolar or greater affinity (i.e. an affinity greater than 10-6 mol) as determined, for example, by Scatchard analysis, see Munson & Pollard, Anal. Biochem., 107:220, 1980.

After hybridoma cells are identified that produce neutralising antibodies of the desired specificity and affinity, the clones can be subcloned by limiting dilution procedures and grown by standard methods. Suitable culture media for this purpose include Dulbecco's Modified Eagle's Medium or RPM1-1640 medium. In addition, the hybridoma cells may be grown in vivo as ascites tumours in an animal.

The monoclonal antibodies secreted by the subclones are suitably separated from the culture medium, ascites fluid, or serum by conventional immunoglobulin purification procedures such as, for example, protein A-Sepharose, hydroxylapatite chromatography, gel electrophoresis, dialysis, or affinity chromatography.

Nucleic acid encoding the monoclonal antibodies used in the invention is readily isolated and sequenced using procedures well known in the art, e.g. by using oligonucleotide probes that are capable of binding specifically to genes encoding the heavy and light chains of murine antibodies. The hybridoma cells used in the invention are a preferred source of nucleic acid encoding the antibodies or fragments thereof. Once isolated, the nucleic acid is ligated into expression or cloning vectors, which are then transfected into host cells, which can be cultured so that the monoclonal antibodies are produced in the recombinant host cell culture.

A hybridoma producing a monoclonal antibody according to the present disclosure may be subject to genetic mutation or other changes. It will further be understood by those skilled in the art that a monoclonal antibody can be subjected to the techniques of recombinant DNA technology to produce other antibodies, humanised antibodies or chimeric molecules which retain the specificity of the original antibody. Such techniques may involve introducing DNA encoding the immunoglobulin variable region, or the complementarity determining regions (CDRs), of an antibody to the constant regions, or constant regions plus framework regions, of a different immunoglobulin. See, for instance, EP 0 184 187 A, GB 2 188 638 A or EP 0 239 400 A. Cloning and expression of chimeric antibodies are described in EP 0 120 694 A and EP 0 125 023 A.

An antibody against a marker protein described herein will bind to said protein. Preferably, said antibody specifically binds said protein. By "specific" is meant that the antibody binds to said protein with an affinity significantly higher than it displays for other molecules.

### Detailed Description of the Invention

Alzheimer's disease (AD) is a progressive neurodegenerative disorder, where definite diagnosis can only be made post-mortem and where the most promising biomarkers so far are found in cerebrospinal fluid (CSF). A biomarker in blood, more accessible than CSF, has industrial application and utility in aiding diagnosis, as well as in population screening applications.

Differences in plasma proteins may exist between AD patients and non-demented controls (NDC). In the examples, we used isobaric mass tagging to compare the plasma protein levels in slow and fast declining AD patients, as well as in NDC subjects in a carefully designed shotgun proteomic approach. Plasma samples were matched for age, gender and cognitive decline (change in MMSE score per year) and then pooled for analysis. Subsequent relative quantification and statistical analysis generated a list of candidate proteins able to distinguish AD from NDC groups. Selected proteins were validated by Western blot analysis in a larger sample set of 90 probable AD and 50 NDC subjects in total. In this cohort, AD patients displayed significantly lower plasma Gelsolin levels compared to NDC subjects. In addition, Gelsolin levels correlated with disease progression rate and were significantly different in slow and fast declining AD patients. Further, C1 protease inhibitor levels were found to be associated with APOE ε4 genotype and lower Ceruloplasmin levels correlated with an earlier age of onset. Gelsolin is, due to its association with progression rate in AD, as well as due to its reported interaction with amyloid β (Aβ) a robust marker of AD. Moreover, gelsolin may advantageously be further included in a panel of biomarkers, for example for use in the monitoring of treatment response or clinical drug trials.

Thus, we disclose detection of gelsolin as a surrogate marker for progression in Alzheimer's disease. In the examples, this is demonstrated using isobaric protein tagging. This is an exemplary method of detection, but in principle any suitable method of detection may be employed. The particular advantages of this specific technique are demonstrated more fully below and in the examples section.

In addition, it is a key teaching that lower plasma Gelsolin levels are found in Alzheimer's disease and show association with disease progression rate.

We demonstrate in some embodiments how a combination of isobaric mass tagging together with more conventional methods for validation can provide useful information in the development of a biomarker in AD.

Several proteins were found that either occur in different levels in plasma of AD compared to NDC and correlate with disease progression rate or that are associated with additional clinical parameters like ApoE genotype or age of onset.

### Definitions

The term "antibody" includes polyclonal antiserum, monoclonal antibodies, fragments of antibodies such as single chain and Fab fragments, and genetically engineered antibodies. The antibodies may be chimeric or of a single species.

The term "marker protein" or "biomarker" includes all biologically relevant forms of the protein identified, including post-translational modification. For example, the marker protein can be present in the body tissue in a glycosylated, phosphorylated, multimeric or precursor form.

The term "control" refers to a normal human subject, i.e. one not suffering from Alzheimer's disease.

The terminology "increased/decreased concentration....compared with a control sample" does not imply that a step of comparing is actually undertaken, since in many cases it will be obvious to the skilled practitioner that the concentration is abnormally high or low. Further, when the stages of AD are being monitored progressively, or when a course of treatment is being monitored, the comparison made can be with the concentration previously seen in the same subject at an earlier stage of progression of the disease, or at an earlier stage of treatment or before treatment has commenced. The term "diagnosis", as used herein, includes determining whether a patient has Alzheimer's disease and may also include determining the stage to which it has progressed (or regressed in the course of treatment). The diagnosis can serve as the basis of a prognosis as to the future outcome for the patient.

The term "valid body tissue" or "relevant tissue" means any tissue in which it may reasonably be expected that a marker protein would accumulate in relation to Alzheimer's disease. It may be a cerebrospinal fluid sample or a sample of blood or a blood derivative such as plasma or serum.

The term "antibody array" or "antibody microarray" means an array of unique addressable elements on a continuous solid surface whereby at each unique addressable element an antibody with defined specificity for an antigen is immobilised in a manner allowing its subsequent capture of the target antigen and subsequent detection of the extent of such binding. Each unique addressable element is spaced from all other unique addressable elements on the solid surface so that the binding and detection of specific antigens does not interfere with any adjacent such unique addressable element.

The term "bead suspension array" means an aqueous suspension of one or more identifiably distinct particles whereby each particle contains coding features relating to its size and colour or fluorescent signature and to which all of the beads of a particular combination of such coding features is coated with an antibody with a defined specificity for an antigen in a manner allowing its subsequent capture of the target antigen and subsequent detection of the extent of such binding. Examples of such arrays can be found at www.luminexcorp.com where application of the xMAP® bead suspension array on the Luminex® 100™ System is described.

The term "mass spectrometer-based assay" means a quantitative measurement of a target analyte using the method of multiple reaction monitoring on a triple quadrupole or ion trap mass spectrometer.

The term 'mutant' of a biomarker such as a polypeptide biomarker used in the invention should have its normal meaning in the art. Mutants are sometimes referred to as 'variants' or 'alleles'. The key is to detect biomarkers as have been set out herein. The biomarkers may possess individual variations in the form of mutations or allelic variants between individuals being studied. Therefore there may be some degree of deviation from the exemplary SEQ ID NOs provided herein. The SEQ ID NOs provided herein are to assist the skilled reader in identifying and working with the polypeptides/biomarkers of the disclosure and are not intended as a restricted and inflexible definition of the individual polypeptides being assayed. Thus minor sequence differences between the SEQ ID NOs provided and the actual sequences of the polypeptide biomarkers being detected will be expected within the boundaries of normal variation between subjects. This should not affect the working of the invention.

The term 'comprises' (comprise, comprising) should be understood to have its normal meaning in the art, i.e. that the stated feature or group of features is included, but that the term does not exclude any other stated feature or group of features from also being present.

### Fragments/Peptides

It will be appreciated by the skilled worker that the details of the biomarkers discussed herein and in particular the sequences presented for them are given to facilitate their detection. The important information being gathered is the presence or absence (or particular level) of the biomarker in the sample being studied. There is no particular requirement that the full length polypeptide be scored. Indeed, via many of the suitable mass spectrometry based modes of detection set out herein, detection takes place by assaying particular fragments of the polypeptide of interest being present which are thus taken to indicate the presence of the overall biomarker polypeptide in the sample. Therefore the invention embraces the detection of fragments of the polypeptide biomarkers. Moreover, the kits and peptides of the disclosure may comprise fragments of the polypeptides and need not comprise the full length sequences exemplified herein. Suitably the fragment is sufficiently long to enable its unique identification by mass spectrometry.

Thus a fragment is suitably at least 6 amino acids in length, suitably at least 7 amino acids in length, suitably at least 8 amino acids in length, suitably at least 9 amino acids in length, suitably at least 10 amino acids in length, suitably at least 15 amino acids, suitably at least 25 amino acids, suitably at least 50 amino acids, suitably at least 100 amino acids, or suitably the majority of the biomarker polypeptide of interest. Suitably a fragment comprises a small fragment of the biomarker polypeptide of interest, whilst being long enough to retain an identifiable mass.

### Sequence Homology/Identity

Although sequence homology can also be considered in terms of functional similarity (i.e., amino acid residues having similar chemical properties/functions), in the context of the present document it is preferred to express homology in terms of sequence identity. Sequence comparisons can be conducted by eye or, more usually, with the aid of readily available sequence comparison programs. These publicly and commercially available computer programs can calculate percent homology (such as percent identity) between two or more sequences.

Percent identity may be calculated over contiguous sequences, i.e., one sequence is aligned with the other sequence and each amino acid in one sequence is directly compared with the corresponding amino acid in the other sequence, one residue at a time. This is called an "ungapped" alignment. Typically, such ungapped alignments are performed only over a relatively short number of residues (for example less than 50 contiguous amino acids). For comparison over longer sequences, gap scoring is used to produce an optimal alignment to accurately reflect identity levels in related sequences having insertion(s) or deletion(s) relative to one another. A suitable computer program for carrying out such an alignment is the GCG Wisconsin Bestfit package (University of Wisconsin, U.S.A; Devereux et al., 1984, Nucleic Acids Research 12:387). Examples of other software than can perform sequence comparisons include, but are not limited to, the BLAST package, FASTA (Altschul et al., 1990, J. Mol. Biol. 215:403-410) and the GENEWORKS suite of comparison tools.

In the context of the present document, a homologous amino acid sequence is taken to include an amino acid sequence which is at least 40, 50, 60, 70, 80 or 90% identical. Most suitably a polypeptide having at least 90% sequence identity to the biomarker of interest will be taken as indicative of the presence of that biomarker; more suitably a polypeptide which is 95% or more suitably 98% identical at the amino acid level will be taken to indicate presence of that biomarker. Suitably said comparison is made over at least the length of the polypeptide or fragment which is being assayed to determine the presence or absence of the biomarker of interest. Most suitably the comparison is made across the full length of the polypeptide of interest. The same considerations apply to nucleic acid nucleotide sequences.

### Alzheimer's disease

Alzheimer's disease (AD) is the most common neurodegenerative disorder and affects more than one in eight people over the age of 65 (Blennow et al., 2006). The disease has major financial and other burdens for national health systems and a biomarker to aid early diagnosis or the monitoring of disease progression would be of great value for the development of new treatments and in clinical practice. Considerable progress in the search for a biomarkers has been made with markers derived from the well known pathological lesions - amyloid beta (Aβ) plaques (Glenner et al., 1984) and neurofibrillary tangles (Lee and Trojanowski, 1992) - using a variety of techniques to investigate biochemical changes in the cerebrospinal fluid (CSF), blood and other tissues and fluids. The most promising sources for biomarkers in AD are the CSF or blood plasma, because compared to brain tissue, these fluids are more easily accessible and, in the case of CSF, in close contact with the central nervous system (CNS), where key biochemical changes take place. However, obtaining CSF through lumbar puncture is a relatively invasive procedure and having a biomarker from blood at hand would represent a significant advance. The inventors thus focussed on this aim.

The identification of biomarkers for AD may be addressed using the profiling of blood and CSF samples with highly sensitive methods in order to identify marker(s), i.e. proteins, peptides or metabolites, able to distinguish AD subjects and controls or to provide information about disease progression or response to treatment. Mass spectrometric methods are highly sensitive and therefore suitable for the identification of markers in all kinds of conditions and several reports have been published which used so called shotgun proteomics with isobaric tags to analyse the proteome of CSF samples (Abdi et al., 2006, Choe et al., 2007, Dayon et al., 2008) and blood samples (Hergenroeder et al., 2008) to find altered protein levels in particular diseases or disease stages.

The inventors carried out the comparison of blood from slow declining (SND) and fast declining (FD) AD patients with non-demented control (NDC) subjects. We aimed to identify 1) protein changes between SND and FD patients for disease progression markers and 2) changes between AD patients and NDC to determine proteins, characteristic for the disease pattern. The examples section presents data from samples which were investigated using isobaric protein tagging and mass spectrometry. Changes were further examined and validated by immunoblotting in a larger dataset.

### Alternate Methods

It will be understood by the skilled reader that specific techniques exemplified herein may be varied if desired using readily available alternatives to achieve the same effect. For example, assay of the gelsolin levels in a blood sample may be carried out by western blot or by isobaric protein tagging or by ELISA or by any other suitable means known in the art.

Thus, in some embodiments the disclosure relates to a method comprising:
a) providing a sample of blood, or a sample comprising protein derived from blood, from a subject
b) optionally extracting the plasma from said blood
c) optionally processing said blood or plasma to produce a sample comprising protein derived from said blood or plasma
d) optionally depleting abundant protein(s) from said blood or plasma
e) optionally size-selecting the proteins from said blood or plasma
f) optionally stabilising the proteins from said blood or plasma
g) optionally concentrating the proteins from said blood or plasma
h) optionally adjusting the buffering of the proteins from said blood or plasma
i) assaying gelsolin in said sample,
j) said assay is suitably determining the concentration or abundance of said biomarker, such as by isobaric protein tagging,
k) comparing the concentration or abundance of said biomarker in the sample from the subject to the concentration or abundance of said biomarker in a sample from a healthy subject, or to a reference sample/value.

Wherein any difference(s) identified in (k) indicate the outcomes set out herein, for example detecting reduced gelsolin levels compared to a sample from a healthy subject or a reference sample indicates increased likelihood of the subject having Alzheimer's disease.

Suitably the sample is provided *in vitro.* The methods of the invention are carried out *in vitro.* The collection of the sample is not a step of the method of the invention. In this way, the invention is not practised directly on the human or animal body.

### Further Applications

Various assay devices, kits or materials which recognise, bind to or have affinity for a polypeptide are described. The polypeptide(s) may comprise Gelsolin (Swiss prot accession number P06396; SEQ ID NO: 1). The polypeptide(s) may comprise one or more proteins selected from
C1 protease inhibitor (SEQ ID NO: 2)
ceruloplasmin (SEQ ID NO: 3)
clusterin (SwissProt accession number P10909; SEQ ID NO:4)
complement c3 (P01024; SEQ ID NO:5)
serum amyloid P component (P02743; SAP; SEQ ID NO:6)
alpha-2-macroglobulin (P01023; A2M; SEQ ID NO:7)
gamma-fibrinogen (P02679; SEQ ID NO:8)
complement factor H (P08603; CFH; SEQ ID NO:9)
apolipoprotein E (SEQ ID NO:10).

### Peptides

For any given polypeptide or set of polypeptides being detected by mass spectrometry based assay, the assay may be conducted via MRM techniques mentioned herein. In this embodiment, certain unique peptides and in particular certain transitions are especially advantageous to detect the peptides of interest. These are typically selected to give the highest representation (or combinations may be used such as any or all peptides giving a particular level of representation if multiple fragments/transitions give similar levels). Especially preferred transitions used for monitoring are those mentioned in the accompanying examples and/or figures.

In particular, certain peptides find utility as standards and/or controls in performing assays according to the invention. For example the following peptides are particularly useful in aiding detection of polypeptides mentioned herein:

**TABLE A**

| **I.D.** | **Protein** | **Peptide** | **SEQ ID NO:** |
|---|---|---|---|
| 1 | clusterin | TLLSNLEEAK | SEQ ID NO:11 |
| 3* | clusterin | IDSLLENDR | SEQ ID NO:12 |
| 5 | clusterin | ALQEYR | SEQ ID NO:13 |
| 6* | clusterin | YNELLK | SEQ ID NO:14 |
| 8 | complement c3 | FYYIYNEK | SEQ ID NO:15 |
| 9 | complement c3 | LVAYYTLIGASGQR | SEQ ID NO:16 |
| 11* | CFH | SPDVINGSPISQK | SEQ ID NO:17 |
| 12 | CFH | IDVHLVPDR | SEQ ID NO:18 |
| 13 | CFH | VGEVLK | SEQ ID NO:19 |
| 14 | alpha-2-m | AIGYLNTGYQR | SEQ ID NO:20 |
| 15 | alpha-2-m | TGTHGLLVK | SEQ ID NO:21 |
| 18* | gamma-fibrinogen | YLQEIYNSNNQK | SEQ ID NO:22 |
| 19 | gamma-fibrinogen | LDGSVDFK | SEQ ID NO:23 |
| 20 | gamma-fibrinogen | VGPEADK | SEQ ID NO:24 |
| 22 | SAP | VGEYSLYIGR | SEQ ID NO:25 |
| 23 | SAP | AYSLFSYNTQGR | SEQ ID NO:26 |
| 24 | apoE | LGPLVEQGR | SEQ ID NO:27 |
| 25 | apoE | LQAEAFQAR | SEQ ID NO:28 |
| 27* | gelsolin | QTQVSVLPEGGETPLFK | SEQ ID NO:29 |
| 29 | gelsolin | TASDFITK | SEQ ID NO:30 |
| 30 | gelsolin | AVEVLPK | SEQ ID NO:31 |
| 31 | gelsolin | HVVPNEVVVQR | SEQ ID NO:32 |
| * | asterisk indicates less preferred peptides for MS | | |

Especially suitable are:

**TABLE B**

| **I.D.** | **Protein** | **Peptide** | **SEQ ID NO:** |
|---|---|---|---|
| 1 | clusterin | TLLSNLEEAK | SEQ ID NO:11 |
| 5 | clusterin | ALQEYR | SEQ ID NO:13 |
| 8 | complement c3 | FYYIYNEK | SEQ ID NO:15 |
| 9 | complement c3 | LVAYYTLIGASGQR | SEQ ID NO:16 |
| 12 | CFH | IDVHLVPDR | SEQ ID NO:18 |
| 13 | CFH | VGEVLK | SEQ ID NO:19 |
| 14 | alpha-2-m | AIGYLNTGYQR | SEQ ID NO:20 |
| 15 | alpha-2-m | TGTHGLLVK | SEQ ID NO:21 |
| 19 | gamma-fibrinogen | LDGSVDFK | SEQ ID NO:23 |
| 20 | gamma-fibrinogen | VGPEADK | SEQ ID NO:24 |
| 22 | SAP | VGEYSLYIGR | SEQ ID NO:25 |
| 23 | SAP | AYSLFSYNTQGR | SEQ ID NO:26 |
| 24 | apoE | LGPLVEQGR | SEQ ID NO:27 |
| 25 | apoE | LQAEAFQAR | SEQ ID NO:28 |
| 29 | gelsolin | TASDFITK | SEQ ID NO:30 |
| 30 | gelsolin | AVEVLPK | SEQ ID NO:31 |
| 31 | gelsolin | HVVPNEVVVQR | SEQ ID NO:32 |

The peptides in this table each have the advantage of excellent performance in the mass spectrometry assays set out herein. Most preferred are SEQ ID NOs: 30, 31 and 32, which are for detection of gelsolin.

The application is now described by way of example, which examples are intended to be illustrative in nature and not to be understood as limiting the appended claims. In the examples, reference is made to the following figures:

### Brief Description of the Figures

Figure 1 shows plots.
Figure 2 shows a graph and some bar charts.
Figure 3A shows a diagram and some graphs. In particular this shows a general workflow diagram for proteomic analysis using isobaric protein tagging. The steps shown are Differential sample preparation e.g. plasma samples; In-solution trypsin digestion; Label individually and combine into one single sample; RP / SCX purification; LC/MS/MS; Search data and compare with databases; Identify and quantify proteins.
Figure 3B shows Table 1.
Figure 4 shows a chart, a graph and a blot. Western blot analysis of Gelsolin in human plasma from AD and NDC subjects. A) (Gelsolin) Box Plot shows a decrease of Gelsolin in AD (p=0.001), but B) (Sensitivity) ROC analysis with Gelsolin did not show favourable test characteristics. Figure 5 shows a table of MS transitions useful for measurement of Gelsolin in an MRM assay.

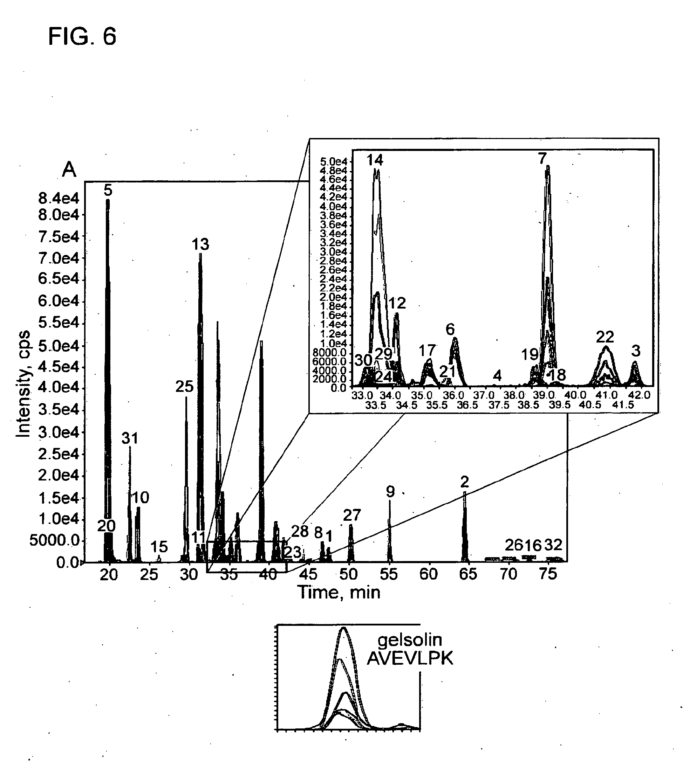

Figure 6 shows a total ion chromatogram of 192 transitions representing 32 peptide biomarkers of AD. Transitions relating to Gelsolin are found in Peak Nos. 27 - 32. (A) An SRM XIC for TMTzero- and TMTsixplex-labeled plasma peptides 1-32. All transitions listed in Table 1 were assessed. It can be seen that TMTzero- and TMTsixplex-labeled peptide pairs co-elute. (B (inset)) MRM XIC showing the peptides which elute over the busiest part of the LC gradient (32min - 42min). (C (graph below marked 'gelsolin AVEVLPK')) MRM XIC for peptide AVEVLPK of gelsolin. TMTzero transitions for each peptide are coloured in blue, red and green, while their TMTsixplex labelled counterparts are coloured in grey (2^{nd} uppermost), cyan (uppermost) and pink (lowermost).

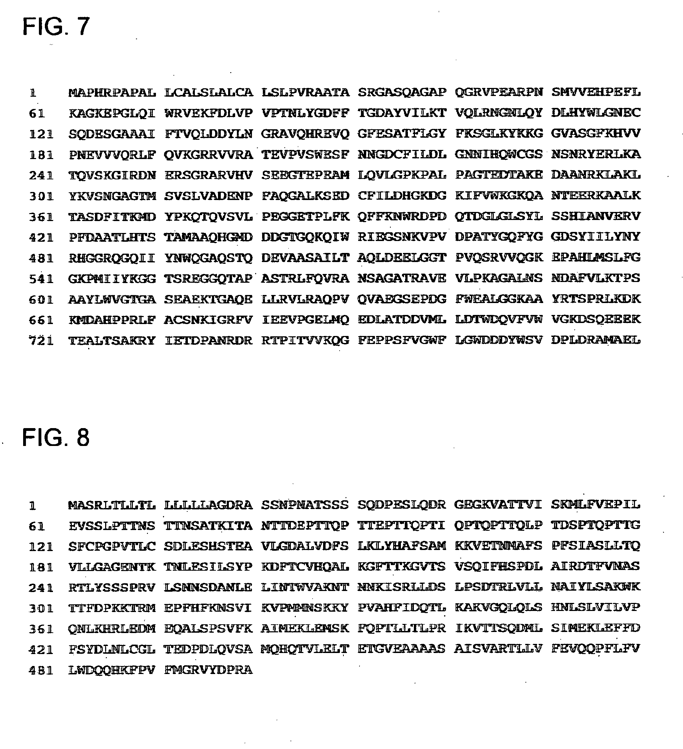

Figure 7 shows the sequence of human gelsolin. It should be noted that certain isoforms may differ very slightly in sequence, for example Unigene accession number IPI00026314 (ISOFORM 1 OF GELSOLIN) has 782 amino acid residues (having the sequence of figure 7 plus a further two alanine residues at 781 and 782). In case of any doubt, suitably SEQ ID NO: 1 should be taken as the reference sequence of gelsolin (Swiss Prot P06396).
Figure 8 shows the sequence of human C1 protease inhibitor (SEQ. ID. NO: 2)
Figure 9 shows the sequence of human ceruloplasmin (SEQ. ID. NO: 3)
Figures 10 to 17 show data and bar charts
Figure 18 shows an overview of the experiment. Ten disease and ten control samples were selected for each protein. Three digests were performed on each sample (technical digests) followed by three analytical measurements of each digest. Each protein had approximately three peptides for quantitation, which was determined from three transition pairs per peptide. This resulted in approximately 3240 measurements for each individual protein.

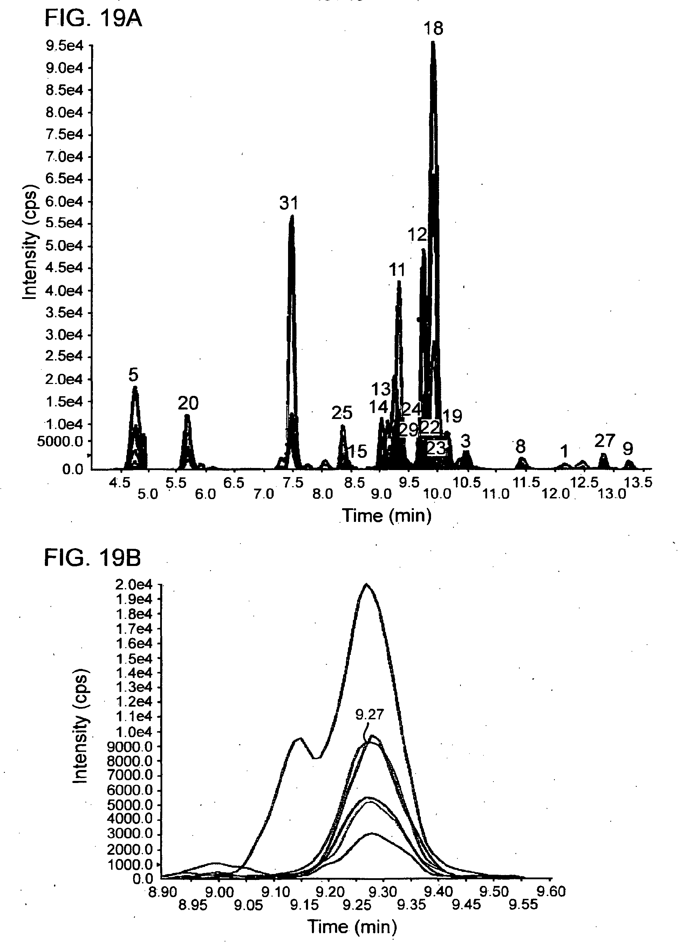

Figure 19A shows an extracted ion chromatogram (XIC) of peptides, light and heavy TMT-labeled. The light labeled sample represents the peptide endogenous to plasma and the heavy labeled sample represents the peptide internal standard. Transitions relating to gelsolin are found in peak nos. 27, 29, 30 and 31. Following poor performance and interference by plasma background, peptides 3, 6, 11, 18 and 27 were removed from the analysis.
Figure 19B shows an XIC of the light TMT and heavy TMT for peptide 13. One transition has been affected by high plasma background (coloured in pink; heavy TMT-labeled) and subsequently, this transition and its corresponding light-TMT partner was removed from the analysis.

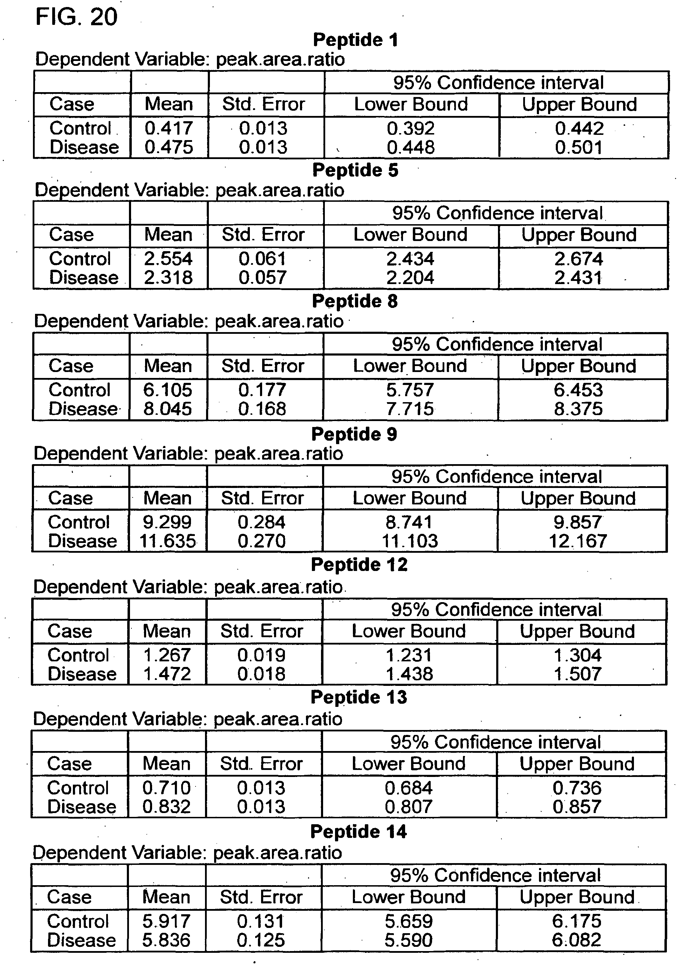

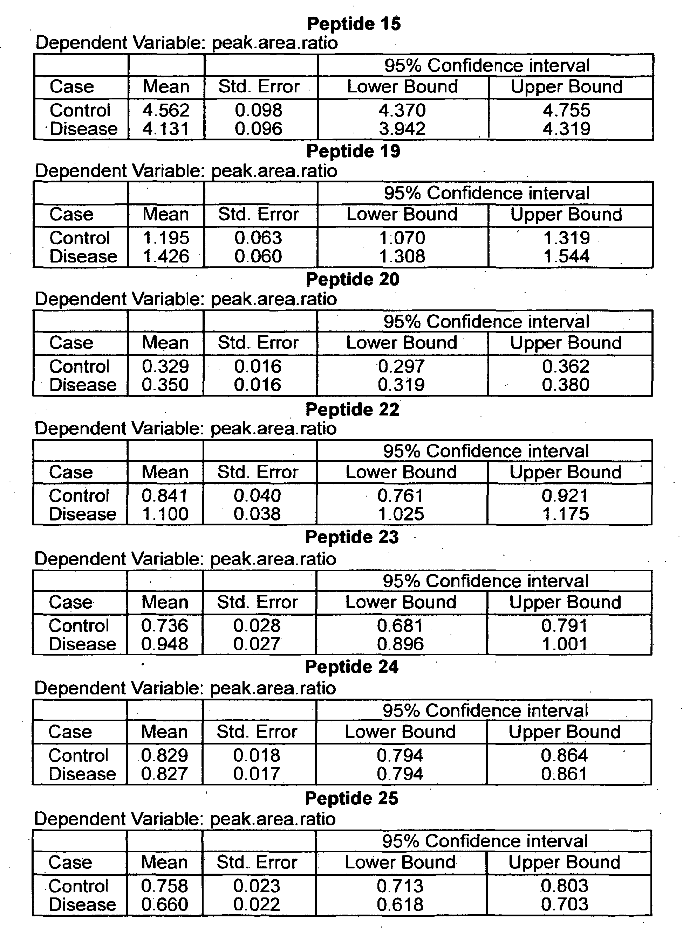

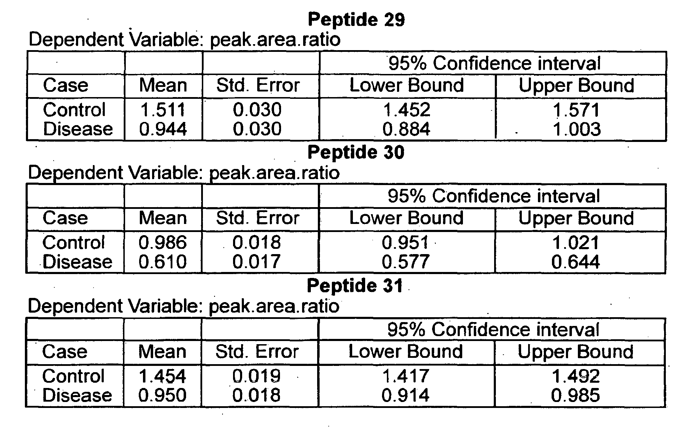

Figure 20 shows tables of data (95%CI) relating to figures 10 to 17.

### Examples

### Overview

Recent studies indicate that differences in plasma proteins levels may exist between AD patients and non-demented controls (NDC). In the current study, we used isobaric mass tagging to compare the plasma protein levels in 30 probable AD and 15 NDC subjects in a shotgun proteomic discovery experiment. Plasma samples were matched for age, gender and cognitive measures (MMSE scores) and pooled for analysis. Subsequent relative quantification and principal component analysis generated a list of candidate proteins able to distinguish the two groups AD and NDC. The most important proteins, i.e. Gelsolin, C1 protease inhibitor and Ceruloplasmin, were validated by Western blot analysis in a bigger sample set of 90 probable AD and 50 NDC subjects in total.

In this cohort, AD patients displayed significantly lower plasma Gelsolin levels compared to NDC subjects. In addition, Gelsolin levels correlated with disease progression rate and were significantly different in slow and fast declining AD patients. Further, C1 protease inhibitor levels were found to be associated with APOE ε4 genotype and lower Ceruloplasmin levels correlated with an earlier age of onset. Gelsolin is, due to its changed levels and its association with progression rate in AD, as well as due to its reported interaction with Amyloid beta (Aβ), an excellent marker. Moreover, this marker should advantageously be included in biomarker panel(s) for AD

The following abbreviations have the given meanings: AD Alzheimer's disease; Aβ Amyloid β; FD Fast decliner; SND Slow / no declining Alzheimer's patients; NDC Non-demented control Alzheimer's patients; APOE Apolipoprotein E; TMT Tandem Mass Tags; (MRM Multiple Reaction Monitoring); C1 inh Plasma protease C1 inhibitor protein; CNS Central nervous system; CSF Cerebrospinal fluid; PBS-T Phosphate buffered saline including 0.01% Tween 20; TCEP Tris(2-carboxyethyl)phosphine; RT Room temperature; TFA Trifluoracetic Acid; RP Reverse Phase; SCX Strong Cation Exchange; LC/MS/MS Liquid Chromatography coupled to tandem mass spectrometry; PCA Principal component analysis; PLS-DA Partial least square discriminant analysis; ROC Response Operator Curve.

### Example 1: Selection of Subjects and pools

The population of the main study was derived from a largely community based population of subjects with Alzheimer's disease and elderly people [Alzheimer's Research Trust (ART) cohort] and were assessed by several cognitive measures including mini mental state examination (MMSE) scale and Alzheimer's disease assessment scale-cognitive subscale (ADAS-cog). The samples were matched for age, gender and baseline MMSE scores between groups and for age, gender and MMSE decline within groups. Samples were collected into EDTA coated glass tubes and stored at -80°C until further analysis.

For the discovery experiment, plasma samples were analysed from FD and SND groups at baseline (year 1) and after two years (year 3); a single plasma sample was collected from each subject in the NDC group. A total of 15 samples were available per group (75 samples in total) and these were pooled into three sets each of five samples. This results in a total of 15 pools for subsequent analysis. The classification of AD samples to one of the two groups - fast decliners and slow decliners - was performed according to their decline in MMSE scores per year over two years. Samples from patients with a decline of 0-3 points per year on the MMSE scale were classified as slow declining AD patients, whereas samples from patients with an annual decline of 6 or more points on the MMSE scale were assigned to the fast declining group. The samples pooled were matched for age and gender (Table 1 a). The fast decliner pools all had a mean age of 78, an average baseline MMSE score of 18-20 and a mean decline of 11 points per year on the MMSE scale. The slow decliner pools on the other hand had an average age of 80-82, a mean baseline MMSE score of 18 - 21 and an average decline of 2 points per year on the MMSE scale. In comparison, the pools of the NDC had an average age of 78 and were also matched in gender.

For validation purposes, a larger sample set, including the original samples, was used. In total, 90 AD patients were compared to 50 controls (Table 1 b). The study was approved by the relevant research ethics committees.

### Example 2: Sample Preparation

In example 3, protein detection by isobaric protein labelling is demonstrated. This example explains how the sample may advantageously be prepared for such an analysis. Of course if a different analysis is used, then a different sample preparation might be chosen.

In general, sample preparation and labelling with tandem mass tags (TMT) was performed as previously described (Dayon et al., 2008) with minor modifications. To increase the number of detectable proteins, plasma was depleted of the six highest abundant proteins (albumin, transferrin, IgG, IgA, antitrypsin, and haptoglobin) with a multiple affinity removal system (MARS, 5188-5332, Agilent, Palo Alto, CA). 30µl of pooled plasma were diluted 1:4 by the addition of 90µl MARS buffer A, vortexed and spinned down for 1 min at 15'500 x g. 100µl of the supernatant was injected on a 4.6mm x 50mm MARS column and processed according to the manufacturers instructions. The flow through fractions (1ml) were collected and transferred to 5kDa MWCO centrifugal filter devices (Vivaspin 4, VS0414, Sartorius, Goettingen, Germany) for buffer exchange and protein concentration. After the addition of 3ml 100mM triethylammonium bicarbonate (TEAB) pH 8.2, the tubes were centrifuged at 2'000 x g at 4°C for 30min. Subsequently, 3ml of TEAB were added and centrifuged for 30min, another 3ml of TEAB were added and centrifuged for 60min until the remaining volume was between 50-100µl. The volume was adjusted to 150µl and the protein content of each plasma pool was determined with a conventional Bradford assay (Protein Assay, Bio-Rad, Hercules, CA).

### Example 3: Protein Detection

In principle, protein detection may be by any suitable means known to the skilled reader. The Isobaric Protein Tagging technique is now described by way of example.

To ensure equal protein amounts, 100µg of protein per sample were transferred to a new tube, 5µl 2%SDS in H₂O (w:w) were added and filled up to 100µl with 100mM TEAB. 5.3µl 20mM Tris(2-carboxyethyl)phosphine hydrochloride (TCEP) in H₂O were added and incubated for 30min at room temperature (RT). Afterwards, 5.5µl 150mM iodoacetamide in acetonitrile (ACN) were added and incubated in the dark for 60min at RT. Subsequently, 10µL of freshly prepared trypsin (Seq. grad modified trypsin, Promega, V5111, Madison, WI, USA) at 0.4µg/µl in 100mM TEAB were added and incubated overnight at 37°C.

The 15 plasma pools were split for analysis into three individual experiments (biological replicates), whereas one of these experiments was repeated three times for technical replication. In each of the three individual experiments, the plasma pools were labelled with 60mM TMT reporter ions (Proteome Sciences Plc, London, UK) in 40.3µl ACN for 1 hour at RT as follows: m/z 126.1: FD year 1, 127.1: FD year 3, 128.1: SD year 1, 129.1: SD year 3, 130.1: NDC, 131.1: Dade Behring reference plasma. The Dade Behring plasma was analyzed in all experiments to enable inter-experimental comparison. After incubation, 8 µL of 5% hydroxylamine in H₂O (w/v) was added to each tube and mixed for 15 min. The six samples were pooled into a new tube and diluted 1:6 with 5% ACN 0.1% trifluoroacetic acid (TFA) in H₂O for reduction of ACN content.

### Example 4: Preparation for MS analysis

In this example the tagged proteins are detected using mass spectrometry (MS). To avoid negative effects of excessive reagents and high salt content in the MS analysis, the samples were manually purified and desalted on a vacuum manifold (Thames Restek UK Limited, 26077, Saunderton, UK) with reverse phase (RP) columns (Waters Corporation, Oasis HLB cartridge, WAT094225, Milford, MA, USA) followed by strong cation exchange (SCX) columns prepared from empty cartridges (Macherey-Nagel GmbH & Co KG, 732501, Dueren, Germany) and sepharose suspension (Sigma-Aldrich, SP Sepharose Fast Flow, S1799-100ML, St. Louis, MO, USA). The eluted sample was lyophilised in a speed vac, dissolved in 2ml H₂O, evacuated to dryness again and stored at -80°C until further analysis.

### Mass spechometry

For LC/MS/MS analysis, the samples were reconstituted in 100µl of 50mM ammonium bicarbonate buffer for 10min at 37 °C. Following, the samples were further diluted (1:60) in ammonium bicarbonate to an approximate concentration of 0.1 µg/µl.

Reversed-phase chromatography was performed using an Ultimate LC system (Dionex, Camberley, UK). Peptides were resolved on a C₁₈ PepMap column (75µm I.D.) using a three-step linear gradient of 0-48% ACN / 0.05% formic acid over 120min at a flow rate of 200nL/min. Peptides were ionised by electrospray ionisation using a Z-spray source fitted to a QTof-micro (Waters Ltd, Elstree, UK) operating under Masslynx v4.0 software. The instrument was run in automated switching mode, selecting precursor ions based on their intensity and charge state for sequencing by collision-induced fragmentation. MS/MS was performed using collision energy profiles based on mass/charge (m/z) ratios and optimised for the fragmentation of TMT-labelled peptides. Raw data were recalibrated against internal complement C3 peptides and processed into peak lists using ProteinLynx Global Server v2.2.5 with the following MS/MS processing parameters: smoothing by Savitzky-Golay method, 2 iterations, 4 channels; peak centroiding top 80%, no deisotoping or background subtraction.

### Example 5: Protein Identification

Proteins were identified in each TMT experiment by searching the MS peak list data against the IPI human database (release v3.32) as a single search using Mascot (v2.2; http://www.matrixscience.com/). The following parameter specifications were employed: precursor ion mass tolerance 150 ppm, fragment ion mass tolerance 0.6 Da, tryptic peptides with up to three missed cleavages, variable modifications: carbamidomethylation of cysteine, methionine oxidation and TMT labelling of epsilon-amino functions of lysine residues and the peptide N-terminus. The resulting Mascot file contains information about the proteins identified and can be used for manual inspection of the spectra. The peak list files were also processed using TiTRE (v1.6), an in-house developed software, to extract the reporter ion data. This was subsequently associated with the Mascot search results.

Search results were processed to report only assignments with an MS/MS ion score >20 and a rank one sequence assignment. For Mascot MS/MS ion searches, a rank one assignment is the highest scoring sequence match for an MS/MS query. These peptide assignments were evaluated for 'uniqueness', i.e. if the assigned sequence only occurred within that protein the assignment was 'unique', or if the assigned sequence also was present in other proteins the assignment was 'non-unique'. Protein hits that were assigned with less than two unique peptides scoring above the Mascot identity score (typically ∼40), which corresponds to a 5% chance of false positive peptide assignment, were validated by manual inspection of the mass spectra for the assigned peptides.

The reporter ion intensities were each expressed as a ratio to the reference plasma standard and converted to log10 values. Data were normalised by subtracting the median value of the log10 reporter ion ratios (a global normalisation factor) from each individual value such that median of the normalised log 1 0 reporter ion ratios was zero.

From previous experience, the minimum threshold at which reporter ion intensities could be reliably determined using the Qtof micro instrument was 40 counts. Below this threshold, the observed protein ratios are not representative of the actual ratios due to poor ion statistics. For this reason, peptides with reporter ion intensities in the reference plasma of below 40 counts were excluded from quantitation. Additionally, peptides that were incompletely labelled with TMT and those containing methionine were removed from the data set. Assignments corresponding to incomplete TMT labelling and/or containing methionine residues were observed to quantitate differently from the main population of assignments and were often not representative of the actual differences in protein amounts. In the latter case, the reporter ion ratios appeared to track differential methionine oxidation between the samples.

The normalised data sets were joined such that proteins identified in different biological replicate experiments were aligned.

### Example 6: Multivariate analysis

The aligned data sets from example 5 were imported into SIMCA-P software (version 11.5). All variables (mean log 1 0 reporter ion ratios for each protein) were scaled to unit variance and variables with more than 50% of values missing were excluded from the analyses. Initially, the data were summarised using principal components analysis (PCA) to check the presence of strong outliers or other issues in the data set that would need to be addressed. Subsequently, partial least squares - discriminant analysis (PLS-DA) was used to identify proteins that differed between the groups.

### TMT analysis of plasma samples

To investigate the plasma proteome of slow and fast declining AD patients and of non-demented controls, 15 samples per group, pooled into 3 sets each, were labelled with isobaric protein tags and analyzed by mass spectrometry. A total of 2365 queries were matched to peptide sequences across all five experiments. These peptides related to a total of 152 unique protein sequences. After removal of proteins with more that 50% of the measurements missing, a total of 52 identified proteins had quantitative data available for analysis (Table 2). Multivariate analysis (PLS-DA) was then used to attempt to discriminate the different groups using the mean relative protein concentration data. Following model fitting, cross validation indicated that significant components could not be fitted to data sets containing SD year 1 or year 3 and NDC observations.

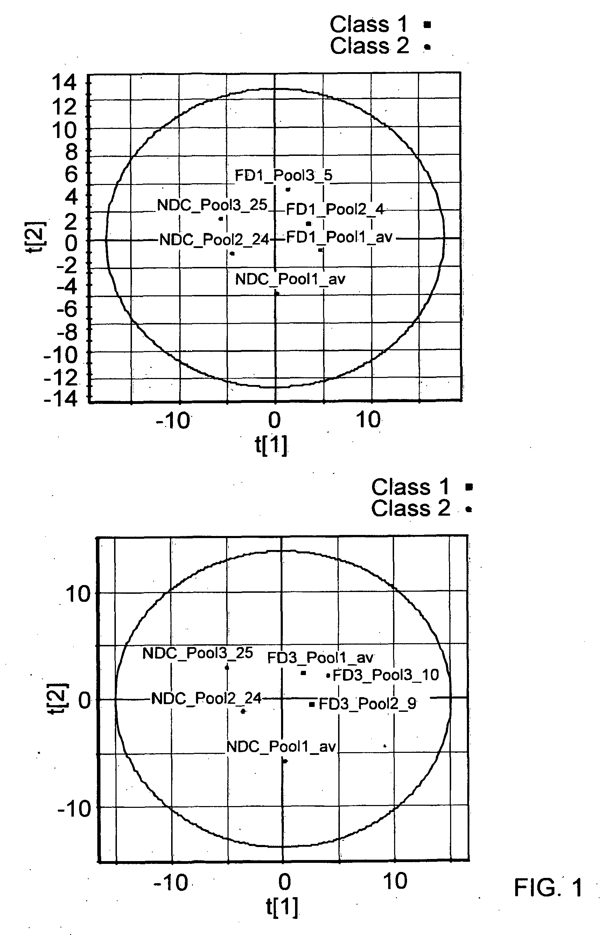

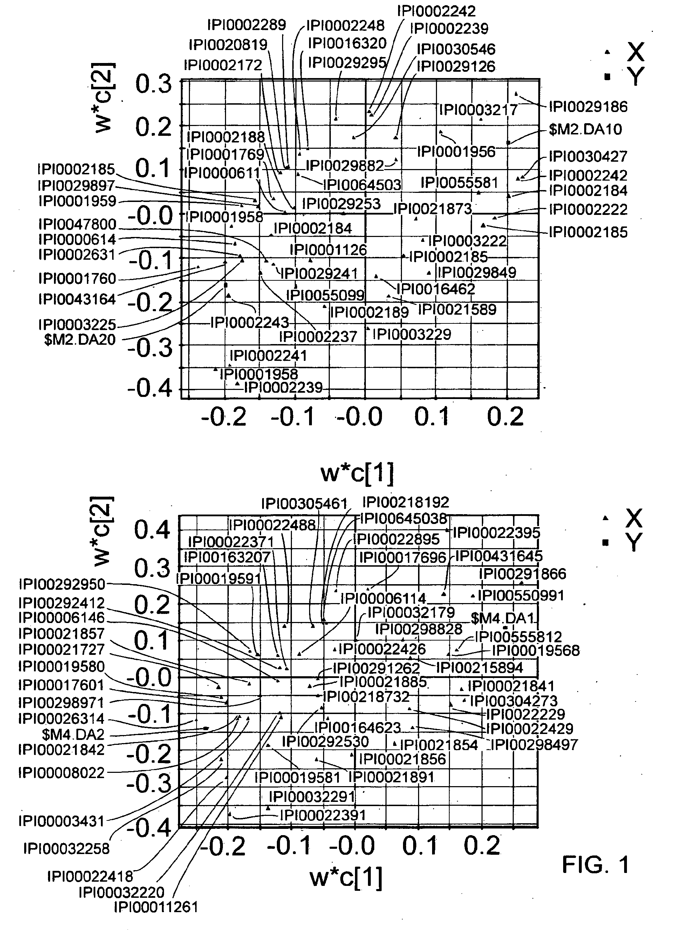

A three component model was fitted to the FD year 1 and NDC data set explaining 99.5% of variance in the class data (R2Y). Figure 1 shows the scores and weights for the PLS-DA model together with additional parameters summarising the model. A similar three component model explaining 99.5% of the variance in the class data was also fitted to the FD year 3 and NDC data set.

The proteins most important for discrimination of FD and NDC are highlighted in Table 1. The relative importance of these proteins was judged based on the PLS regression coefficients in the previously described models.

### Example 7:Western blot analysis

As noted throught the application, isobaric protein tagging is only one way in which detection of the markers used in the invention may be executed. For validation of the data obtained by isobaric protein tagging, selected proteins were analysed by Western blotting in a larger sample set. Plasma samples were diluted as follows: 4µl raw plasma, 96µl of PBS containing protease inhibitor cocktail (Complete^{®}, 1836145, Roche Applied Science, Penzberg, Germany) and 100µl of 2x sample buffer (Laemmli, S3401, Sigma), heated to 100°C for 5 min, spun down at 15'500 x g and separated on SDS polyacrylamide gels (NuPAGE^{®} Novex 4-12% Bis-Tris Midi Gel, 26W, Invitrogen, Carlsbad, CA, USA). After transfer to 0.2µm nitrocellulose membranes (Schleicher & Schuell, BA-S 83, Keene, NH, USA) blots were blocked with 5% non-fat milk in 0.1% PBS-Tween (PBS-T) and probed with antibodies to Ceruloplasmin (ab8813, 1:50000, Abcam, Cambridge, UK) C1 protease inhibitor (ab36992, 1:2000, Abcam) and Gelsolin (ab55070, 1:500, Abcam). Primary antibodies were detected with appropriate secondary antibodies (dilution: 1:10000) conjugated to fluorophors, emitting at wavelengths of either 700 or 800 nm, using a near infrared Odyssey imager (Licor, Lincoln, NE, USA). Densitometric analysis was performed using the Odyssey software v2.1.

All samples were run in duplicates and, for adjustment of intensities, a reference plasma sample was run on each gel. Equal volumes of plasma were subjected to immunoblot and subsequent quantitative densitrometry. The ratio of every sample with the reference plasma was build to allow inter-gel quantitative comparisons and the duplicate runs were averaged. When assessing the reproducibility of the duplicate gels, a large positive correlation of 0.84 was found by performing a Pearson correlation. The results were tested for significance by student's t-test using the statistical software program SPSS v15.0. In addition, a response operator curve (ROC) analysis was performed to assess sensitivity and specificity.

In order to confirm the proteomic data, from example 6, selected proteins were analysed by Western blotting to investigate the difference between AD and NDC samples found in the multivariate analysis. A high absolute value of a PLS coefficient indicates a high relative importance of a certain protein in separating respective groups. Therefore we selected 3 proteins - Gelsolin, C1 protease inhibitor (C1 inh) and Ceruloplasmin - with the highest PLS coefficient values (Table 2) for further validation in a larger data set. The data set consisted of the samples used for the initial study and of additional samples to give a total of 90 AD and 50 NDC samples to be compared.

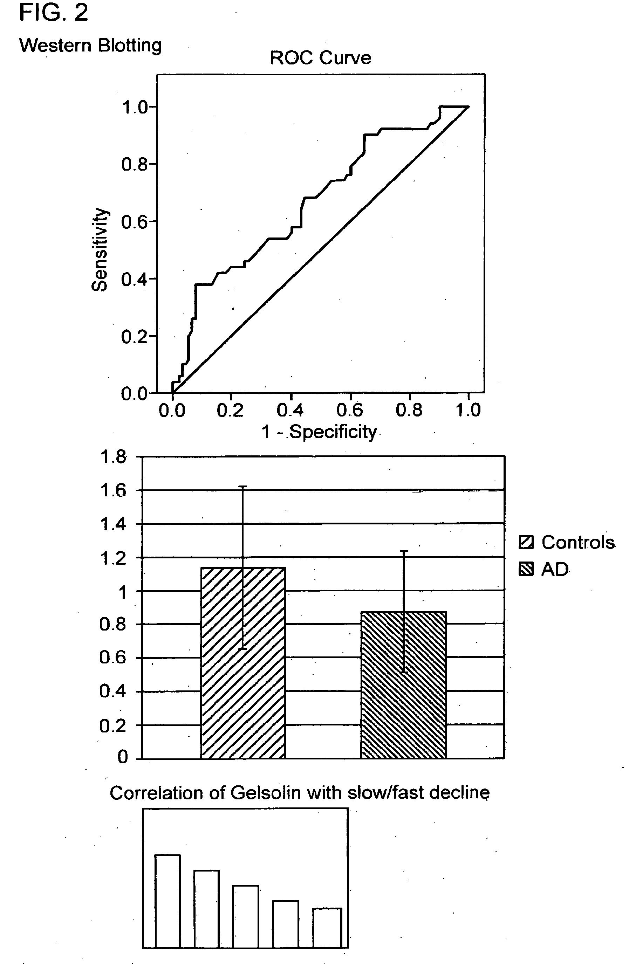

The results were tested for significance using student's t-tests and a highly significant reduction of Gelsolin (p = 0.001) levels in plasma from AD patients could be confirmed (Figure 2a). Changes in C1 inh and Ceruloplasmin levels seem to be less pronounced and did not reach statistical significance. These three proteins were additionally analysed in relation to APOE genotype, MMSE score as a measure of severity, MMSE decline per year and also age of onset. Gelsolin levels were found to be correlated with MMSE decline per year (Pearson correlation -0.209, p = 0.05). This finding is supported by the fact that, after assigning AD patients to slow and fast declining groups applying identical classification criteria as in the initial dataset, Gelsolin levels were found to be significantly lower in the FD group compared to SND (student's t-test; p = 0.019). Therefore, the results from the TMT analysis (Figure 2b) could be confirmed. Further, C1 inh showed a weak association with number of APOE ε4 alleles (Pearson correlation -0.175; p = 0.043) and Ceruloplasmin showed a correlation with age of onset (Spearman correlation 0.231; p = 0.031).

In addition, a ROC analysis for Gelsolin was performed. The area under the curve was highly significant (p = 0.001), but the protein did not show favourable test characteristics: Setting specificity at 80% gave a sensitivity value of 44%, whereas setting the sensitivity value to 80% resulted in a specificity value of 39%.

### Summary

The inventors disclose the first analysis of unfractionated plasma with isobaric protein tags in Alzheimer's disease.

Moreover, using tandem mass tags in a shotgun proteomics discovery experiment, we were able to identify several proteins able to differentiate fast declining Alzheimer's patients and non-demented controls. Selected candidate proteins were validated in a larger dataset with quantitative Western blotting. It was shown that plasma levels of Gelsolin were decreased in AD compared to controls and that the levels also differ between fast and slow declining AD patients. Most important, Gelsolin levels correlated with disease progression.

In addition, C1 protease inhibitor levels were found to be associated with APOE ε4 genotype and lower Ceruloplasmin levels correlated with an earlier age of onset.

### Example 8 - DISCOVERY AND VALIDATION OF MARKERS FOR ALZHEIMER'S DISEASE USING ISOBARIC PROTEIN TAGGING

Biomarkers for Alzheimer's disease (AD) are urgently needed. Recent studies indicate that differences in plasma proteins levels exist between AD patients and non-demented controls (NDC) suggesting the possibility of a peripheral biomarker for AD. In the current study, we used isobaric mass tagging to compare the plasma protein levels in 30 probable AD and 15 NDC subjects in a shotgun proteomic approach. Plasma samples were matched for age, gender and cognitive measures (MMSE scores) and pooled for analysis. Subsequent relative quantification and principal component analysis generated a list of candidate proteins able to distinguish the two groups AD and NDC. The most important proteins, i.e. Gelsolin, C1 protease inhibitor and Ceruloplasmin, were validated by Western blot analysis in a bigger sample set of 90 probable AD and 50 NDC subjects in total. To further validate some of the findings, multiple reaction monitoring (MRM), a method, which is not only highly specific but also has a very high sensitivity, was used to analyze a subset of samples. In summary, AD patients displayed significantly lower plasma Gelsolin levels compared to NDC subjects. In addition, C1 protease inhibitor levels were found to be associated with ApoE epsilon4 genotype and lower Ceruloplasmin levels correlated with an earlier age of onset. Gelsolin is, due to its changed levels in AD, as well as due to its reported interaction with amyloid beta (Abeta) a highly interesting protein with regards to AD and needs further evaluation.

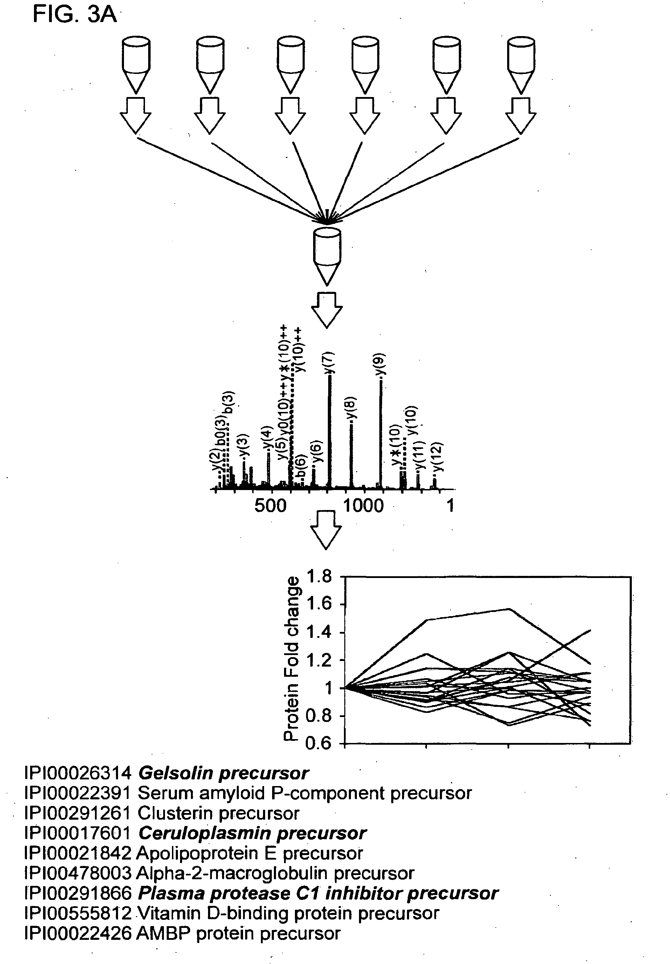

A general method is shown in Figure 3A.

### Isobaric protein tagging using LC/MS/MS

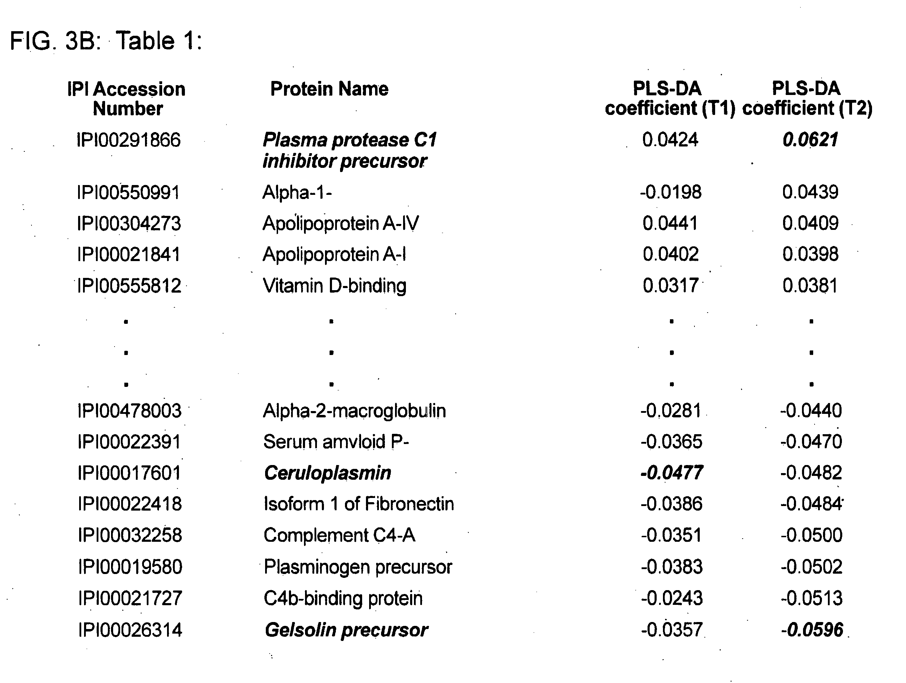

A total of 2365 queries were matched to peptide sequences across all experiments. These peptides related to a total of 152 unique protein sequences. Including only proteins detected in more than 50% of the experiments, a total of 52 identified proteins had quantitative data available for analysis. Multivariate analysis (PLS-DA) generated a list of candidate proteins (Table 1 - Figure 3B) able to discriminate the different groups of subjects using the protein mean data

Table 1 (Figure 3B): List of candidate proteins discriminating AD and NDC subjects. High absolute values of PLS-DA coefficients indicate a high importance of a respective protein in the discrimination between the groups, whereas positive/negative values denote increased/decreased levels in the AD group. T1 and T2 refer to measurements of samples at baseline and 2 years follow-up.

### Western blotting

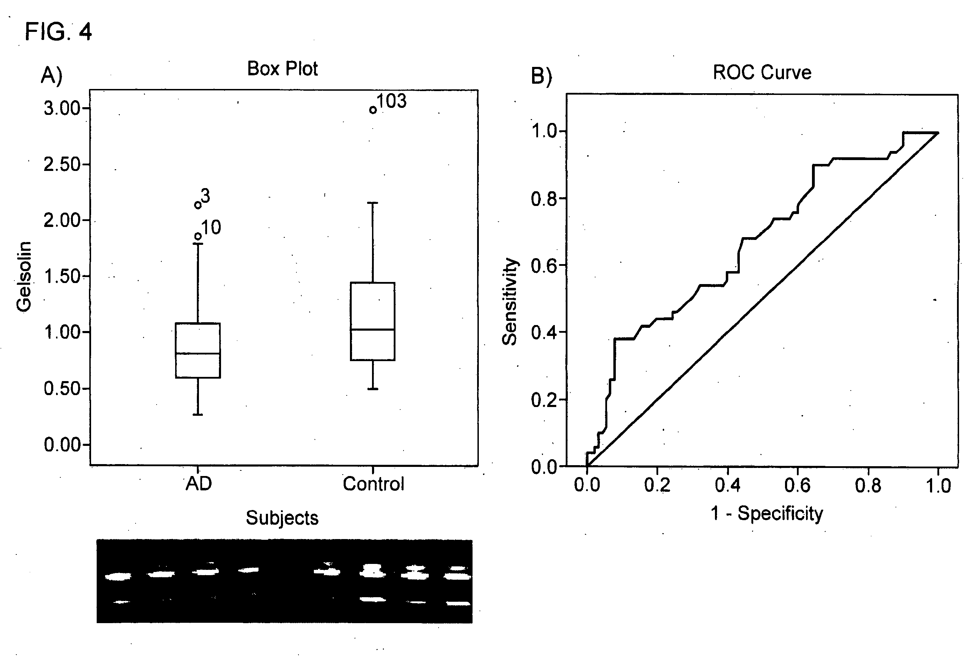

In order to confirm the proteomic data, selected proteins were analysed by Western blotting to investigate the change between AD and NDC extracted by multivariate analysis. Therefore we selected 3 proteins - Gelsolin, C1 protease inhibitor and Ceruloplasmin - with the highest PLS coefficient values (Table 1; bold) at baseline and two years follow-up respectively for further validation in a larger data set. The data set consisted of the samples used for the initial study and of additional samples to give a total of 90 AD and 50 NDC samples to be compared. The results were tested for significance using independent samples t-Test. For the first time, a highly significant reduction (p = 0.001) of Gelsolin levels in plasma from AD patients could be established (Fig. 4A). Changes in C1 inhibitor protein and Ceruloplasmin seem to be less pronounced and did not reach statistical significance. Correlation analyses were performed with clinical parameters such as MMSE scores, MMSE decline per year and age of onset and it was tested for an association with ApoE e4 genotype. In summary, C1 protease inhibitor showed a significant association with ApoE e4 carriers (p = 0.035) and Ceruloplasmin showed a correlation with age of disease onset (Spearman correlation 0.231, p = 0.031), thereby providing an explanation for their high PLS-DA coefficient values in the multivariate analysis.

In addition, a Response Operator Characteristic (ROC) analysis for Gelsolin was performed (Fig. 4B). The area under the curve was highly significant (p = 0.001) but the protein did not show favourable test characteristics: Setting specificity at 80% gave a sensitivity value of 44%, whereas setting the sensitivity value to 80% resulted in a specificity value of 39%.

### Conclusions:

- AD subjects displayed significantly lower plasma Gelsolin levels compared to NDC subjects.
- C1 protease inhibitor levels were found to be associated with ApoE e4 genotype.
- Ceruloplasmin levels showed a positive correlation with age of disease onset.

Use of Gelsolin as a biomarker is merited given its apparent changes in plasma between AD and control subjects, and its previously reported interaction with Ab (Chauhan et al., 1999, Ray et al., 2008).

### Example 9: Selective Reaction Monitoring of Gelsolin as part of a larger panel of plasma AD biomarkers

A number of plasma proteins including gelsolin have emerged as candidate AD biomarkers from discovery exercises. Assays are required for the validation of these candidates; SRM-based approaches are an attractive alternative to ELISAs due to the sensitivity and selectivity of the technique, the capacity to muliplex and the limited availability of antibodies. Here, signature peptides unique to the protein of interest are measured to provide quantitative information of that protein in the sample. Accuracy in the quantitation of the analytes of interest by SRM can be improved by combining with TMT as this allows the incorporation of an internal reference into the analysis. Initial results have demonstrated TMT SRM as an accurate and reproducible method of peptide quantitation. We now move to provide a full TMT SRM assay allowing the evaluation of eight candidate biomarkers in AD and control plasma samples.

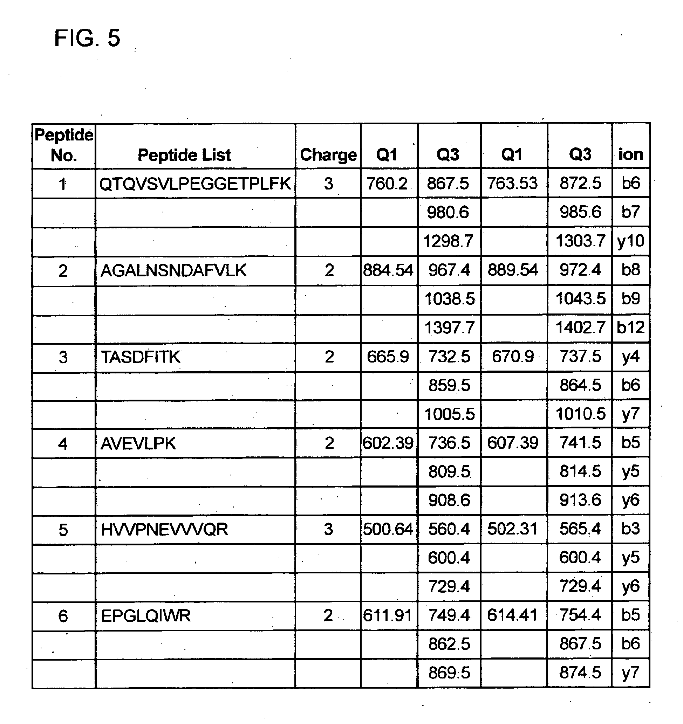

Using existing MS/MS data, at least three peptides per protein were selected for quantitation and the representative peptides for Gelsolin are shown in Figure 5. Criteria for selection included; no missed cleavages with trypsin, no variable modifications (in-vivo or experimental) and each was proteotypic (unique). Synthetic versions of each peptide were prepared and labeled with TMTzero to act as a reference for quantitation. Peptides were infused into a 4000 QTRAP (Applied Biosystems) and MS/MS data was acquired. Optimal fragment ions were chosen for all peptides to facilitate maximum detection of each in Q3. Corresponding TMTsixplex-labeled fragment ion masses were calculated and MS instrument parameters optimised for individual Q1 and Q3 transition pairs. A pooled plasma sample was digested, labeled with TMTsixplex and combined with the TMTzero-labeled reference peptides. LC/MS/MS analysis was performed using an Ultimate 3000 nano LC (Dionex) and a 4000 QTRAP. Peptides were resolved by reversed-phase chromatography over a 90min ACN gradient. Using accurate retention times for each peptide, SRM scheduling was applied to the method (+/- 3min detection window; 2.5sec cycle time; 37.8msec dwell time and 21 data points at FHMW).

All peptides were observed in the plasma sample by TMT SRM at varying intensities. To achieve accurate quantitation of each candidate peptide, it was necessary to establish whether there was significant contribution of non-specific signal from the plasma, when no reference peptides were added. TMTzero-labeled plasma was analysed over all TMTzero and TMTsixplex transitions. Signals observed from any TMTzero SRM transitions represented non-specific background. Those transitions which had significant background were subsequently removed from the method.

### Example 10: Validation of gelsolin and further expanded panel of markers

### 1. Introduction

The establishment of specific and sensitive biomarkers for Alzheimer's disease (AD) is required to assist in the diagnosis and monitoring of disease progression. From discovery exercises, clusterin (SwissProt accession number P10909), complement c3 (P01024), serum amyloid P component (P02743; SAP), alpha-2-macroglobulin (P01023; A2M), gelsolin (P06396), gamma-fibrinogen (P02679) and complement factor H (P08603; CFH) were found to be differentially expressed in an AD versus control samples Additionally, possession of the apolipoprotein e4 allele is the only unequivocal genetic risk factor known to date for late-onset AD and expression of the apolipoprotein E (P02649; ApoE) protein may be altered in AD. These proteins present as potential candidates for which progression of the disease may be monitored. This example details the application of a Tandem Mass Tag Selected Reaction Monitoring (TMT-SRM) assay to a clinical sample cohort (n=20; 10 Alzheimer's disease (AD), 10 controls). The aims of the study were firstly, the validation of western blot (WB) findings for one of the proteins, gelsolin (found to be decreased in AD patients versus control) using an orthogonal technology (TMT SRM) and secondly, to assess the performance of the remaining proteins in an AD cohort by TMT SRM.

### 2. Methods

### 2.1. Sample selection

Samples were selected based on WB measurements for gelsolin. To give the best chance of detecting a difference between disease and control by TMT-SRM, those samples which showed the highest and lowest concentrations of gelsolin (n=10 per group) were carried forward for analysis.

### 2.2. Selection of candidate peptides for SRM quantitation

Existing MS/MS spectra of TMT-labeled plasma datasets, including those from discovery exercises, were mined to determine the most suitable peptides for candidate biomarker validation by SRM. At least three peptides per protein candidate were selected for quantitation (32 in total), 16 of which were observed in discovery exercises. The criteria for the selection of these peptides for SRM were; good high mass fragment ions, no missed cleavages with trypsin, no variable modifications (in-vivo or experimental). Due to the poor endogenous detection and the poor accuracy, precision and reproducibility when plotting previous calibration curves, several of the peptides were removed from the method. This left 22 peptides to be quantitated in this part of the study.

### 2.3. Sample preparation of synthetic peptides

Synthetic versions of each peptide were prepared in-house to act as a reference for quantitation. Previous results demonstrated a variation in the amounts recovered from peptides of the same protein. In order to minimise such variation, peptides of the same protein were combined in an equimolar mixture (62nmoles/peptide; 8 mixtures in total) prior to TMT-labeling. Each mixture was processed using a typical TMT-SRM workflow consisting of reduction, alkylation, trypsin digestion and chemical labeling of individual samples with TMT. Mixtures were labeled with light TMT (to act as an internal standard for the generation of reverse calibration curves in plasma) and heavy TMT (used for the generation of a reverse calibration curve in plasma and for use as an internal standard for quantitation of the experimental sample set). Both mixtures underwent subsequent purification by solid-phase extraction and strong cation exchange using volatile buffers.

### 2.4. Preparation of plasma samples

An equal volume of each plasma sample (25ul) was removed from the stock and diluted 10-fold. From this, 12.5ul was added to each digestion to give approximately 100ug of protein per digest. Additionally, a pool of all samples (100ug per sample; 2mg of protein in total) was prepared for preparation of calibration curves and for quality control purposes. Following solubilisation with SDS and dilution each sample was reduced, alkylated and digested with trypsin. Samples were labeled with light TMT prior to purification by solid-phase extraction and strong cation exchange using volatile buffers. Samples were lyophilised prior to MS analysis. All samples were processed in triplicate (technical repeats).

### 2.5. SRM analysis of sample set

SRM analysis was performed on a 4000 QTRAP mass spectrometer (Applied Biosystems) coupled to an Ultimate3000 LC system (Dionex). The mass spectrometer was fitted with a micro-ion spray source for micro litre flow rates, operated in positive ion mode and Q1 and Q3 resolution was set to unit (0.7 FWHM). Peptides were resolved by reversed phase chromatography using a Hypersil gold column (1 mm i.d. x 50 mm; 1.9 µm, Thermo Fisher Scientific), over a 14 min gradient of 5-30% ACN/0.2% formic acid at a flow rate of 100 µL/min. Washing and equilibration of the column increased the total run time to 20min.

Labeled peptides were directly infused into the QTRAP MS and in the first instance selected for MS/MS fragmentation. Optimal MS/MS fragment ions (three) were chosen for each peptide to facilitate maximum detection and specificity of each transition in Q3. All peptides were then measured by SRM by direct infusion using the selected Q3 transitions. The sensitivity of detection of each SRM was further optimised by tuning the collision energy, declustering potential and collision cell exit potential. Peptides were analysed by LC-SRM to define accurate retention times. The final SRM method applied to the analysis of the samples had a SRM scheduling window of 45sec, 1 sec cycle time, >20ms dwell time/transition with 5-10 data points at FWHM.

Samples were resuspended in 333.33 µL of 3% ACN/0.2% formic acid. For each individual analysis, 100ul was aliquoted into a microtitre plate well and lyophilised. All samples were processed in triplicate (analytical repeats). Immediately prior to analysis, samples were resuspended in 25ul of heavy TMT-labeled peptides (5fmoles/ul; 100fm on column). This concentration of sample ensured good detection of the target analyte with no carry-over taken into subsequent runs. Sample (23 µl) was injected on column using a full loop injection (Figure 1). A 12-point calibration curve was produced by resuspending the pooled plasma sample with 25ul of light TMT and heavy TMT-labeled peptides (light TMT peptides constant at 5fmoles/ul: 100fm on column and heavy TMT peptides varied from 1-6000fm on column).

The experimental samples were analysed in three consecutive sets of 60 samples to provide three analytical repeats. In each set of 60 the run order was shuffled to exclude run-time and run order bias and to ensure that a particular sample was not preceded and followed by the same sample twice. A calibration curve was run immediately prior to running a set of 60 samples with two extra curves ran after the full sample set was completed (five in total). Prior to the analysis of the sample set system checks were undertaken to ensure the MS and LC were performing with optimal sensitivity, mass accuracy, calibration and ion stability. During the analysis of the sample set a reference sample was acquired multiple times to ensure LC-SRM performance was maintained.

### 2.6. SRM data processing

SRMs were visualised through Analyst's quantitation wizard (Applied Biosystems). All peak matching was visually verified and peak areas were exported into Microsoft Excel. Transitions were excluded if there was poor peak definition from the background signal. The peak area for each light-labeled transition (experimental plasma sample or pooled plasma sample for quality control) was measured relative to the peak area of the corresponding heavy-labeled transition (synthetic peptide; reference sample). These ratios (L/H) for each transition pair were taken forward for quantitative analysis.

### 2.7. Statistical analysis

The experimental design was hierarchical (nested) with transitions nested within peptides nested within digests and three replicate measurements made for each digest. We chose an approach to analysis that would give a realistic estimate of the 95% confidence interval, taking into account the variance attributable to all factors in the hierarchy. Three-way analysis of variance was used to separate and estimate the different sources of variation and these were then recombined to give an estimate of the variance of the mean. The variance of the mean was used to estimate the standard error and this, together with a value from the t-distribution was used to estimate the 95% confidence interval around the grand mean.

Gelsolin measurements were correlated to WB results by calculating the Spearman coefficient for each gelsolin peptide (L/H ratio) in each sample, as compared to the corresponding WB sample measurement.

### 3. Results

Table shows Gelsolin levels as determined by WB for disease (AD) and control (CTL) samples. High and low gelsolin levels were specifically selected to give the best chance of detecting a difference by TMT-SRM. It can be seen from the mean values of each group that there is ∼4-fold reduction in gelsolin levels in AD as compared to controls.

| **AD sample no. with low gelsolin levels by WB** | **levels** | **CTL sample no. with high gelsolin levels by WB** | **levels** |
|---|---|---|---|
| 68 | 0.33 | 352 | 1.55 |
| 156 | 0.37 | 436 | 1.55 |
| 284 | 0.27 | 446 | 1.80 |
| 371 | 0.40 | 449 | 1.67 |
| 768 | 0.36 | 520 | 2.16 |
| 891 | 0.48 | 880 | 1.63 |
| 1212 | 0.34 | 886 | 1.53 |
| 1219 | 0.52 | 960 | 1.78 |
| 1239 | 0.43 | 1035 | 2.99 |
| 1312 | 0.52 | 1172 | 1.76 |
| **mean** | **0.40** | **mean** | **1.84** |

### 3.1 Removal of transitions and peptides which may result in inaccuracies in quantitation

Transitions were removed from the data analysis if they had poor peak detection or possessed high plasma background (Figure 2). Furthermore, four peptides were removed from the analysis as the endogenous detection was poor in the majority of samples (peptides 3, 6, 11 and 27) or a high variance was observed for all measurements across all samples (peptide 18). Therefore, 17 peptides remained for quantitation, with at least two peptides per protein.

### 3.2 TMT SRM of gelsolin and comparison to WB results

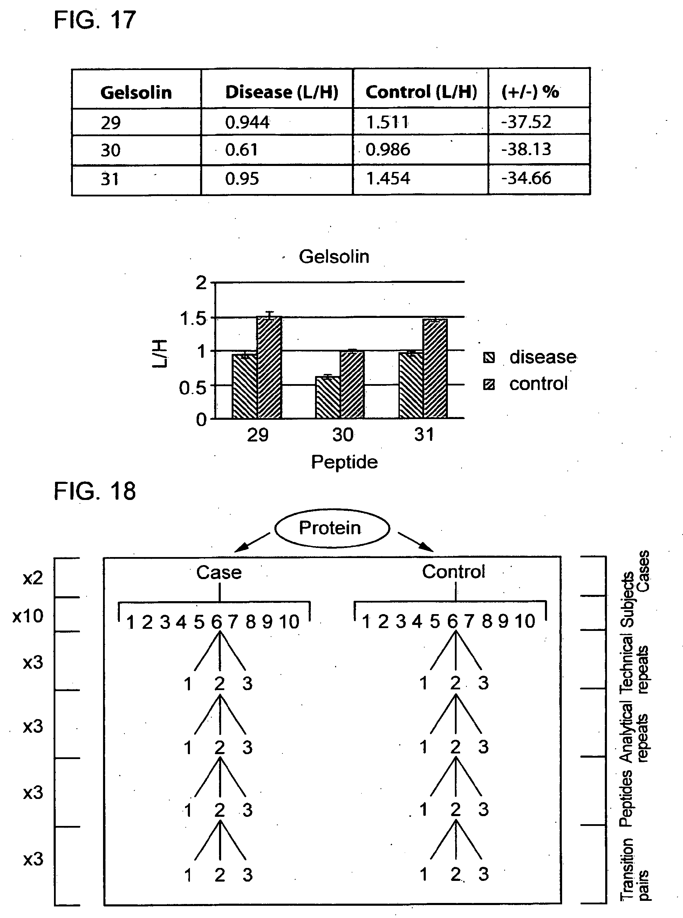

Four gelsolin peptides were originally included in the method (peptides 27, 29, 30 and 31). Peptide 27 had poor peak detection and so was removed from the data analysis. The remaining three peptides performed similarly, showing a reduction in gelsolin levels of ∼30% in AD as compared to the control samples (see fig 17). This is in-line with the WB results and discovery exercises. Upon Spearman calculation to compare the TMT SRM and WB platforms, a very high correlation (0.65-0.73; Table 2) was observed for each gelsolin peptide in each sample, as compared to the corresponding WB sample measurement:

**Table 2. Correlation of TMT SRM measurements for gelsolin peptides 29, 30 and 31 to WB measurements. A high correlation is observed for all peptides.**

| **Correlations** | | | | | | |
|---|---|---|---|---|---|---|
| | | | Gelsolin_WB | Peptides_29 | Peptide_30 | Peptide_31 |
| Spearman's rho | Gelsolin_B | Correlation Coefficient | 1.000 | 0.651 | 0.664 | 0.731 |
| | | Sig. (2-tailed) | | 0.002 | 0.001 | 0.000 |
| | | N | 20 | 20 | 20 | 20 |
| | Peptide 29 | Correlation Coefficient | 0.651 | 1.000 | 0.923 | 0.902 |
| | | Sig. (2-tailed) | 0.002 | 0.002 | 0.000 | 0.000 |
| | | N | 20 | 20 | 20 | 20 |
| | Peptide 30 | Correlation Coefficient | 0.664 | 0.923 | 1.000 | 0.808 |
| | | Sig. (2-tailed) | 0.001 | 0.000 | | 0.000 |
| | | N | 20 | 20 | 20 | 20 |
| | Peptide 31 | Correlation Coefficient | 0.731 | 0.902 | 0.808 | 1.000 |
| | | Sig. (2-tailed) | 0.000 | 0.000 | 0.000 | |
| | | N | 20 | 20 | 20 | 20 |
| **. Correlation is significant at the 0.01 level (2-tailed). | | | | | | |

### 3.3 TMT SRM of the remaining peptides

### 3.3.1 Clusterin

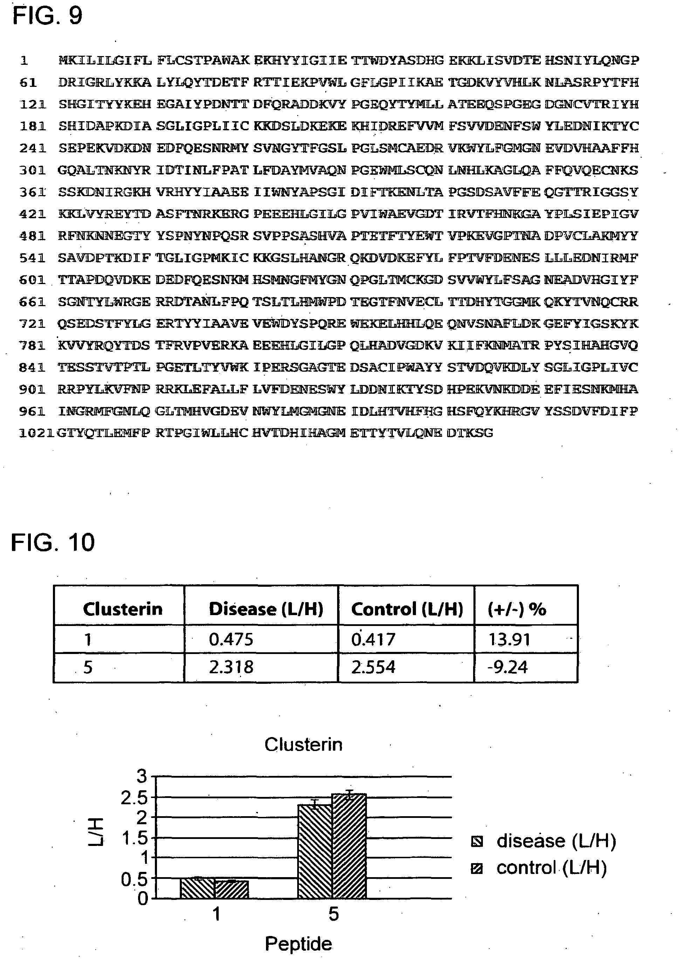

From a total of four peptides, two clusterin peptides (peptides 3 and 6) were removed from the data analysis due to poor peak detection. The two remaining peptides cover both the clusterin alpha-chain (peptide 1) and beta-chain (peptide 5). Both peptides show no significant change between AD and control samples (see fig 10), which is in-line with previous validation studies.

### 3.3.2 Complement c3

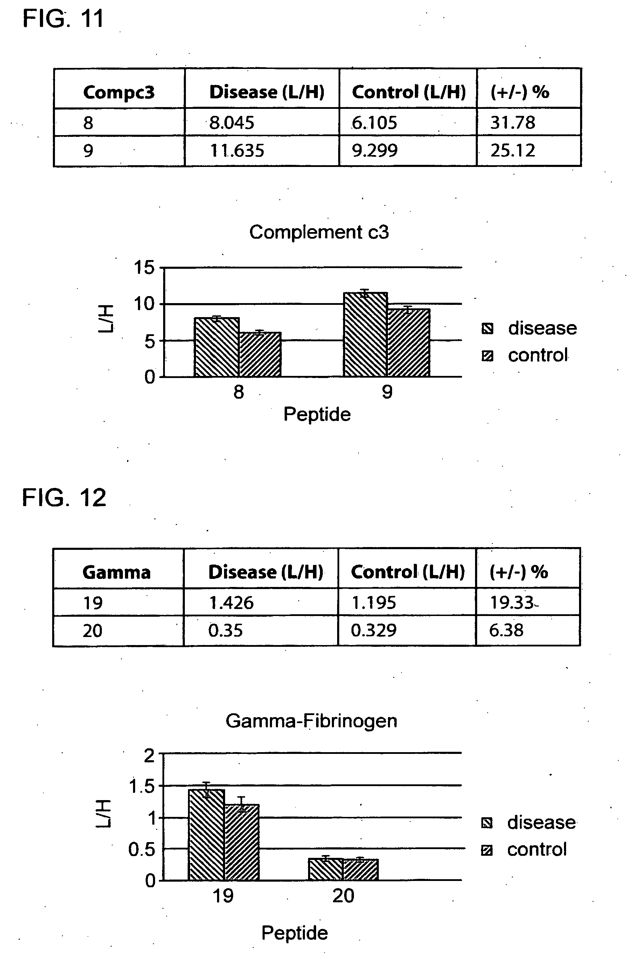

The two complement c3 peptides (peptides 8 and 9) showed a similar increase in AD as compared to control subjects (see fig 11). This increase is in-line with previous discovery exercises.

### 3.3.3 Complement Factor H

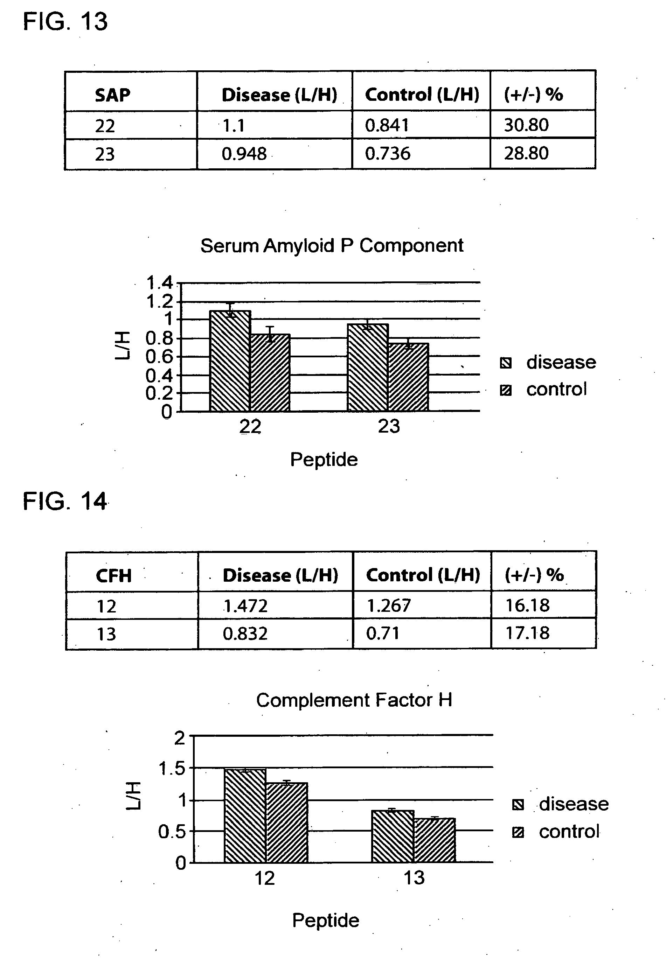

One of the CFH peptides (peptide 11) had poor peak detection and was removed from the data analysis. The two remaining peptides (peptides 12 and 13) showed a similar increase in AD as compared to control subjects (see fig 14). This increase is in-line with previous discovery exercises.

### 3.3.4 Alpha-2-macroglobulin

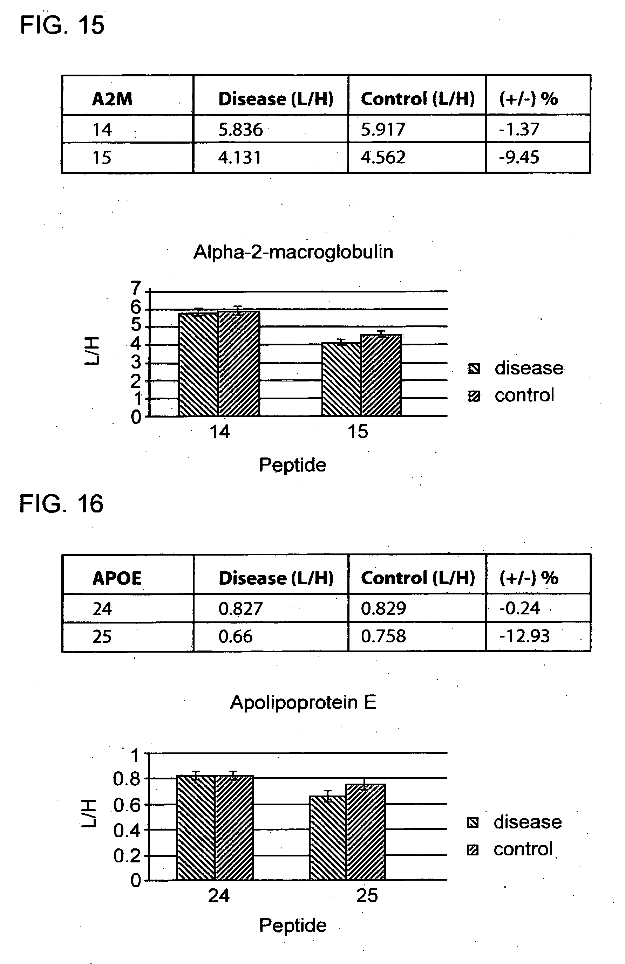

The two A2M peptides (14 and 15) performed similarly, showing no significant change between AD and control subjects. Previous discovery exercises showed an increase in A2M in AD as compared to controls (see fig 15).

### 3.3.5 Gamma-fibrinogen

A high variance was observed one peptide (peptide 18) for all measurements across all samples and so was removed from the data analysis. Gamma-fibrinogen peptides 19 and 20 both showed an increase in AD as compared to controls (see fig 12), which is in-line with discovery results.

### 3.3.6 Serum amyloid P component

The two SAP peptides (peptides 22 and 23) showed a similar increase in AD as compared to control subjects (see fig 13). This increase is in-line with discovery results.

### 3.3.7 Apolipoprotein E

The two ApoE peptides (24 and 25) performed similarly, showing no significant change between AD and control subjects (see fig 16). This is reflected in the literature, with many groups showing no change in ApoE protein levels in AD as compared to controls.

### 4. Conclusions

The results demonstrate the performance of TMT SRM as a tool for the relative quantitation of candidate biomarkers of AD. Removal of those peptides which had poor peak detection resulted in improved statistics. Peptides of the same protein showed similar differences in AD and control samples, with low variance across all samples (95% CI). The comparison of AD and control samples for the majority of peptides was in agreement with discovery exercises. TMT SRM of gelsolin was found to be highly correlated to WB results. Following on from this, calibration curves will be incorporated into the analysis to calculate the endogenous amounts of each peptide in each sample. Additionally, the dataset will be normalised to a reference sample to account for the differences observed in the L/H ratios of peptides of the same protein. The results demonstrate the good performance of TMT SRM for the relative quantiation of a small sample set. Larger cohorts may be quantitated to give in the same manner.

### REFERENCES

Abdi F, Quinn JF, Jankovic J, McIntosh M, Leverenz JB, Peskind E, Nixon R, Nutt J, Chung K, Zabetian C, Samii A, Lin M, Hattan S, Pan C, Wang Y, Jin J, Zhu D, Li GJ, Liu Y, Waichunas D, Montine TJ, Zhang J (Detection of biomarkers with a multiplex quantitative proteomic platform in cerebrospinal fluid of patients with neurodegenerative disorders. J Alzheimers Dis 9:293-348.2006).
Aggarwal K, Choe LH, Lee KH (Shotgun proteomics using the iTRAQ isobaric tags. Briefings in functional genomics & proteomics 5:112-120.2006).
Akuffo EL, Davis JB, Fox SM, Gloger IS, Hosford D, Kinsey EE, Jones NA, Nock CM, Roses AD, Saunders AM, Skehel JM, Smith MA, Cutler P (The discovery and early validation of novel plasma biomarkers in mild-to-moderate Alzheimer's disease patients responding to treatment with rosiglitazone. Biomarkers 13:618-636.2008).
Anderson NL, Anderson NG (The human plasma proteome: history, character, and diagnostic prospects. Mol Cell Proteomics 1:845-867.2002).
Baranowska-Bik A, Bik W, Wolinska-Witort E, Martynska L, Chmielowska M, Barcikowska M, Baranowska B (Plasma beta amyloid and cytokine profile in women with Alzheimer's disease. Neuro endocrinology letters 29:75-79.2008).
Blennow K, de Leon MJ, Zetterberg H (Alzheimer's disease. Lancet 368:387-403.2006).
Carugati A, Pappalardo E, Zingale LC, Cicardi M (C1-inhibitor deficiency and angioedema. Molecular immunology 38:161-173.2001).
Chauhan VP, Ray I, Chauhan A, Wisniewski HM (Binding of gelsolin, a secretory protein, to amyloid beta-protein. Biochemical and biophysical research communications 258:241-246.1999).
Choe L, D'Ascenzo M, Relkin NR, Pappin D, Ross P, Williamson B, Guertin S, Pribil P, Lee KH (8-plex quantitation of changes in cerebrospinal fluid protein expression in subjects undergoing intravenous immunoglobulin treatment for Alzheimer's disease. Proteomics 7:3651-3660.2007).
Connor JR, Tucker P, Johnson M, Snyder B (Ceruloplasmin levels in the human superior temporal gyrus in aging and Alzheimer's disease. Neuroscience letters 159:88-90.1993).
Dayon L, Hainard A, Licker V, Turck N, Kuhn K, Hochstrasser DF, Burkhard PR, Sanchez JC (Relative quantification of proteins in human cerebrospinal fluids by MS/MS using 6-plex isobaric tags. Analytical chemistry 80:2921-2931.2008).
Dayon L, Turck N, Kienle S, Schulz-Knappe P, Hochstrasser D F, Scherl A, Sanchez J-C (Isobaric Tagging-Based Selection and Quantitation of Cerebrospinal Fluid Tryptic Peptides with Reporter Calibration Curves. Analytical Chemistry DOI: 10.1021 /ac901854k, January 8, 2010).
Gaggelli E, Kozlowski H, Valensin D, Valensin G (Copper homeostasis and neurodegenerative disorders (Alzheimer's, prion, and Parkinson's diseases and amyotrophic lateral sclerosis). Chemical reviews 106:1995-2044.2006).
Garbis SD, Tyritzis SI, Roumeliotis T, Zerefos P, Giannopoulou EG, Vlahou A, Kossida S, Diaz J, Vourekas S, Tamvakopoulos C, Pavlakis K, Sanoudou D, Constantinides CA (Search for Potential Markers for Prostate Cancer Diagnosis, Prognosis and Treatment in Clinical Tissue Specimens Using Amine-Specific Isobaric Tagging (iTRAQ) with Two-Dimensional Liquid Chromatography and Tandem Mass Spectrometry. Journal of proteome research.2008).
Gilman S, Koller M, Black RS, Jenkins L, Griffith SG, Fox NC, Eisner L, Kirby L, Rovira MB, Forette F, Orgogozo JM (Clinical effects of Abeta immunization (AN1792) in patients with AD in an interrupted trial. Neurology 64:1553-1562.2005).
Giometto B, Argentiero V, Sanson F, Ongaro G, Tavolato B (Acute-phase proteins in Alzheimer's disease. European neurology 28:30-33.1988).
Glenner GG, Wong CW, Quaranta V, Eanes ED (The amyloid deposits in Alzheimer's disease: their nature and pathogenesis. Appl Pathol 2:357-369.1984).
Hergenroeder G, Redell JB, Moore AN, Dubinsky WP, Funk RT, Crommett J, Clifton GL, Levine R, Valadka A, Dash PK (Identification of serum biomarkers in brain-injured adults: potential for predicting elevated intracranial pressure. J Neurotrauma 25:79-93.2008).
Hirko AC, Meyer EM, King MA, Hughes JA (Peripheral transgene expression of plasma gelsolin reduces amyloid in transgenic mouse models of Alzheimer's disease. Mol Ther 15:1623-1629.2007).
Hye A, Lynham S, Thambisetty M, Causevic M, Campbell J, Byers HL, Hooper C, Rijsdijk F, Tabrizi SJ, Banner S, Shaw CE, Foy C, Poppe M, Archer N, Hamilton G, Powell J, Brown RG, Sham P, Ward M, Lovestone S (Proteome-based plasma biomarkers for Alzheimer's disease. Brain 129:3042-3050.2006).
Jacobs JM, Adkins JN, Qian WJ, Liu T, Shen Y, Camp DG, 2nd, Smith RD (Utilizing human blood plasma for proteomic biomarker discovery. Journal of proteome research 4:1073-1085.2005).
Janus C, Pearson J, McLaurin J, Mathews PM, Jiang Y, Schmidt SD, Chishti MA, Horne P, Heslin D, French J, Mount HT, Nixon RA, Mercken M, Bergeron C, Fraser PE, St George-Hyslop P, Westaway D (A beta peptide immunization reduces behavioural impairment and plaques in a model of Alzheimer's disease. Nature 408:979-982.2000).
Ji L, Chauhan A, Chauhan V (Cytoplasmic gelsolin in pheochromocytoma-12 cells forms a complex with amyloid beta-protein. Neuroreport 19:463-466.2008).
Kessler H, Pajonk FG, Meisser P, Schneider-Axmann T, Hoffmann KH, Supprian T, Herrmann W, Obeid R, Multhaup G, Falkai P, Bayer TA (Cerebrospinal fluid diagnostic markers correlate with lower plasma copper and ceruloplasmin in patients with Alzheimer's disease. J Neural Transm 113:1763-1769.2006).
Kiuru S, Salonen O, Haltia M (Gelsolin-related spinal and cerebral amyloid angiopathy. Annals of neurology 45:305-311.1999).
Lee VM, Trojanowski JQ (The disordered neuronal cytoskeleton in Alzheimer's disease. Current opinion in neurobiology 2:653-656.1992).
Levy E, Haltia M, Fernandez-Madrid I, Koivunen O, Ghiso J, Prelli F, Frangione B (Mutation in gelsolin gene in Finnish hereditary amyloidosis. The Journal of experimental medicine 172:1865-1867.1990).
Loeffler DA, DeMaggio AJ, Juneau PL, Brickman CM, Mashour GA, Finkelman JH, Pomara N, LeWitt PA (Ceruloplasmin is increased in cerebrospinal fluid in Alzheimer's disease but not Parkinson's disease. Alzheimer disease and associated disorders 8:190-197.1994).
Loeffler DA, LeWitt PA, Juneau PL, Sima AA, Nguyen HU, DeMaggio AJ, Brickman CM, Brewer GJ, Dick RD, Troyer MD, Kanaley L (Increased regional brain concentrations of ceruloplasmin in neurodegenerative disorders. Brain research 738:265-274.1996).
Loeffler DA, Sima AA, LeWitt PA (Ceruloplasmin immunoreactivity in neurodegenerative disorders. Free radical research 35:111-118.2001).
Lu H, Yang Y, Allister EM, Wijesekara N, Wheeler MB (The identification of potential factors associated with the development of type 2 diabetes: A quantitative proteomic approach. Mol Cell Proteomics.2008).
Matsuoka Y, Saito M, LaFrancois J, Saito M, Gaynor K, Olm V, Wang L, Casey E, Lu Y, Shiratori C, Lemere C, Duff K (Novel therapeutic approach for the treatment of Alzheimer's disease by peripheral administration of agents with an affinity to beta-amyloid. J Neurosci 23:29-33.2003).
Matta A, DeSouza LV, Shukla NK, Gupta SD, Ralhan R, Siu KW (Prognostic significance of head-and-neck cancer biomarkers previously discovered and identified using iTRAQ-labeling and multidimensional liquid chromatography-tandem mass spectrometry. Journal of proteome research 7:2078-2087.2008).
Maury CP, Kere J, Tolvanen R, de la Chapelle A (Finnish hereditary amyloidosis is caused by a single nucleotide substitution in the gelsolin gene. FEBS letters 276:75-77.1990).
Maurya P, Meleady P, Dowling P, Clynes M (Proteomic approaches for serum biomarker discovery in cancer. Anticancer research 27:1247-1255.2007).
McGeer PL, McGeer EG (The possible role of complement activation in Alzheimer disease. Trends in molecular medicine 8:519-523.2002).
Qiao H, Koya RC, Nakagawa K, Tanaka H, Fujita H, Takimoto M, Kuzumaki N (Inhibition of Alzheimer's amyloid-beta peptide-induced reduction of mitochondrial membrane potential and neurotoxicity by gelsolin. Neurobiology of aging 26:849-855.2005).
Ralhan R, Desouza LV, Matta A, Chandra Tripathi S, Ghanny S, Datta Gupta S, Bahadur S, Siu KW (Discovery and verification of head-and-neck cancer biomarkers by differential protein expression analysis using iTRAQ labeling, multidimensional liquid chromatography, and tandem mass spectrometry. Mol Cell Proteomics 7:1162-1173.2008).
Ray I, Chauhan A, Wegiel J, Chauhan VP (Gelsolin inhibits the fibrillization of amyloid beta-protein, and also defibrillizes its preformed fibrils. Brain research 853:344-351.2000).
Ray S, Britschgi M, Herbert C, Takeda-Uchimura Y, Boxer A, Blennow K, Friedman LF, Galasko DR, Jutel M, Karydas A, Kaye JA, Leszek J, Miller BL, Minthon L, Quinn JF, Rabinovici GD, Robinson WH, Sabbagh MN, So YT, Sparks DL, Tabaton M, Tinklenberg J, Yesavage JA, Tibshirani R, Wyss-Coray T (Classification and prediction of clinical Alzheimer's diagnosis based on plasma signaling proteins. Nature medicine 13:1359-1362.2007).
Strohmeyer R, Ramirez M, Cole GJ, Mueller K, Rogers J (Association of factor H of the alternative pathway of complement with agrin and complement receptor 3 in the Alzheimer's disease brain. Journal of neuroimmunology 131:135-146.2002).
Sun HQ, Yamamoto M, Mejillano M, Yin HL (Gelsolin, a multifunctional actin regulatory protein. The Journal of biological chemistry 274:33179-33182.1999).
Thompson A, Schafer J, Kuhn K, Kienle S, Schwarz J, Schmidt G, Neumann T, Johnstone R, Mohammed AK, Hamon C (Tandem mass tags: a novel quantification strategy for comparative analysis of complex protein mixtures by MS/MS. Analytical chemistry 75:1895-1904.2003).
Veerhuis R, Janssen I, Hoozemans JJ, De Groot CJ, Hack CE, Eikelenboom P (Complement C1-inhibitor expression in Alzheimer's disease. Acta neuropathologica 96:287-296.1998).
Yasojima K, McGeer EG, McGeer PL (Complement regulators C1 inhibitor and CD59 do not significantly inhibit complement activation in Alzheimer disease. Brain research 833:297-301.1999).

## Claims

1. A method for aiding the diagnosis of Alzheimer's disease in a subject, said method comprising; providing a sample of a blood derivative, wherein said blood derivative is serum or plasma, obtained from said subject; assaying the amount of gelsolin present in said sample; comparing the amount of gelsolin present in said sample to a reference amount of gelsolin present in a sample from a healthy subject, wherein detection of a gelsolin level in the sample from said subject which is lower than the gelsolin level in the reference sample indicates an increased likelihood of Alzheimer's disease in said subject.

2. A method according to claim 1 wherein said sample comprises blood plasma.

3. A method according to claim 2 wherein said blood plasma has been depleted for one or more of albumin; transferrin; IgG; IgA; antitrypsin or haptoglobin.

4. A method according to claim 3, wherein said blood plasma has been depleted for each of albumin; transferrin; IgG: IgA; antitrypsin or haptoglobin.

5. A method according to any of claims 1 to 4 wherein the protein is detected by western blotting.

6. A method according to any of claims 1 to 4 wherein the protein is detected by bead suspension array.

7. A method according to any of claims 1 to 4 wherein the protein is detected by planar array.

8. A method according to any of claims 1 to 4 wherein the protein is detected by isobaric protein tagging or by isotopic protein tagging.

9. A method according to any of claims 1 to 4 or claim 8 wherein the protein is detected by mass spectrometer-based assay.

10. A method according to any of claims 1 to 4, 8 or 9 wherein the protein is gelsolin and is detected by reference to one or more of the following peptides of Table B: SEQ ID NO: 30, SEQ ID NO: 31, SEQ ID NO: 32.

## Patentansprüche

1. Verfahren zur Unterstützung der Diagnose von Morbus Alzheimer bei einer Versuchsperson, mit den folgenden Schritten: Bereitstellen einer Probe eines Blutderivates, wobei das Blutderivat ein Serum oder ein Plasma ist, das von der Versuchsperson erhalten wurde; Nachweisen der in dieser Probe vorliegenden Menge an Gelsolin; Vergleichen der in dieser Probe vorliegenden Menge an Gelsolin mit einer in einer Probe von einer gesunden Person vorliegenden Bezugsmenge an Gelsolin, wobei die Feststellung eines Gelsolinspiegels in der Probe von der Versuchsperson, der niedriger ist als der Gelsolinspiegel in der Bezugsprobe, eine erhöhte Wahrscheinlichkeit von Morbus Alzheimer bei dieser Versuchsperson anzeigt.

2. Verfahren gemäß Anspruch 1, wobei diese Probe Blutplasma enthält.

3. Verfahren gemäß Anspruch 2, wobei dem Blutplasma einer oder mehrere der Stoffe Albumin, Transferrin, IgG, IgA, Antitrypsin oder Haptoglobin entzogen worden ist.

4. Verfahren gemäß Anspruch 3, wobei dem Blutplasma jeweils Albumin, Transferrin, IgG, IgA, Antitrypsin oder Haptoglobin entzogen worden ist.

5. Verfahren gemäß einem der Ansprüche 1 bis 4, wobei das Protein durch die Western-Blot-Technik festgestellt wird.

6. Verfahren gemäß einem der Ansprüche 1 bis 4, wobei das Protein durch ein Kügelchensuspensionsarray festgestellt wird.

7. Verfahren gemäß einem der Ansprüche 1 bis 4, wobei das Protein durch ein Planararray festgestellt wird.

8. Verfahren gemäß einem der Ansprüche 1 bis 4, wobei das Protein durch isobare Proteinmarkierung oder durch isotope Proteinmarkierung festgestellt wird.

9. Verfahren gemäß einem der Ansprüche 1 bis 4 oder Anspruch 8, wobei das Protein durch ein Nachweisverfahren auf Massenspektrometerbasis festgestellt wird.

10. Verfahren gemäß einem der Ansprüche 1 bis 4, 8 oder 9, wobei das Protein Gelsolin ist und unter Bezug auf eines oder mehrere der folgenden Peptide der Tabelle B festgestellt wird: SEQ ID Nr. 30, SEQ ID Nr. 31, SEQ ID Nr. 32.

## Revendications

1. Procédé permettant d'aider au diagnostic de la maladie d'Alzheimer chez un sujet, ledit procédé comprenant les étapes consistant à : fournir un échantillon d'un dérivé sanguin, où ledit dérivé sanguin est du sérum ou du plasma, obtenu auprès dudit sujet ; doser la quantité de gelsoline présente dans ledit échantillon ; comparer la quantité de gelsoline présente dans ledit échantillon à une quantité de référence de gelsoline présente dans un échantillon d'un sujet sain, où une détection d'un niveau de gelsoline dans l'échantillon dudit sujet qui est inférieure au niveau de gelsoline dans l'échantillon de référence indique une probabilité accrue de maladie d'Alzheimer chez ledit sujet.

2. Procédé selon la revendication 1, dans lequel ledit échantillon comprend du plasma sanguin.

3. Procédé selon la revendication 2, dans lequel ledit plasma sanguin a été appauvri en une ou plusieurs protéines parmi l'albumine ; la transferrine ; l'IgG ; l'IgA, l'antitrypsine ou l'haptoglobine.

4. Procédé selon la revendication 3, dans lequel ledit plasma sanguin a été appauvri en chacune de l'albumine ; la transferrine ; l'IgG ; l'IgA, l'antitrypsine ou l'haptoglobine.

5. Procédé selon l'une quelconque des revendications 1 à 4, dans lequel la protéine est détectée par transfert de Western.

6. Procédé selon l'une quelconque des revendications 1 à 4, dans lequel la protéine est détectée par un arrangement de billes en suspension.

7. Procédé selon l'une quelconque des revendications 1 à 4, dans lequel la protéine est détectée par un arrangement planaire.

8. Procédé selon l'une quelconque des revendications 1 à 4, dans lequel la protéine est détectée par marquage isobarique de protéine ou par marquage isotopique de protéine.

9. Procédé selon l'une quelconque des revendications 1 à 4 ou selon la revendication 8, dans lequel la protéine est détectée par une analyse utilisant un spectromètre de masse.

10. Procédé selon l'une quelconque des revendications 1 à 4, 8 ou 9, dans lequel la protéine est la gelsoline et est détectée par référence à un ou plusieurs des peptides suivants du tableau B : ID SEQ. n° 30, ID SEQ. n° 31, ID SEQ. n° 32.


### SEQUENCE LISTING

<110> Electrophoretics Limited King's College London Lovestone, Simon H Guntert, Andreas C Campbell, James O'Brien, Darragh P W Byers, Helen L
<120> Methods
<130> P2864PC01
<160> 32
<170> PatentIn version 3.3
<210> 1
   <211> 782
   <212> PRT
   <213> Homo sapiens
<400> 1
<210> 2
   <211> 500
   <212> PRT
   <213> Homo sapiens
<400> 2
<210> 3
   <211> 1065
   <212> PRT
   <213> Homo sapiens
<400> 3
   Met Lys Ile Leu Ile Leu Gly Ile Phe Leu Phe Leu Cys Ser Thr Pro
   1               5                   10                  15
   Ala Trp Ala Lys Glu Lys His Tyr Tyr Ile Gly Ile Ile Glu Thr Thr
               20                  25                  30
   Trp Asp Tyr Ala Ser Asp His Gly Glu Lys Lys Leu Ile Ser Val Asp
           35                  40                  45
   Thr Glu His Ser Asn Ile Tyr Leu Gln Asn Gly Pro Asp Arg Ile Gly
       50                  55                  60
   Arg Leu Tyr Lys Lys Ala Leu Tyr Leu Gln Tyr Thr Asp Glu Thr Phe
   65                  70                  75                  80
   Arg Thr Thr Ile Glu Lys Pro Val Trp Leu Gly Phe Leu Gly Pro Ile
                   85                  90                  95
   Ile Lys Ala Glu Thr Gly Asp Lys Val Tyr Val His Leu Lys Asn Leu
               100                 105                 110
   Ala Ser Arg Pro Tyr Thr Phe His Ser His Gly Ile Thr Tyr Tyr Lys
           115                 120                 125
   Glu His Glu Gly Ala Ile Tyr Pro Asp Asn Thr Thr Asp Phe Gln Arg
       130                 135                 140
   Ala Asp Asp Lys Val Tyr Pro Gly Glu Gln Tyr Thr Tyr Met Leu Leu
   145                 150                 155                 160
   Ala Thr Glu Glu Gln Ser Pro Gly Glu Gly Asp Gly Asn Cys Val Thr
                   165                 170                 175
   Arg Ile Tyr His Ser His Ile Asp Ala Pro Lys Asp Ile Ala Ser Gly
               180                 185                 190
   Leu Ile Gly Pro Leu Ile Ile Cys Lys Lys Asp Ser Leu Asp Lys Glu
           195                 200                 205
   Lys Glu Lys His Ile Asp Arg Glu Phe Val Val Met Phe Ser Val Val
       210                 215                 220
   Asp Glu Asn Phe Ser Trp Tyr Leu Glu Asp Asn Ile Lys Thr Tyr Cys
   225                 230                 235                 240
   Ser Glu Pro Glu Lys Val Asp Lys Asp Asn Glu Asp Phe Gln Glu Ser
                   245                 250                 255
   Asn Arg Met Tyr Ser Val Asn Gly Tyr Thr Phe Gly Ser Leu Pro Gly
               260                 265                 270
   Leu Ser Met Cys Ala Glu Asp Arg Val Lys Trp Tyr Leu Phe Gly Met
           275                 280                 285
   Gly Asn Glu Val Asp Val His Ala Ala Phe Phe His Gly Gln Ala Leu
       290                 295                 300
   Thr Asn Lys Asn Tyr Arg Ile Asp Thr Ile Asn Leu Phe Pro Ala Thr
   305                 310                 315                 320
   Leu Phe Asp Ala Tyr Met Val Ala Gln Asn Pro Gly Glu Trp Met Leu
                   325                 330                 335
   Ser Cys Gln Asn Leu Asn His Leu Lys Ala Gly Leu Gln Ala Phe Phe
               340                 345                 350
   Gln Val Gln Glu Cys Asn Lys Ser Ser Ser Lys Asp Asn Ile Arg Gly
           355                 360                 365
   Lys His Val Arg His Tyr Tyr Ile Ala Ala Glu Glu Ile Ile Trp Asn
       370                 375                 380
   Tyr Ala Pro Ser Gly Ile Asp Ile Phe Thr Lys Glu Asn Leu Thr Ala
   385                 390                 395                 400
   Pro Gly Ser Asp Ser Ala Val Phe Phe Glu Gln Gly Thr Thr Arg Ile
                   405                 410                 415
   Gly Gly Ser Tyr Lys Lys Leu Val Tyr Arg Glu Tyr Thr Asp Ala Ser
               420                 425                 430
   Phe Thr Asn Arg Lys Glu Arg Gly Pro Glu Glu Glu His Leu Gly Ile
           435                 440                 445
   Leu Gly Pro Val Ile Trp Ala Glu Val Gly Asp Thr Ile Arg Val Thr
       450                 455                 460
   Phe His Asn Lys Gly Ala Tyr Pro Leu Ser Ile Glu Pro Ile Gly Val
   465                 470                 475                 480
   Arg Phe Asn Lys Asn Asn Glu Gly Thr Tyr Tyr Ser Pro Asn Tyr Asn
                   485                 490                 495
   Pro Gln Ser Arg Ser Val Pro Pro Ser Ala Ser His Val Ala Pro Thr
               500                 505                 510
   Glu Thr Phe Thr Tyr Glu Trp Thr Val Pro Lys Glu Val Gly Pro Thr
           515                 520                 525
   Asn Ala Asp Pro Val Cys Leu Ala Lys Met Tyr Tyr Ser Ala Val Asp
       530                 535                 540
   Pro Thr Lys Asp Ile Phe Thr Gly Leu Ile Gly Pro Met Lys Ile Cys
   545 550 555 560
   Lys Lys Gly Ser Leu His Ala Asn Gly Arg Gln Lys Asp Val Asp Lys
   565 570 575
   Glu Phe Tyr Leu Phe Pro Thr Val Phe Asp Glu Asn Glu Ser Leu Leu
   580 585 590
   Leu Glu Asp Asn Ile Arg Met Phe Thr Thr Ala Pro Asp Gln Val Asp
   595 600 605
   Lys Glu Asp Glu Asp Phe Gln Glu Ser Asn Lys Met His Ser Met Asn
   610 615 620
   Gly Phe Met Tyr Gly Asn Gln Pro Gly Leu Thr Met Cys Lys Gly Asp
   625 630 635 640
   Ser Val Val Trp Tyr Leu Phe Ser Ala Gly Asn Glu Ala Asp Val His
   645 650 655
   Gly Ile Tyr Phe Ser Gly Asn Thr Tyr Leu Trp Arg Gly Glu Arg Arg
   660 665 670
   Asp Thr Ala Asn Leu Phe Pro Gln Thr Ser Leu Thr Leu His Met Trp
   675 680 685
   Pro Asp Thr Glu Gly Thr Phe Asn Val Glu Cys Leu Thr Thr Asp His
   690 695 700
   Tyr Thr Gly Gly Met Lys Gln Lys Tyr Thr Val Asn Gln Cys Arg Arg
   705 710 715 720
   Gln Ser Glu Asp Ser Thr Phe Tyr Leu Gly Glu Arg Thr Tyr Tyr Ile
   725 730 735
   Ala Ala Val Glu Val Glu Trp Asp Tyr Ser Pro Gln Arg Glu Trp Glu
   740 745 750
   Lys Glu Leu His His Leu Gln Glu Gln Asn Val Ser Asn Ala Phe Leu
   755 760 765
   Asp Lys Gly Glu Phe Tyr Ile Gly Ser Lys Tyr Lys Lys Val Val Tyr
   770 775 780
   Arg Gln Tyr Thr Asp Ser Thr Phe Arg Val Pro Val Glu Arg Lys Ala
   785 790 795 800
   Glu Glu Glu His Leu Gly Ile Leu Gly Pro Gln Leu His Ala Asp Val
   805 810 815
   Gly Asp Lys Val Lys Ile Ile Phe Lys Asn Met Ala Thr Arg Pro Tyr
   820 825 830
   Ser Ile His Ala His Gly Val Gln Thr Glu Ser Ser Thr Val Thr Pro
   835 840 845
   Thr Leu Pro Gly Glu Thr Leu Thr Tyr Val Trp Lys Ile Pro Glu Arg
   850 855 860
   Ser Gly Ala Gly Thr Glu Asp Ser Ala Cys Ile Pro Trp Ala Tyr Tyr
   865 870 875 880
   Ser Thr Val Asp Gln Val Lys Asp Leu Tyr Ser Gly Leu Ile Gly Pro
   885 890 895
   Leu Ile Val Cys Arg Arg Pro Tyr Leu Lys Val Phe Asn Pro Arg Arg
   900 905 910
   Lys Leu Glu Phe Ala Leu Leu Phe Leu Val Phe Asp Glu Asn Glu Ser
   915 920 925
   Trp Tyr Leu Asp Asp Asn Ile Lys Thr Tyr Ser Asp His Pro Glu Lys
   930 935 940
   Val Asn Lys Asp Asp Glu Glu Phe Ile Glu Ser Asn Lys Met His Ala
   945 950 955 960
   Ile Asn Gly Arg Met Phe Gly Asn Leu Gln Gly Leu Thr Met His Val
   965 970 975
   Gly Asp Glu Val Asn Trp Tyr Leu Met Gly Met Gly Asn Glu Ile Asp
   980 985 990
   Leu His Thr Val His Phe His Gly His Ser Phe Gln Tyr Lys His Arg
   995 1000 1005
   Gly Val Tyr Ser Ser Asp Val Phe Asp Ile Phe Pro Gly Thr Tyr
   1010 1015 1020
   Gln Thr Leu Glu Met Phe Pro Arg Thr Pro Gly Ile Trp Leu Leu
   1025 1030 1035
   His Cys His Val Thr Asp His Ile His Ala Gly Met Glu Thr Thr
   1040 1045 1050
   Tyr Thr Val Leu Gln Asn Glu Asp Thr Lys Ser Gly
   1055 1060 1065
<210> 4
   <211> 449
   <212> PRT
   <213> Homo sapiens
<400> 4
   Met Met Lys Thr Leu Leu Leu Phe Val Gly Leu Leu Leu Thr Trp Glu
   1 5 10 15
   Ser Gly Gln Val Leu Gly Asp Gln Thr Val Ser Asp Asn Glu Leu Gln
   20 25 30
   Glu Met Ser Asn Gln Gly Ser Lys Tyr Val Asn Lys Glu Ile Gln Asn
   35 40 45
   Ala Val Asn Gly Val Lys Gln Ile Lys Thr Leu Ile Glu Lys Thr Asn
   50 55 60
   Glu Glu Arg Lys Thr Leu Leu Ser Asn Leu Glu Glu Ala Lys Lys Lys
   65 70 75 80
   Lys Glu Asp Ala Leu Asn Glu Thr Arg Glu Ser Glu Thr Lys Leu Lys
   85 90 95
   Glu Leu Pro Gly Val Cys Asn Glu Thr Met Met Ala Leu Trp Glu Glu
   100 105 110
   Cys Lys Pro Cys Leu Lys Gln Thr Cys Met Lys Phe Tyr Ala Arg Val
   115 120 125
   Cys Arg Ser Gly Ser Gly Leu Val Gly Arg Gln Leu Glu Glu Phe Leu
   130 135 140
   Asn Gln Ser Ser Pro Phe Tyr Phe Trp Met Asn Gly Asp Arg Ile Asp
   145 150 155 160
   Ser Leu Leu Glu Asn Asp Arg Gln Gln Thr His Met Leu Asp Val Met
   165 170 175
   Gln Asp His Phe Ser Arg Ala Ser Ser Ile Ile Asp Glu Leu Phe Gln
   180 185 190
   Asp Arg Phe Phe Thr Arg Glu Pro Gln Asp Thr Tyr His Tyr Leu Pro
   195 200 205
   Phe Ser Leu Pro His Arg Arg Pro His Phe Phe Phe Pro Lys Ser Arg
   210 215 220
   Ile Val Arg Ser Leu Met Pro Phe Ser Pro Tyr Glu Pro Leu Asn Phe
   225 230 235 240
   His Ala Met Phe Gln Pro Phe Leu Glu Met Ile His Glu Ala Gln Gln
   245 250 255
   Ala Met Asp Ile His Phe His Ser Pro Ala Phe Gln His Pro Pro Thr
   260 265 270
   Glu Phe Ile Arg Glu Gly Asp Asp Asp Arg Thr Val Cys Arg Glu Ile
   275 280 285
   Arg His Asn Ser Thr Gly Cys Leu Arg Met Lys Asp Gln Cys Asp Lys
       290                 295                 300
   Cys Arg Glu Ile Leu Ser Val Asp Cys Ser Thr Asn Asn Pro Ser Gln
   305                 310                 315                 320
   Ala Lys Leu Arg Arg Glu Leu Asp Glu Ser Leu Gln Val Ala Glu Arg
                   325                 330                 335
   Leu Thr Arg Lys Tyr Asn Glu Leu Leu Lys Ser Tyr Gln Trp Lys Met
               340                 345                 350
   Leu Asn Thr Ser Ser Leu Leu Glu Gln Leu Asn Glu Gln Phe Asn Trp
           355                 360                 365
   Val Ser Arg Leu Ala Asn Leu Thr Gln Gly Glu Asp Gln Tyr Tyr Leu
       370                 375                 380
   Arg Val Thr Thr Val Ala Ser His Thr Ser Asp Ser Asp Val Pro Ser
   385                 390                 395                 400
   Gly Val Thr Glu Val Val Val Lys Leu Phe Asp Ser Asp Pro Ile Thr
                   405                 410                 415
   Val Thr Val Pro Val Glu Val Ser Arg Lys Asn Pro Lys Phe Met Glu
               420                 425                 430
   Thr Val Ala Glu Lys Ala Leu Gln Glu Tyr Arg Lys Lys His Arg Glu
           435                 440                 445
   Glu
<210> 5
   <211> 1663
   <212> PRT
   <213> Homo sapiens
<400> 5
<210> 6
   <211> 223
   <212> PRT
   <213> Homo sapiens
<400> 6
   Met Asn Lys Pro Leu Leu Trp Ile Ser Val Leu Thr Ser Leu Leu Glu
   1               5                   10                  15
   Ala Phe Ala His Thr Asp Leu Ser Gly Lys Val Phe Val Phe Pro Arg
               20                  25                  30
   Glu Ser Val Thr Asp His Val Asn Leu Ile Thr Pro Leu Glu Lys Pro
           35                  40                  45
   Leu Gln Asn Phe Thr Leu Cys Phe Arg Ala Tyr Ser Asp Leu Ser Arg
       50                  55                  60
   Ala Tyr Ser Leu Phe Ser Tyr Asn Thr Gln Gly Arg Asp Asn Glu Leu
   65                  70                  75                  80
   Leu Val Tyr Lys Glu Arg Val Gly Glu Tyr Ser Leu Tyr Ile Gly Arg
                   85                  90                  95
   His Lys Val Thr Ser Lys Val Ile Glu Lys Phe Pro Ala Pro Val His
               100                 105                 110
   Ile Cys Val Ser Trp Glu Ser Ser Ser Gly Ile Ala Glu Phe Trp Ile
           115                 120                 125
   Asn Gly Thr Pro Leu Val Lys Lys Gly Leu Arg Gln Gly Tyr Phe Val
       130                 135                 140
   Glu Ala Gln Pro Lys Ile Val Leu Gly Gln Glu Gln Asp Ser Tyr Gly
   145                 150                 155                 160
   Gly Lys Phe Asp Arg Ser Gln Ser Phe Val Gly Glu Ile Gly Asp Leu
                   165                 170                 175
   Tyr Met Trp Asp Ser Val Leu Pro Pro Glu Asn Ile Leu Ser Ala Tyr
               180                 185                 190
   Gln Gly Thr Pro Leu Pro Ala Asn Ile Leu Asp Trp Gln Ala Leu Asn
           195                 200                 205
   Tyr Glu Ile Arg Gly Tyr Val Ile Ile Lys Pro Leu Val Trp Val
       210                 215                 220
<210> 7
   <211> 1474
   <212> PRT
   <213> Homo sapiens
<400> 7
   Met Gly Lys Asn Lys Leu Leu His Pro Ser Leu Val Leu Leu Leu Leu
   1               5                   10                  15
   Val Leu Leu Pro Thr Asp Ala Ser Val Ser Gly Lys Pro Gln Tyr Met
               20                  25                  30
   Val Leu Val Pro Ser Leu Leu His Thr Glu Thr Thr Glu Lys Gly Cys
           35                  40                  45
   Val Leu Leu Ser Tyr Leu Asn Glu Thr Val Thr Val Ser Ala Ser Leu
       50                  55                  60
   Glu Ser Val Arg Gly Asn Arg Ser Leu Phe Thr Asp Leu Glu Ala Glu
   65                  70                  75                  80
   Asn Asp Val Leu His Cys Val Ala Phe Ala Val Pro Lys Ser Ser Ser
                   85                  90                  95
   Asn Glu Glu Val Met Phe Leu Thr Val Gln Val Lys Gly Pro Thr Gln
               100                 105                 110
   Glu Phe Lys Lys Arg Thr Thr Val Met Val Lys Asn Glu Asp Ser Leu
           115                 120                 125
   Val Phe Val Gln Thr Asp Lys Ser Ile Tyr Lys Pro Gly Gln Thr Val
       130                 135                 140
   Lys Phe Arg Val Val Ser Met Asp Glu Asn Phe His Pro Leu Asn Glu
   145                 150                 155                 160
   Leu Ile Pro Leu Val Tyr Ile Gln Asp Pro Lys Gly Asn Arg Ile Ala
                   165                 170                 175
   Gln Trp Gln Ser Phe Gln Leu Glu Gly Gly Leu Lys Gln Phe Ser Phe
               180                 185                 190
   Pro Leu Ser Ser Glu Pro Phe Gln Gly Ser Tyr Lys Val Val Val Gln
           195                 200                 205
   Lys Lys Ser Gly Gly Arg Thr Glu His Pro Phe Thr Val Glu Glu Phe
       210                 215                 220
   Val Leu Pro Lys Phe Glu Val Gln Val Thr Val Pro Lys Ile Ile Thr
   225                 230                 235                 240
   Ile Leu Glu Glu Glu Met Asn Val Ser Val Cys Gly Leu Tyr Thr Tyr
                   245                 250                 255
   Gly Lys Pro Val Pro Gly His Val Thr Val Ser Ile Cys Arg Lys Tyr
               260                 265                 270
   Ser Asp Ala Ser Asp Cys His Gly Glu Asp Ser Gln Ala Phe Cys Glu
           275                 280                 285
   Lys Phe Ser Gly Gln Leu Asn Ser His Gly Cys Phe Tyr Gln Gln Val
       290                 295                 300
   Lys Thr Lys Val Phe Gln Leu Lys Arg Lys Glu Tyr Glu Met Lys Leu
   305                 310                 315                 320
   His Thr Glu Ala Gln Ile Gln Glu Glu Gly Thr Val Val Glu Leu Thr
                   325                 330                 335
   Gly Arg Gln Ser Ser Glu Ile Thr Arg Thr Ile Thr Lys Leu Ser Phe
               340                 345                 350
   Val Lys Val Asp Ser His Phe Arg Gln Gly Ile Pro Phe Phe Gly Gln
           355                 360                 365
   Val Arg Leu Val Asp Gly Lys Gly Val Pro Ile Pro Asn Lys Val Ile
   370 375 380
   Phe Ile Arg Gly Asn Glu Ala Asn Tyr Tyr Ser Asn Ala Thr Thr Asp
   385 390 395 400
   Glu His Gly Leu Val Gln Phe Ser Ile Asn Thr Thr Asn Val Met Gly
   405 410 415
   Thr Ser Leu Thr Val Arg Val Asn Tyr Lys Asp Arg Ser Pro Cys Tyr
   420 425 430
   Gly Tyr Gln Trp Val Ser Glu Glu His Glu Glu Ala His His Thr Ala
   435 440 445
   Tyr Leu Val Phe Ser Pro Ser Lys Ser Phe Val His Leu Glu Pro Met
   450 455 460
   Ser His Glu Leu Pro Cys Gly His Thr Gln Thr Val Gln Ala His Tyr
   465 470 475 480
   Ile Leu Asn Gly Gly Thr Leu Leu Gly Leu Lys Lys Leu Ser Phe Tyr
   485 490 495
   Tyr Leu Ile Met Ala Lys Gly Gly Ile Val Arg Thr Gly Thr His Gly
   500 505 510
   Leu Leu Val Lys Gln Glu Asp Met Lys Gly His Phe Ser Ile Ser Ile
   515 520 525
   Pro Val Lys Ser Asp Ile Ala Pro Val Ala Arg Leu Leu Ile Tyr Ala
   530 535 540
   Val Leu Pro Thr Gly Asp Val Ile Gly Asp Ser Ala Lys Tyr Asp Val
   545 550 555 560
   Glu Asn Cys Leu Ala Asn Lys Val Asp Leu Ser Phe Ser Pro Ser Gln
   565 570 575
   Ser Leu Pro Ala Ser His Ala His Leu Arg Val Thr Ala Ala Pro Gln
   580 585 590
   Ser Val Cys Ala Leu Arg Ala Val Asp Gln Ser Val Leu Leu Met Lys
   595 600 605
   Pro Asp Ala Glu Leu Ser Ala Ser Ser Val Tyr Asn Leu Leu Pro Glu
   610 615 620
   Lys Asp Leu Thr Gly Phe Pro Gly Pro Leu Asn Asp Gln Asp Asp Glu
   625 630 635 640
   Asp Cys Ile Asn Arg His Asn Val Tyr Ile Asn Gly Ile Thr Tyr Thr
   645 650 655
   Pro Val Ser Ser Thr Asn Glu Lys Asp Met Tyr Ser Phe Leu Glu Asp
   660 665 670
   Met Gly Leu Lys Ala Phe Thr Asn Ser Lys Ile Arg Lys Pro Lys Met
   675 680 685
   Cys Pro Gln Leu Gln Gln Tyr Glu Met His Gly Pro Glu Gly Leu Arg
   690 695 700
   Val Gly Phe Tyr Glu Ser Asp Val Met Gly Arg Gly His Ala Arg Leu
   705 710 715 720
   Val His Val Glu Glu Pro His Thr Glu Thr Val Arg Lys Tyr Phe Pro
   725 730 735
   Glu Thr Trp Ile Trp Asp Leu Val Val Val Asn Ser Ala Gly Val Ala
   740 745 750
   Glu Val Gly Val Thr Val Pro Asp Thr Ile Thr Glu Trp Lys Ala Gly
   755 760 765
   Ala Phe Cys Leu Ser Glu Asp Ala Gly Leu Gly Ile Ser Ser Thr Ala
   770 775 780
   Ser Leu Arg Ala Phe Gln Pro Phe Phe Val Glu Leu Thr Met Pro Tyr
   785 790 795 800
   Ser Val Ile Arg Gly Glu Ala Phe Thr Leu Lys Ala Thr Val Leu Asn
   805 810 815
   Tyr Leu Pro Lys Cys Ile Arg Val Ser Val Gln Leu Glu Ala Ser Pro
   820 825 830
   Ala Phe Leu Ala Val Pro Val Glu Lys Glu Gln Ala Pro His Cys Ile
   835 840 845
   Cys Ala Asn Gly Arg Gln Thr Val Ser Trp Ala Val Thr Pro Lys Ser
   850 855 860
   Leu Gly Asn Val Asn Phe Thr Val Ser Ala Glu Ala Leu Glu Ser Gln
   865 870 875 880
   Glu Leu Cys Gly Thr Glu Val Pro Ser Val Pro Glu His Gly Arg Lys
   885 890 895
   Asp Thr Val Ile Lys Pro Leu Leu Val Glu Pro Glu Gly Leu Glu Lys
   900 905 910
   Glu Thr Thr Phe Asn Ser Leu Leu Cys Pro Ser Gly Gly Glu Val Ser
   915 920 925
   Glu Glu Leu Ser Leu Lys Leu Pro Pro Asn Val Val Glu Glu Ser Ala
   930 935 940
   Arg Ala Ser Val Ser Val Leu Gly Asp Ile Leu Gly Ser Ala Met Gln
   945 950 955 960
   Asn Thr Gln Asn Leu Leu Gln Met Pro Tyr Gly Cys Gly Glu Gln Asn
   965 970 975
   Met Val Leu Phe Ala Pro Asn Ile Tyr Val Leu Asp Tyr Leu Asn Glu
   980 985 990
   Thr Gln Gln Leu Thr Pro Glu Ile Lys Ser Lys Ala Ile Gly Tyr Leu
   995 1000 1005
   Asn Thr Gly Tyr Gln Arg Gln Leu Asn Tyr Lys His Tyr Asp Gly
   1010 1015 1020
   Ser Tyr Ser Thr Phe Gly Glu Arg Tyr Gly Arg Asn Gln Gly Asn
   1025 1030 1035
   Thr Trp Leu Thr Ala Phe Val Leu Lys Thr Phe Ala Gln Ala Arg
   1040 1045 1050
   Ala Tyr Ile Phe Ile Asp Glu Ala His Ile Thr Gln Ala Leu Ile
   1055 1060 1065
   Trp Leu Ser Gln Arg Gln Lys Asp Asn Gly Cys Phe Arg Ser Ser
   1070 1075 1080
   Gly Ser Leu Leu Asn Asn Ala Ile Lys Gly Gly Val Glu Asp Glu
   1085 1090 1095
   Val Thr Leu Ser Ala Tyr Ile Thr Ile Ala Leu Leu Glu Ile Pro
   1100 1105 1110
   Leu Thr Val Thr His Pro Val Val Arg Asn Ala Leu Phe Cys Leu
   1115 1120 1125
   Glu Ser Ala Trp Lys Thr Ala Gln Glu Gly Asp His Gly Ser His
   1130 1135 1140
   Val Tyr Thr Lys Ala Leu Leu Ala Tyr Ala Phe Ala Leu Ala Gly
   1145 1150 1155
   Asn Gln Asp Lys Arg Lys Glu Val Leu Lys Ser Leu Asn Glu Glu
   1160 1165 1170
   Ala Val Lys Lys Asp Asn Ser Val His Trp Glu Arg Pro Gln Lys
   1175 1180 1185
   Pro Lys Ala Pro Val Gly His Phe Tyr Glu Pro Gln Ala Pro Ser
   1190 1195 1200
   Ala Glu Val Glu Met Thr Ser Tyr Val Leu Leu Ala Tyr Leu Thr
   1205 1210 1215
   Ala Gln Pro Ala Pro Thr Ser Glu Asp Leu Thr Ser Ala Thr Asn
   1220 1225 1230
   Ile Val Lys Trp Ile Thr Lys Gln Gln Asn Ala Gln Gly Gly Phe
   1235 1240 1245
   Ser Ser Thr Gln Asp Thr Val Val Ala Leu His Ala Leu Ser Lys
   1250 1255 1260
   Tyr Gly Ala Ala Thr Phe Thr Arg Thr Gly Lys Ala Ala Gln Val
   1265 1270 1275
   Thr Ile Gln Ser Ser Gly Thr Phe Ser Ser Lys Phe Gln Val Asp
   1280 1285 1290
   Asn Asn Asn Arg Leu Leu Leu Gln Gln Val Ser Leu Pro Glu Leu
   1295 1300 1305
   Pro Gly Glu Tyr Ser Met Lys Val Thr Gly Glu Gly Cys Val Tyr
   1310 1315 1320
   Leu Gln Thr Ser Leu Lys Tyr Asn Ile Leu Pro Glu Lys Glu Glu
   1325 1330 1335
   Phe Pro Phe Ala Leu Gly Val Gln Thr Leu Pro Gln Thr Cys Asp
   1340 1345 1350
   Glu Pro Lys Ala His Thr Ser Phe Gln Ile Ser Leu Ser Val Ser
   1355 1360 1365
   Tyr Thr Gly Ser Arg Ser Ala Ser Asn Met Ala Ile Val Asp Val
   1370 1375 1380
   Lys Met Val Ser Gly Phe Ile Pro Leu Lys Pro Thr Val Lys Met
   1385 1390 1395
   Leu Glu Arg Ser Asn His Val Ser Arg Thr Glu Val Ser Ser Asn
   1400 1405 1410
   His Val Leu Ile Tyr Leu Asp Lys Val Ser Asn Gln Thr Leu Ser
   1415 1420 1425
   Leu Phe Phe Thr Val Leu Gln Asp Val Pro Val Arg Asp Leu Lys
   1430 1435 1440
   Pro Ala Ile Val Lys Val Tyr Asp Tyr Tyr Glu Thr Asp Glu Phe
   1445 1450 1455
   Ala Ile Ala Glu Tyr Asn Ala Pro Cys Ser Lys Asp Leu Gly Asn
   1460 1465 1470
   Ala
<210> 8
   <211> 453
   <212> PRT
   <213> Homo sapiens
<400> 8
   Met Ser Trp Ser Leu His Pro Arg Asn Leu Ile Leu Tyr Phe Tyr Ala
   1               5                   10                  15
   Leu Leu Phe Leu Ser Ser Thr Cys Val Ala Tyr Val Ala Thr Arg Asp
               20                  25                  30
   Asn Cys Cys Ile Leu Asp Glu Arg Phe Gly Ser Tyr Cys Pro Thr Thr
           35                  40                  45
   Cys Gly Ile Ala Asp Phe Leu Ser Thr Tyr Gln Thr Lys Val Asp Lys
       50                  55                  60
   Asp Leu Gln Ser Leu Glu Asp Ile Leu His Gln Val Glu Asn Lys Thr
   65                  70                  75                  80
   Ser Glu Val Lys Gln Leu Ile Lys Ala Ile Gln Leu Thr Tyr Asn Pro
                   85                  90                  95
   Asp Glu Ser Ser Lys Pro Asn Met Ile Asp Ala Ala Thr Leu Lys Ser
               100                 105                 110
   Arg Lys Met Leu Glu Glu Ile Met Lys Tyr Glu Ala Ser Ile Leu Thr
           115                 120                 125
   His Asp Ser Ser Ile Arg Tyr Leu Gln Glu Ile Tyr Asn Ser Asn Asn
       130                 135                 140
   Gln Lys Ile Val Asn Leu Lys Glu Lys Val Ala Gln Leu Glu Ala Gln
   145                 150                 155                 160
   Cys Gln Glu Pro Cys Lys Asp Thr Val Gln Ile His Asp Ile Thr Gly
                   165                 170                 175
   Lys Asp Cys Gln Asp Ile Ala Asn Lys Gly Ala Lys Gln Ser Gly Leu
               180                 185                 190
   Tyr Phe Ile Lys Pro Leu Lys Ala Asn Gln Gln Phe Leu Val Tyr Cys
           195                 200                 205
   Glu Ile Asp Gly Ser Gly Asn Gly Trp Thr Val Phe Gln Lys Arg Leu
       210                 215                 220
   Asp Gly Ser Val Asp Phe Lys Lys Asn Trp Ile Gln Tyr Lys Glu Gly
   225                 230                 235                 240
   Phe Gly His Leu Ser Pro Thr Gly Thr Thr Glu Phe Trp Leu Gly Asn
                   245                 250                 255
   Glu Lys Ile His Leu Ile Ser Thr Gln Ser Ala Ile Pro Tyr Ala Leu
               260                 265                 270
   Arg Val Glu Leu Glu Asp Trp Asn Gly Arg Thr Ser Thr Ala Asp Tyr
           275                 280                 285
   Ala Met Phe Lys Val Gly Pro Glu Ala Asp Lys Tyr Arg Leu Thr Tyr
       290                 295                 300
   Ala Tyr Phe Ala Gly Gly Asp Ala Gly Asp Ala Phe Asp Gly Phe Asp
   305                 310                 315                 320
   Phe Gly Asp Asp Pro Ser Asp Lys Phe Phe Thr Ser His Asn Gly Met
                   325                 330                 335
   Gln Phe Ser Thr Trp Asp Asn Asp Asn Asp Lys Phe Glu Gly Asn Cys
               340                 345                 350
   Ala Glu Gln Asp Gly Ser Gly Trp Trp Met Asn Lys Cys His Ala Gly
           355                 360                 365
   His Leu Asn Gly Val Tyr Tyr Gln Gly Gly Thr Tyr Ser Lys Ala Ser
       370                 375                 380
   Thr Pro Asn Gly Tyr Asp Asn Gly Ile Ile Trp Ala Thr Trp Lys Thr
   385                 390                 395                 400
   Arg Trp Tyr Ser Met Lys Lys Thr Thr Met Lys Ile Ile Pro Phe Asn
                   405                 410                 415
   Arg Leu Thr Ile Gly Glu Gly Gln Gln His His Leu Gly Gly Ala Lys
               420                 425                 430
   Gln Val Arg Pro Glu His Pro Ala Glu Thr Glu Tyr Asp Ser Leu Tyr
           435                 440                 445
   Pro Glu Asp Asp Leu
       450
<210> 9
   <211> 1231
   <212> PRT
   <213> Homo sapiens
<400> 9
   Met Arg Leu Leu Ala Lys Ile Ile Cys Leu Met Leu Trp Ala Ile Cys
   1               5                   10                  15
   Val Ala Glu Asp Cys Asn Glu Leu Pro Pro Arg Arg Asn Thr Glu Ile
               20                  25                  30
   Leu Thr Gly Ser Trp Ser Asp Gln Thr Tyr Pro Glu Gly Thr Gln Ala
           35                  40                  45
   Ile Tyr Lys Cys Arg Pro Gly Tyr Arg Ser Leu Gly Asn Val Ile Met
       50                  55                  60
   Val Cys Arg Lys Gly Glu Trp Val Ala Leu Asn Pro Leu Arg Lys Cys
   65                  70                  75                  80
   Gln Lys Arg Pro Cys Gly His Pro Gly Asp Thr Pro Phe Gly Thr Phe
                   85                  90                  95
   Thr Leu Thr Gly Gly Asn Val Phe Glu Tyr Gly Val Lys Ala Val Tyr
               100                 105                 110
   Thr Cys Asn Glu Gly Tyr Gln Leu Leu Gly Glu Ile Asn Tyr Arg Glu
           115                 120                 125
   Cys Asp Thr Asp Gly Trp Thr Asn Asp Ile Pro Ile Cys Glu Val Val
       130                 135                 140
   Lys Cys Leu Pro Val Thr Ala Pro Glu Asn Gly Lys Ile Val Ser Ser
   145                 150                 155                 160
   Ala Met Glu Pro Asp Arg Glu Tyr His Phe Gly Gln Ala Val Arg Phe
                   165                 170                 175
   Val Cys Asn Ser Gly Tyr Lys Ile Glu Gly Asp Glu Glu Met His Cys
               180                 185                 190
   Ser Asp Asp Gly Phe Trp Ser Lys Glu Lys Pro Lys Cys Val Glu Ile
           195                 200                 205
   Ser Cys Lys Ser Pro Asp Val Ile Asn Gly Ser Pro Ile Ser Gln Lys
       210                 215                 220
   Ile Ile Tyr Lys Glu Asn Glu Arg Phe Gln Tyr Lys Cys Asn Met Gly
   225                 230                 235                 240
   Tyr Glu Tyr Ser Glu Arg Gly Asp Ala Val Cys Thr Glu Ser Gly Trp
                   245                 250                 255
   Arg Pro Leu Pro Ser Cys Glu Glu Lys Ser Cys Asp Asn Pro Tyr Ile
               260                 265                 270
   Pro Asn Gly Asp Tyr Ser Pro Leu Arg Ile Lys His Arg Thr Gly Asp
           275                 280                 285
   Glu Ile Thr Tyr Gln Cys Arg Asn Gly Phe Tyr Pro Ala Thr Arg Gly
       290                 295                 300
   Asn Thr Ala Lys Cys Thr Ser Thr Gly Trp Ile Pro Ala Pro Arg Cys
   305 310 315 320
   Thr Leu Lys Pro Cys Asp Tyr Pro Asp Ile Lys His Gly Gly Leu Tyr
   325 330 335
   His Glu Asn Met Arg Arg Pro Tyr Phe Pro Val Ala Val Gly Lys Tyr
   340 345 350
   Tyr Ser Tyr Tyr Cys Asp Glu His Phe Glu Thr Pro Ser Gly Ser Tyr
   355 360 365
   Trp Asp His Ile His Cys Thr Gln Asp Gly Trp Ser Pro Ala Val Pro
   370 375 380
   Cys Leu Arg Lys Cys Tyr Phe Pro Tyr Leu Glu Asn Gly Tyr Asn Gln
   385 390 395 400
   Asn Tyr Gly Arg Lys Phe Val Gln Gly Lys Ser Ile Asp Val Ala Cys
   405 410 415
   His Pro Gly Tyr Ala Leu Pro Lys Ala Gln Thr Thr Val Thr Cys Met
   420 425 430
   Glu Asn Gly Trp Ser Pro Thr Pro Arg Cys Ile Arg Val Lys Thr Cys
   435 440 445
   Ser Lys Ser Ser Ile Asp Ile Glu Asn Gly Phe Ile Ser Glu Ser Gln
   450 455 460
   Tyr Thr Tyr Ala Leu Lys Glu Lys Ala Lys Tyr Gln Cys Lys Leu Gly
   465 470 475 480
   Tyr Val Thr Ala Asp Gly Glu Thr Ser Gly Ser Ile Thr Cys Gly Lys
   485 490 495
   Asp Gly Trp Ser Ala Gln Pro Thr Cys Ile Lys Ser Cys Asp Ile Pro
   500 505 510
   Val Phe Met Asn Ala Arg Thr Lys Asn Asp Phe Thr Trp Phe Lys Leu
   515 520 525
   Asn Asp Thr Leu Asp Tyr Glu Cys His Asp Gly Tyr Glu Ser Asn Thr
   530 535 540
   Gly Ser Thr Thr Gly Ser Ile Val Cys Gly Tyr Asn Gly Trp Ser Asp
   545 550 555 560
   Leu Pro Ile Cys Tyr Glu Arg Glu Cys Glu Leu Pro Lys Ile Asp Val
   565 570 575
   His Leu Val Pro Asp Arg Lys Lys Asp Gln Tyr Lys Val Gly Glu Val
   580 585 590
   Leu Lys Phe Ser Cys Lys Pro Gly Phe Thr Ile Val Gly Pro Asn Ser
           595                 600                 605
   Val Gln Cys Tyr His Phe Gly Leu Ser Pro Asp Leu Pro Ile Cys Lys
       610                 615                 620
   Glu Gln Val Gln Ser Cys Gly Pro Pro Pro Glu Leu Leu Asn Gly Asn
   625                 630                 635                 640
   Val Lys Glu Lys Thr Lys Glu Glu Tyr Gly His Ser Glu Val Val Glu
                   645                 650                 655
   Tyr Tyr Cys Asn Pro Arg Phe Leu Met Lys Gly Pro Asn Lys Ile Gln
               660                 665                 670
   Cys Val Asp Gly Glu Trp Thr Thr Leu Pro Val Cys Ile Val Glu Glu
           675                 680                 685
   Ser Thr Cys Gly Asp Ile Pro Glu Leu Glu His Gly Trp Ala Gln Leu
       690                 695                 700
   Ser Ser Pro Pro Tyr Tyr Tyr Gly Asp Ser Val Glu Phe Asn Cys Ser
   705                 710                 715                 720
   Glu Ser Phe Thr Met Ile Gly His Arg Ser Ile Thr Cys Ile His Gly
                   725                 730                 735
   Val Trp Thr Gln Leu Pro Gln Cys Val Ala Ile Asp Lys Leu Lys Lys
               740                 745                 750
   Cys Lys Ser Ser Asn Leu Ile Ile Leu Glu Glu His Leu Lys Asn Lys
           755                 760                 765
   Lys Glu Phe Asp His Asn Ser Asn Ile Arg Tyr Arg Cys Arg Gly Lys
       770                 775                 780
   Glu Gly Trp Ile His Thr Val Cys Ile Asn Gly Arg Trp Asp Pro Glu
   785                 790                 795                 800
   Val Asn Cys Ser Met Ala Gln Ile Gln Leu Cys Pro Pro Pro Pro Gln
                   805                 810                 815
   Ile Pro Asn Ser His Asn Met Thr Thr Thr Leu Asn Tyr Arg Asp Gly
               820                 825                 830
   Glu Lys Val Ser Val Leu Cys Gln Glu Asn Tyr Leu Ile Gln Glu Gly
           835                 840                 845
   Glu Glu Ile Thr Cys Lys Asp Gly Arg Trp Gln Ser Ile Pro Leu Cys
       850                 855                 860
   Val Glu Lys Ile Pro Cys Ser Gln Pro Pro Gln Ile Glu His Gly Thr
   865                 870                 875                 880
   Ile Asn Ser Ser Arg Ser Ser Gln Glu Ser Tyr Ala His Gly Thr Lys
   885 890 895
   Leu Ser Tyr Thr Cys Glu Gly Gly Phe Arg Ile Ser Glu Glu Asn Glu
   900 905 910
   Thr Thr Cys Tyr Met Gly Lys Trp Ser Ser Pro Pro Gln Cys Glu Gly
   915 920 925
   Leu Pro Cys Lys Ser Pro Pro Glu Ile Ser His Gly Val Val Ala His
   930 935 940
   Met Ser Asp Ser Tyr Gln Tyr Gly Glu Glu Val Thr Tyr Lys Cys Phe
   945 950 955 960
   Glu Gly Phe Gly Ile Asp Gly Pro Ala Ile Ala Lys Cys Leu Gly Glu
   965 970 975
   Lys Trp Ser His Pro Pro Ser Cys Ile Lys Thr Asp Cys Leu Ser Leu
   980 985 990
   Pro Ser Phe Glu Asn Ala Ile Pro Met Gly Glu Lys Lys Asp Val Tyr
   995 1000 1005
   Lys Ala Gly Glu Gln Val Thr Tyr Thr Cys Ala Thr Tyr Tyr Lys
   1010 1015 1020
   Met Asp Gly Ala Ser Asn Val Thr Cys Ile Asn Ser Arg Trp Thr
   1025 1030 1035
   Gly Arg Pro Thr Cys Arg Asp Thr Ser Cys Val Asn Pro Pro Thr
   1040 1045 1050
   Val Gln Asn Ala Tyr Ile Val Ser Arg Gln Met Ser Lys Tyr Pro
   1055 1060 1065
   Ser Gly Glu Arg Val Arg Tyr Gln Cys Arg Ser Pro Tyr Glu Met
   1070 1075 1080
   Phe Gly Asp Glu Glu Val Met Cys Leu Asn Gly Asn Trp Thr Glu
   1085 1090 1095
   Pro Pro Gln Cys Lys Asp Ser Thr Gly Lys Cys Gly Pro Pro Pro
   1100 1105 1110
   Pro Ile Asp Asn Gly Asp Ile Thr Ser Phe Pro Leu Ser Val Tyr
   1115 1120 1125
   Ala Pro Ala Ser Ser Val Glu Tyr Gln Cys Gln Asn Leu Tyr Gln
   1130 1135 1140
   Leu Glu Gly Asn Lys Arg Ile Thr Cys Arg Asn Gly Gln Trp Ser
   1145 1150 1155
   Glu Pro Pro Lys Cys Leu His Pro Cys Val Ile Ser Arg Glu Ile
   1160 1165 1170
   Met Glu Asn Tyr Asn Ile Ala Leu Arg Trp Thr Ala Lys Gln Lys
   1175 1180 1185
   Leu Tyr Ser Arg Thr Gly Glu Ser Val Glu Phe Val Cys Lys Arg
   1190 1195 1200
   Gly Tyr Arg Leu Ser Ser Arg Ser His Thr Leu Arg Thr Thr Cys
   1205 1210 1215
   Trp Asp Gly Lys Leu Glu Tyr Pro Thr Cys Ala Lys Arg
   1220 1225 1230
<210> 10
   <211> 317
   <212> PRT
   <213> Homo sapiens
<400> 10
<210> 11
   <211> 10
   <212> PRT
   <213> Artificial
<220>
   <223> peptide
<400> 11
   Thr Leu Leu Ser Asn Leu Glu Glu Ala Lys
   1 5 10
<210> 12
   <211> 9
   <212> PRT
   <213> Artificial
<220>
   <223> peptide
<400> 12
   Ile Asp Ser Leu Leu Glu Asn Asp Arg
   1 5
<210> 13
   <211> 6
   <212> PRT
   <213> Artificial
<220>
   <223> HVVPNEVWQR
<400> 13
   Ala Leu Gln Glu Tyr Arg
   1               5
<210> 14
   <211> 6
   <212> PRT
   <213> Artificial
<220>
   <223> peptide
<400> 14
   Tyr Asn Glu Leu Leu Lys
   1               5
<210> 15
   <211> 8
   <212> PRT
   <213> Artificial
<220>
   <223> peptide
<400> 15
   Phe Tyr Tyr Ile Tyr Asn Glu Lys
   1               5
<210> 16
   <211> 14
   <212> PRT
   <213> Artificial
<220>
   <223> peptide
<400> 16
   Leu Val Ala Tyr Tyr Thr Leu Ile Gly Ala Ser Gly Gln Arg
   1               5                   10
<210> 17
   <211> 13
   <212> PRT
   <213> Artificial
<220>
   <223> peptide
<400> 17
   Ser Pro Asp Val Ile Asn Gly Ser Pro Ile Ser Gln Lys
   1 5 10
<210> 18
   <211> 9
   <212> PRT
   <213> Artificial
<220>
   <223> peptide
<400> 18
   Ile Asp Val His Leu Val Pro Asp Arg
   1 5
<210> 19
   <211> 6
   <212> PRT
   <213> Artificial
<220>
   <223> peptide
<400> 19
   Val Gly Glu Val Leu Lys
   1 5
<210> 20
   <211> 11
   <212> PRT
   <213> Artificial
<220>
   <223> peptide
<400> 20
   Ala Ile Gly Tyr Leu Asn Thr Gly Tyr Gln Arg
   1 5 10
<210> 21
   <211> 9
   <212> PRT
   <213> Artificial
<220>
   <223> peptide
<400> 21
   Thr Gly Thr His Gly Leu Leu Val Lys
   1 5
<210> 22
   <211> 12
   <212> PRT
   <213> Artificial
<220>
   <223> peptide
<400> 22
   Tyr Leu Gln Glu Ile Tyr Asn Ser Asn Asn Gln Lys
   1 5 10
<210> 23
   <211> 8
   <212> PRT
   <213> Artificial
<220>
   <223> peptide
<400> 23
   Leu Asp Gly Ser Val Asp Phe Lys
   1 5
<210> 24
   <211> 7
   <212> PRT
   <213> Artificial
<220>
   <223> peptide
<400> 24
   Val Gly Pro Glu Ala Asp Lys
   1 5
<210> 25
   <211> 10
   <212> PRT
   <213> Artificial
<220>
   <223> peptide
<400> 25
   Val Gly Glu Tyr Ser Leu Tyr Ile Gly Arg
   1 5 10
<210> 26
   <211> 12
   <212> PRT
   <213> Artificial
<220>
   <223> peptide
<400> 26
   Ala Tyr Ser Leu Phe Ser Tyr Asn Thr Gln Gly Arg
   1 5 10
<210> 27
   <211> 9
   <212> PRT
   <213> Artificial
<220>
   <223> peptide
<400> 27
   Leu Gly Pro Leu Val Glu Gln Gly Arg
   1 5
<210> 28
   <211> 9
   <212> PRT
   <213> Artificial
<220>
   <223> peptide
<400> 28
   Leu Gln Ala Glu Ala Phe Gln Ala Arg
   1 5
<210> 29
   <211> 17
   <212> PRT
   <213> Artificial
<220>
   <223> peptide
<400> 29
   Gln Thr Gln Val Ser Val Leu Pro Glu Gly Gly Glu Thr Pro Leu Phe
   1 5 10 15
   Lys
<210> 30
   <211> 8
   <212> PRT
   <213> Artificial
<220>
   <223> peptide
<400> 30
   Thr Ala Ser Asp Phe Ile Thr Lys
   1               5
<210> 31
   <211> 7
   <212> PRT
   <213> Artificial
<220>
   <223> peptide
<400> 31
   Ala Val Glu Val Leu Pro Lys
   1               5
<210> 32
   <211> 11
   <212> PRT
   <213> Artificial
<220>
   <223> peptide
<400> 32
   His Val Val Pro Asn Glu Val Val Val Gln Arg
   1               5                   10